(19) Europäisches Patentamt
European Patent Office
Office européen des brevets

(11) EP 4 752 149 A1

(12) EUROPEAN PATENT APPLICATION
published in accordance with Art. 153(4) EPC

(43) Date of publication:
**03.06.2026 Bulletin 2026/23**

(21) Application number: **24844875.5**

(22) Date of filing: **26.07.2024**

(51) International Patent Classification (IPC):
***C07D 498/06*** *(2006.01)* ***A61P 35/00*** *(2006.01)*
***A61K 31/5383*** *(2006.01)*

(52) Cooperative Patent Classification (CPC):
**A61K 31/5383; A61P 35/00; C07D 498/06**

(86) International application number:
**PCT/CN2024/107786**

(87) International publication number:
**WO 2025/021189 (30.01.2025 Gazette 2025/05)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **27.07.2023 CN 202310930530**
**08.12.2023 CN 202311681118**

(71) Applicant: **Tuojie Biotech (Shanghai) Co., Ltd.**
**Shanghai 201203 (CN)**

(72) Inventors:
- **LI, Yunfei**
  **Shanghai 201203 (CN)**
- **LIU, Haomiao**
  **Shanghai 201203 (CN)**
- **TAN, Liang**
  **Shanghai 201203 (CN)**
- **LI, Jian**
  **Shanghai 201203 (CN)**

(74) Representative: **Potter Clarkson**
**Chapel Quarter**
**Mount Street**
**Nottingham NG1 6HQ (GB)**

# (54) OXYGEN-CONTAINING FUSED TRICYCLIC DERIVATIVE, AND PREPARATION METHOD THEREFOR AND USE THEREOF

(57) Provided are an oxygen-containing fused tricyclic derivative, and a preparation method therefor and a use thereof. Specifically, provided is a compound as shown in formula (I) or a pharmaceutically acceptable salt thereof. The compound as shown in formula (I) can be used as a cyclin-dependent kinase inhibitor for preventing and/or treating diseases related to cyclin-dependent kinase. The definition of each substituent in formula (I) is as recited in the description.

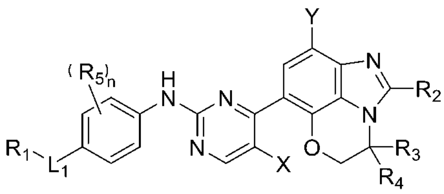

I

EP 4 752 149 A1

## Description

### TECHNICAL FIELD

The present disclosure relates to an oxygen-containing fused tricyclic derivative, a preparation method therefor, and use thereof, and pertains to the field of pharmaceuticals.

### BACKGROUND

Cyclin-dependent kinases (CDKs) represent an important category of kinases and play a crucial role in the division and proliferation of cancer cells and the transcriptional regulation of oncogenes. To date, over 20 subtypes of cyclin-dependent kinases (CDKs) have been identified. Given the sequence and structural similarity in the kinase domains of CDK family members, achieving selective and precise regulation of individual subtypes remains an important challenge.

In recent years, the greatest progress in the field of breast cancer therapy has undoubtedly been the use of CDK4/6 alone or in combination with endocrine therapy for hormone receptor-positive advanced breast cancer. For example, palbociclib, ribociclib, and abemaciclib have been approved for use in combination with aromatase inhibitors to treat hormone receptor (HR)-positive and human epidermal growth factor receptor 2 (HER2)-negative advanced or metastatic breast cancer in post-menopausal women, and palbociclib and abemaciclib have been approved for use in combination with fulvestrant to treat hormone receptor (HR)-positive and human epidermal growth factor receptor 2 (HER2)-negative advanced or metastatic breast cancer in post-menopausal women who have experienced disease progression after endocrine therapy (Nature Reviews (2016) 13:417-430; and J Clin Oncol 2017, 35, 2875-2884). While CDK4/6 inhibitors have shown significant clinical efficacy in estrogen receptor (ER)-positive metastatic breast cancer, their effects-like those of other kinases-may be limited over time by the development of primary or acquired resistance.

In the cell cycle pathway, the degree of phosphorylation of the Rb protein is co-regulated by CDK4/6 and CDK2. In the CDK4/6 inhibitor-resistant population, loss of Rb protein function, elevated CCNE1 expression, increased MYC protein levels, etc., have been observed. Cyclin E is overexpressed in various cancers, particularly in breast cancer, lung cancer, leukemia, and lymphoma (Guo Cuiping et al., Regulation of Cyclin E and Malignant Tumors. Journal of International Oncology, 2012, 39(005):337-340). Amplification or overexpression of cyclin E is also associated with poor prognosis in ovarian cancer, gastric cancer, endometrial cancer, and other cancers.

Research has shown that inhibiting CDK2 kinase can induce the activity of tumor cells with high CCNE1 expression, but inhibiting only the activity of CDK2 is insufficient to sustain inhibitory effects on tumors. Research has found that breast cancer cells treated with a CDK2 inhibitor can activate cyclin A2 through the CDK4 pathway, which, in turn, facilitates the upregulation of CDK2 protein expression levels, so that the cells develop resistance to the CDK2 inhibitor (Cell. 2023 Jun 8; 186(12):2628-2643.e21.). Therefore, inhibiting the activity of CDK4 and the activity of CDK2 simultaneously is of great significance for long-term tumor inhibition. Moreover, inhibiting CDK4 and CDK2 simultaneously may play an important role in reducing resistance to CDK4/6 inhibitors and even resistance to CDK2 inhibitors.

### SUMMARY

In a first aspect, the present disclosure provides a compound represented by formula I or a pharmaceutically acceptable salt thereof,

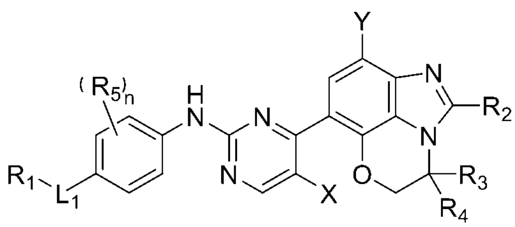

I

wherein:

$R_2$ is selected from the group consisting of hydrogen, $C_{1-6}$ alkyl, $C_{1-6}$ alkoxy, 3-8 membered cycloalkyl, and 3-12 membered heterocycloalkyl, and the $C_{1-6}$ alkyl, $C_{1-6}$ alkoxy, 3-8 membered cycloalkyl, and 3-12 membered heterocycloalkyl are optionally substituted with one or more $R_{21}$;

each $R_{21}$ is independently selected from the group consisting of deuterium, halogen, hydroxy, amino, $C_{1-3}$ alkoxy, 3-7

membered cycloalkyl, and 3-7 membered heterocycloalkyl, and the $C_{1-3}$ alkoxy, 3-7 membered cycloalkyl, and 3-7 membered heterocycloalkyl are optionally substituted with one or more substituents selected from the group consisting of hydroxy, halogen, and deuterium;

$R_3$ and $R_4$ are each independently selected from the group consisting of hydrogen, cyano, halogen, $C_{1-6}$ alkyl, 3-8 membered cycloalkyl, $C_{1-6}$ alkoxy, and 3-12 membered heterocycloalkyl, and the $C_{1-6}$ alkyl, 3-8 membered cycloalkyl, $C_{1-6}$ alkoxy, and 3-12 membered heterocycloalkyl are optionally substituted with one or more $R_{31}$;

each $R_{31}$ is independently selected from the group consisting of halogen, hydroxy, amino, $C_{1-3}$ alkoxy, 3-7 membered cycloalkyl, and 3-7 membered heterocycloalkyl, and the $C_{1-3}$ alkoxy, 3-7 membered cycloalkyl, and 3-7 membered heterocycloalkyl are optionally substituted with one or more substituents selected from the group consisting of hydroxy and halogen;

Y is selected from the group consisting of hydrogen, halogen, cyano, $C_{1-6}$ alkyl, and $C_{1-6}$ haloalkyl;

X is selected from the group consisting of hydrogen, halogen, cyano, $C_{1-6}$ alkyl, and $C_{1-6}$ alkoxy, and the $C_{1-6}$ alkyl and $C_{1-6}$ alkoxy are optionally substituted with one or more $R^a$;

each $R^a$ is independently selected from the group consisting of halogen, hydroxy, amino, $C_{1-3}$ alkoxy, 3-7 membered cycloalkyl, and 3-7 membered heterocycloalkyl, and the $C_{1-3}$ alkoxy, 3-7 membered cycloalkyl, and 3-7 membered heterocycloalkyl are optionally substituted with one or more substituents selected from the group consisting of hydroxy and halogen;

$L_1$ is selected from the group consisting of -CO- and a bond;

$R_1$ is selected from the group consisting of $C_{1-6}$ alkyl, heterocycloalkyl, heteroaryl, - CO-NH-$R_{11}$, -S(O)$_2$-NH-$R_{12}$,

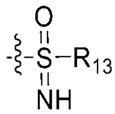 , and 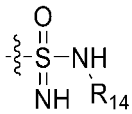 ,

and the $C_{1-6}$ alkyl, heterocycloalkyl, heteroaryl, -CO-NH-$R_{11}$, -S(O)$_2$-NH-$R_{12}$,

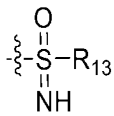 , and 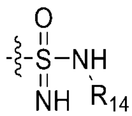

are optionally substituted with one or more $R^b$;

$R_{11}$, $R_{12}$, $R_{13}$, and $R_{14}$ are each independently selected from the group consisting of $C_{1-6}$ alkyl, 3-7 membered cycloalkyl, and 3-7 membered heterocycloalkyl;

each $R^b$ is independently selected from the group consisting of $C_{1-6}$ alkyl, $C_{1-6}$ alkoxy, $C_{1-6}$ haloalkyl, $C_{1-6}$ haloalkoxy, halogen, hydroxy, cyano, oxo, and -N($R^c$)$_2$;

each $R^c$ is independently selected from the group consisting of hydrogen, $C_{1-6}$ alkyl, and $C_{1-6}$ haloalkyl;

or the two $R^c$, together with the N atom to which they are attached, form 3-6 membered heterocycloalkyl, and the 3-6 membered heterocycloalkyl is optionally substituted with one or more halogens;

each $R_5$ is independently selected from the group consisting of hydroxy, cyano, halogen, $C_{1-6}$ alkyl, 3-8 membered cycloalkyl, $C_{1-6}$ alkoxy, and 3-12 membered heterocycloalkyl, and the $C_{1-6}$ alkyl, 3-8 membered cycloalkyl, $C_{1-6}$ alkoxy, and 3-12 membered heterocycloalkyl are optionally substituted with one or more $R_{51}$;

each $R_{51}$ is independently selected from the group consisting of halogen, hydroxy, amino, $C_{1-3}$ alkoxy, 3-7 membered cycloalkyl, and 3-7 membered heterocycloalkyl, and the $C_{1-3}$ alkoxy, 3-7 membered cycloalkyl, and 3-7 membered heterocycloalkyl are optionally substituted with one or more substituents selected from the group consisting of hydroxy and halogen;

n is selected from the group consisting of 0, 1, 2, 3, and 4.

In some embodiments, the compound represented by formula I or the pharmaceutically acceptable salt thereof is provided, wherein $L_1$ is a bond.

In some embodiments, the compound represented by formula I or the pharmaceutically acceptable salt thereof is provided, wherein $L_1$ is -CO-.

In some embodiments, the compound represented by formula I or the pharmaceutically acceptable salt thereof is provided, wherein $R_1$ is selected from the group consisting of 5-7 membered heterocycloalkyl optionally substituted with one or more $R^b$; $R^b$ is as defined in formula I.

In some embodiments, the compound represented by formula I or the pharmaceutically acceptable salt thereof is provided, wherein $R_1$ is selected from the group consisting of 6-membered heterocycloalkyl optionally substituted with one

or more $R^b$; $R^b$ is as defined in formula I.

In some embodiments, the compound represented by formula I or the pharmaceutically acceptable salt thereof is provided, wherein $R_1$ is selected from the group consisting of

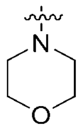 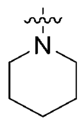 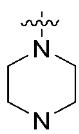 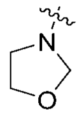 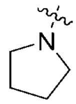

optionally substituted with one or more $R^b$; $R^b$ is as defined in formula I.

In some embodiments, the compound represented by formula I or the pharmaceutically acceptable salt thereof is provided, wherein $R_1$ is selected from the group consisting of

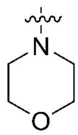 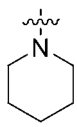 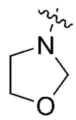 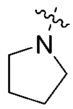

optionally substituted with one or more $R^b$; $R^b$ is as defined in formula I.

In some embodiments, the compound represented by formula I or the pharmaceutically acceptable salt thereof is provided, wherein $R_1$ is selected from the group consisting of

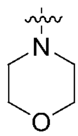 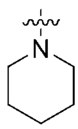 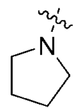

optionally substituted with one or more $R^b$; $R^b$ is as defined in formula I.

In some embodiments, the compound represented by formula I or the pharmaceutically acceptable salt thereof is provided, wherein $R_1$ is $C_{1-6}$ alkyl optionally substituted with one or more $R^b$, and $R^b$ is as defined in formula I.

In some embodiments, the compound represented by formula I or the pharmaceutically acceptable salt thereof is provided, wherein $R_1$ is 5-6 membered heteroaryl optionally substituted with one or more $R^b$, and $R^b$ is as defined in formula I.

In some embodiments, the compound represented by formula I or the pharmaceutically acceptable salt thereof is provided, wherein $R_1$ is 5-membered heteroaryl optionally substituted with one or more $R^b$, and $R^b$ is as defined in formula I.

In some embodiments, the compound represented by formula I or the pharmaceutically acceptable salt thereof is provided, wherein $R_1$ is

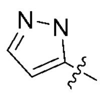

optionally substituted with one or more $R^b$, and $R^b$ is as defined in formula I.

In some embodiments, the compound represented by formula I or the pharmaceutically acceptable salt thereof is provided, wherein $R_1$ is -CO-NH-$R_{11}$ optionally substituted with one or more $R^b$; $R_{11}$ is selected from the group consisting of $C_{1-6}$ alkyl, 3-7 membered cycloalkyl, and 3-7 membered heterocycloalkyl; preferably, $R_{11}$ is $C_{1-6}$ alkyl or 3-7 membered cycloalkyl; more preferably, $R_{11}$ is $C_{1-6}$ alkyl; $R^b$ is as defined in formula I.

In some embodiments, the compound represented by formula I or the pharmaceutically acceptable salt thereof is provided, wherein $R_1$ is -S(O)$_2$-NH-$R_{12}$ optionally substituted with one or more $R^b$; $R_{12}$ is selected from the group consisting of $C_{1-6}$ alkyl, 3-7 membered cycloalkyl, and 3-7 membered heterocycloalkyl; preferably, $R_{12}$ is selected from the group consisting of $C_{1-6}$ alkyl and 3-7 membered cycloalkyl; more preferably, $R_{12}$ is $C_{1-6}$ alkyl; $R^b$ is as defined in formula I.

In some embodiments, the compound represented by formula I or the pharmaceutically acceptable salt thereof is provided, wherein $R_1$ is

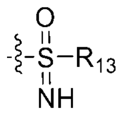

optionally substituted with one or more $R^b$; $R_{13}$ is selected from the group consisting of $C_{1-6}$ alkyl, 3-7 membered cycloalkyl, and 3-7 membered heterocycloalkyl; preferably, $R_{13}$ is selected from the group consisting of $C_{1-6}$ alkyl and 3-7 membered cycloalkyl; more preferably, $R_{13}$ is $C_{1-6}$ alkyl; $R^b$ is as defined in formula I.

In some embodiments, the compound represented by formula I or the pharmaceutically acceptable salt thereof is provided, wherein $R_1$ is

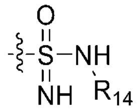

optionally substituted with one or more $R^b$; $R_{14}$ is selected from the group consisting of $C_{1-6}$ alkyl, 3-7 membered cycloalkyl, and 3-7 membered heterocycloalkyl; preferably, $R_{14}$ is selected from the group consisting of $C_{1-6}$ alkyl and 3-7 membered cycloalkyl; more preferably, $R_{14}$ is $C_{1-6}$ alkyl; $R^b$ is as defined in formula I.

In some embodiments, the compound represented by formula I or the pharmaceutically acceptable salt thereof is provided, wherein each $R^b$ is independently selected from the group consisting of halogen, hydroxy, cyano, and oxo.

In some embodiments, the compound represented by formula I or the pharmaceutically acceptable salt thereof is provided, wherein each $R^b$ is independently selected from the group consisting of oxo.

In some embodiments, the compound represented by formula I or the pharmaceutically acceptable salt thereof is provided, wherein each $R^b$ is independently selected from the group consisting of $C_{1-6}$ alkyl, $C_{1-6}$ alkoxy, $C_{1-6}$ haloalkyl, and $C_{1-6}$ haloalkoxy.

In some embodiments, the compound represented by formula I or the pharmaceutically acceptable salt thereof is provided, wherein each $R^b$ is independently selected from the group consisting of $C_{1-6}$ alkyl and $C_{1-6}$ haloalkyl.

In some embodiments, the compound represented by formula I or the pharmaceutically acceptable salt thereof is provided, wherein each $R^b$ is independently selected from the group consisting of halogen.

In some embodiments, the compound represented by formula I or the pharmaceutically acceptable salt thereof is provided, wherein each $R^b$ is independently selected from the group consisting of $-N(R^c)_2$, and each $R^c$ is independently selected from the group consisting of hydrogen, $C_{1-6}$ alkyl, and $C_{1-6}$ haloalkyl.

In some embodiments, the compound represented by formula I or the pharmaceutically acceptable salt thereof is provided, wherein each $R^b$ is independently selected from the group consisting of $-N(R^c)_2$; the two $R^c$, together with the N atom to which they are attached, form 3-6 membered heterocycloalkyl, and the 3-6 membered heterocycloalkyl is optionally substituted with one or more halogens.

In a second aspect, the present disclosure further provides a compound represented by formula II or a pharmaceutically acceptable salt thereof,

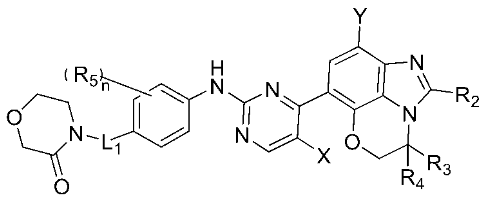

II

wherein $R_2$, $R_3$, $R_4$, $R_5$, X, Y, $L_1$, and n are as defined in formula I.

In some embodiments, the compound represented by formula I or formula II or the pharmaceutically acceptable salt thereof is provided, wherein $R_2$ is selected from the group consisting of hydrogen, $C_{1-6}$ alkyl, and 3-8 membered cycloalkyl, and the $C_{1-6}$ alkyl and 3-8 membered cycloalkyl are optionally substituted with one or more $R_{21}$; $R_{21}$ is as defined in formula I.

In some embodiments, the compound represented by formula I or formula II or the pharmaceutically acceptable salt thereof is provided, wherein $R_2$ is 3-8 membered cycloalkyl, and the 3-8 membered cycloalkyl is optionally substituted with one or more $R_{21}$; $R_{21}$ is as defined in formula I.

In some embodiments, the compound represented by formula I or formula II or the pharmaceutically acceptable salt thereof is provided, wherein $R_2$ is 3-6 membered cycloalkyl, and the 3-6 membered cycloalkyl is optionally substituted

with one or more $R_{21}$; $R_{21}$ is as defined in formula I.

In some embodiments, the compound represented by formula I or formula II or the pharmaceutically acceptable salt thereof is provided, wherein $R_2$ is selected from the group consisting of hydrogen and $C_{1-6}$ alkyl optionally substituted with one or more $R_{21}$; $R_{21}$ is as defined in formula I.

In some embodiments, the compound represented by formula I or formula II or the pharmaceutically acceptable salt thereof is provided, wherein each $R_{21}$ is independently selected from the group consisting of deuterium, halogen, and hydroxy; preferably, $R_{21}$ is independently selected from the group consisting of hydroxy.

In some embodiments, the compound represented by formula I or formula II or the pharmaceutically acceptable salt thereof is provided, wherein each $R_{21}$ is independently selected from the group consisting of fluorine, chlorine, and bromine.

In some embodiments, the compound represented by formula I or formula II or the pharmaceutically acceptable salt thereof is provided, wherein each $R_{21}$ is independently selected from the group consisting of $C_{1-3}$ alkoxy, and the $C_{1-3}$ alkoxy is optionally substituted with one or more substituents selected from the group consisting of hydroxy, halogen, and deuterium.

In some embodiments, the compound represented by formula I or formula II or the pharmaceutically acceptable salt thereof is provided, wherein $R_3$ and $R_4$ are each independently selected from the group consisting of hydrogen, cyano, halogen, and $C_{1-6}$ alkyl, and the $C_{1-6}$ alkyl is optionally substituted with one or more $R_{31}$; $R_{31}$ is independently selected from the group consisting of halogen, hydroxy, amino, and $C_{1-3}$ alkoxy, and the $C_{1-3}$ alkoxy is optionally substituted with one or more substituents selected from the group consisting of hydroxy and halogen.

In some embodiments, the compound represented by formula I or formula II or the pharmaceutically acceptable salt thereof is provided, wherein $R_3$ and $R_4$ are each independently selected from the group consisting of hydrogen, cyano, halogen, $C_{1-6}$ alkyl, and $C_{1-6}$ haloalkyl.

In some embodiments, the compound represented by formula I or formula II or the pharmaceutically acceptable salt thereof is provided, wherein $R_3$ is selected from the group consisting of H.

In some embodiments, the compound represented by formula I or formula II or the pharmaceutically acceptable salt thereof is provided, wherein $R_4$ is selected from the group consisting of $C_{1-6}$ alkyl and $C_{1-6}$ haloalkyl.

In some embodiments, the compound represented by formula I or formula II or the pharmaceutically acceptable salt thereof is provided, wherein $R_4$ is selected from the group consisting of $C_{1-6}$ alkyl.

In some embodiments, the compound represented by formula I or formula II or the pharmaceutically acceptable salt thereof is provided, wherein $R_4$ is methyl.

In some embodiments, the compound represented by formula I or formula II or the pharmaceutically acceptable salt thereof is provided, wherein Y is selected from the group consisting of halogen.

In some embodiments, the compound represented by formula I or formula II or the pharmaceutically acceptable salt thereof is provided, wherein Y is selected from the group consisting of fluorine, chlorine, and H; preferably, Y is selected from the group consisting of fluorine.

In some embodiments, the compound represented by formula I or formula II or the pharmaceutically acceptable salt thereof is provided, wherein X is selected from the group consisting of hydrogen, halogen, and cyano.

In some embodiments, the compound represented by formula I or formula II or the pharmaceutically acceptable salt thereof is provided, wherein X is selected from the group consisting of halogen and cyano.

In some embodiments, the compound represented by formula I or formula II or the pharmaceutically acceptable salt thereof is provided, wherein X is selected from the group consisting of fluorine and chlorine.

In some embodiments, the compound represented by formula I or formula II or the pharmaceutically acceptable salt thereof is provided, wherein each $R_5$ is independently selected from the group consisting of hydroxy, cyano, and halogen.

In some embodiments, the compound represented by formula I or formula II or the pharmaceutically acceptable salt thereof is provided, wherein each $R_5$ is independently selected from the group consisting of $C_{1-6}$ alkyl, 3-8 membered cycloalkyl, $C_{1-6}$ alkoxy, and 3-6 membered heterocycloalkyl, and the $C_{1-6}$ alkyl, 3-8 membered cycloalkyl, $C_{1-6}$ alkoxy, and 3-6 membered heterocycloalkyl are optionally substituted with one or more $R_{51}$;

each $R_{51}$ is independently selected from the group consisting of halogen, hydroxy, amino, $C_{1-3}$ alkoxy, 3-7 membered cycloalkyl, and 3-7 membered heterocycloalkyl, and the $C_{1-3}$ alkoxy, 3-7 membered cycloalkyl, and 3-7 membered heterocycloalkyl are optionally substituted with one or more substituents selected from the group consisting of hydroxy and halogen.

In some embodiments, the compound represented by formula I or formula II or the pharmaceutically acceptable salt thereof is provided, wherein each $R_5$ is independently selected from the group consisting of halogen, $C_{1-6}$ alkyl, 3-6 membered cycloalkyl, $C_{1-6}$ alkoxy, and 3-6 membered heterocycloalkyl, and the $C_{1-6}$ alkyl, 3-6 membered cycloalkyl, $C_{1-6}$ alkoxy, and 3-6 membered heterocycloalkyl are optionally substituted with one or more $R_{51}$;

each $R_{51}$ is independently selected from the group consisting of halogen, hydroxy, amino, $C_{1-3}$ alkoxy, 3-7 membered cycloalkyl, and 3-7 membered heterocycloalkyl, and the $C_{1-3}$ alkoxy, 3-7 membered cycloalkyl, and 3-7 membered

heterocycloalkyl are optionally substituted with one or more substituents selected from the group consisting of hydroxy and halogen.

In some embodiments, the compound represented by formula II or the pharmaceutically acceptable salt thereof according to the second aspect is provided, wherein:

$L_1$ is a bond;
$R_2$ is selected from the group consisting of hydrogen, $C_{1-6}$ alkyl, and 3-8 membered cycloalkyl, and the $C_{1-6}$ alkyl and 3-8 membered cycloalkyl are optionally substituted with one or more $R_{21}$; each $R_{21}$ is independently selected from the group consisting of hydroxy and $C_{1-3}$ alkoxy, and the $C_{1-3}$ alkoxy is optionally substituted with one or more substituents selected from the group consisting of hydroxy, halogen, and deuterium;
$R_3$ is selected from the group consisting of H;
$R_4$ is selected from the group consisting of $C_{1-6}$ alkyl and $C_{1-6}$ haloalkyl; preferably, $R_4$ is selected from the group consisting of $C_{1-6}$ alkyl;
Y is selected from the group consisting of hydrogen and halogen; preferably, Y is selected from the group consisting of fluorine, chlorine, and hydrogen;
X is selected from the group consisting of hydrogen, halogen, and cyano; preferably, X is selected from the group consisting of fluorine, chlorine, and hydrogen;
n is selected from the group consisting of 0 and 1;
$R_5$ is independently selected from the group consisting of halogen, $C_{1-6}$ alkyl, 3-6 membered cycloalkyl, $C_{1-6}$ alkoxy, and 3-6 membered heterocycloalkyl, and the $C_{1-6}$ alkyl, 3-6 membered cycloalkyl, $C_{1-6}$ alkoxy, and 3-6 membered heterocycloalkyl are optionally substituted with one or more $R_{51}$;
each $R_{51}$ is independently selected from the group consisting of halogen, hydroxy, and amino.

In a third aspect, the present disclosure further provides a compound represented by the following formula or a pharmaceutically acceptable salt thereof:

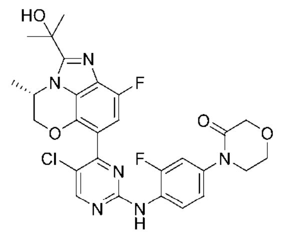,
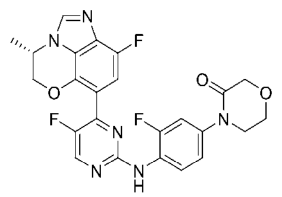,
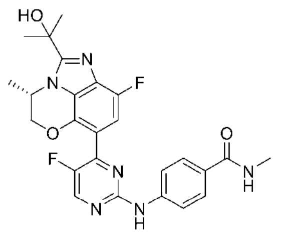,
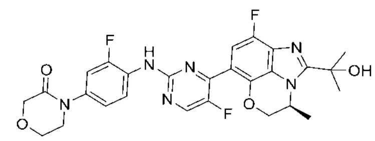,
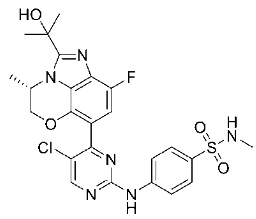,
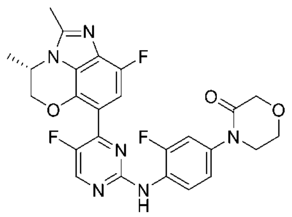,
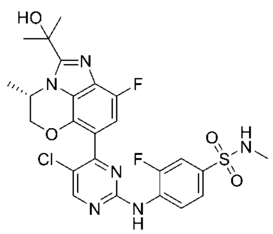,
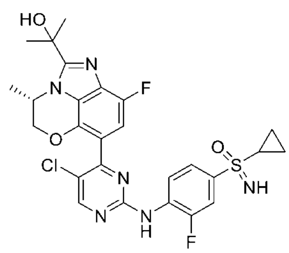,
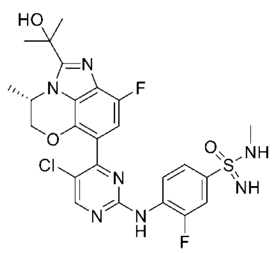,
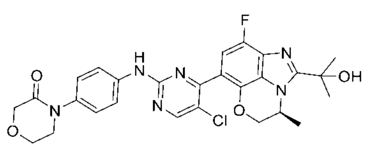,
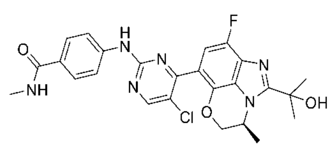,
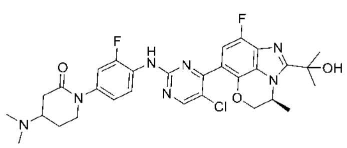,

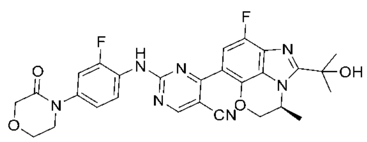, 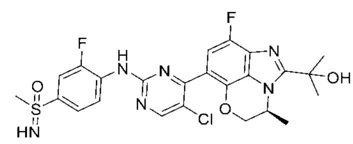, 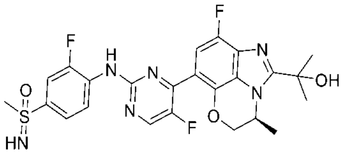,

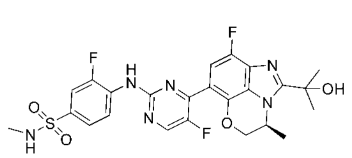, 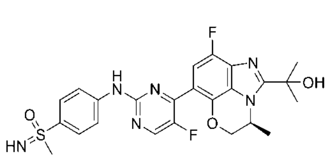, 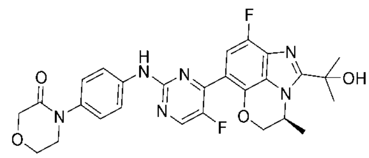,

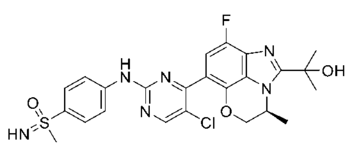, 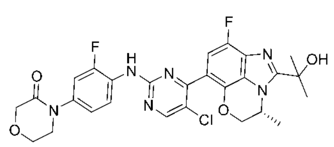, 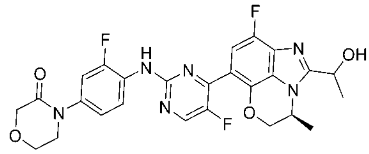,

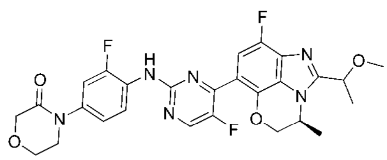, 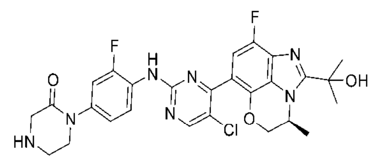, 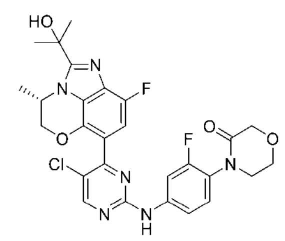,

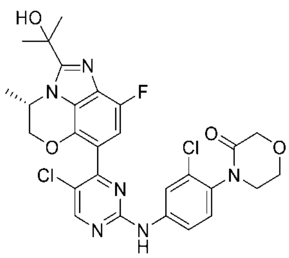, 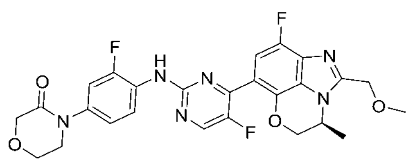, 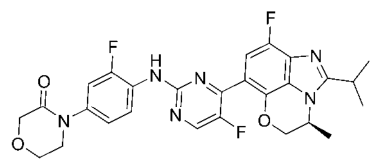,

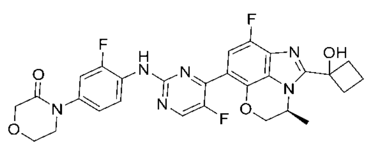, 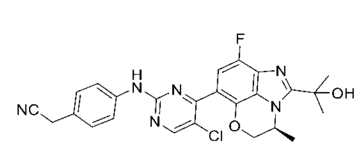, 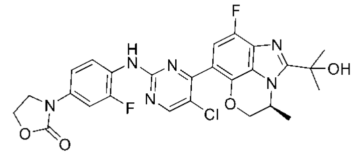,

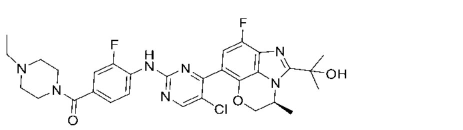, 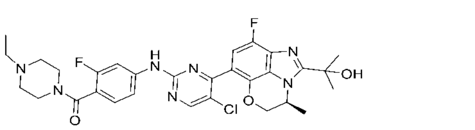,

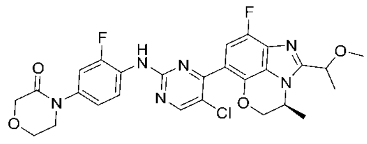, 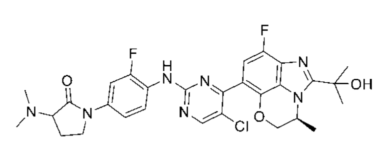, 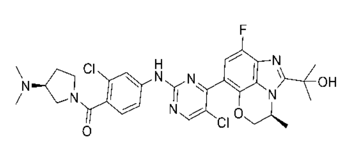,

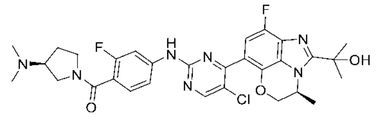,

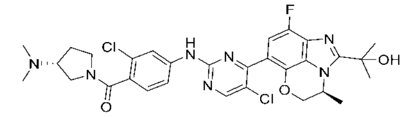,

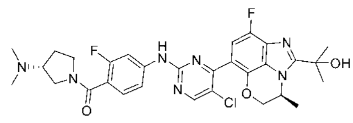,

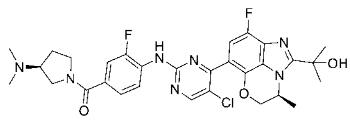,

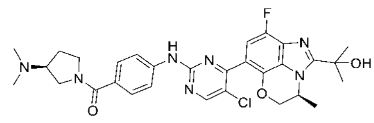,

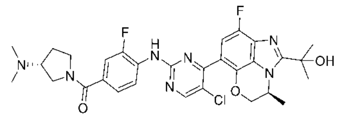,

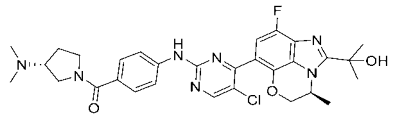,

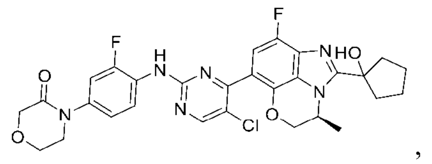,

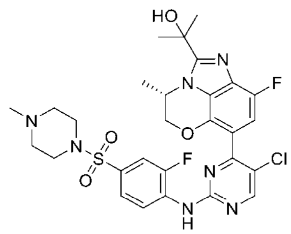,

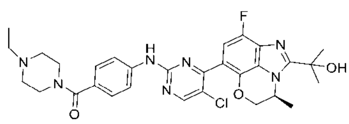,

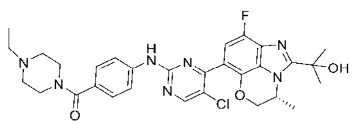,

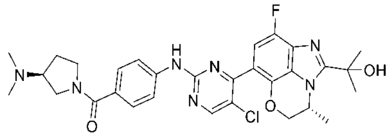,

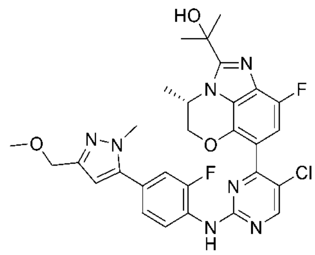,

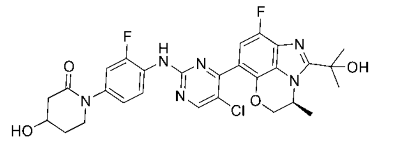,

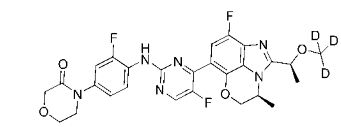,

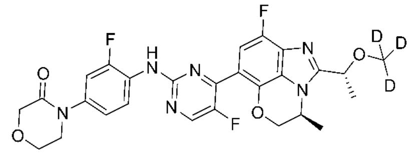,

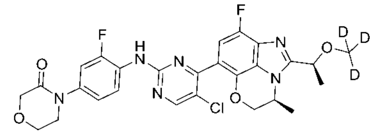,

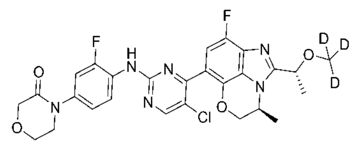,

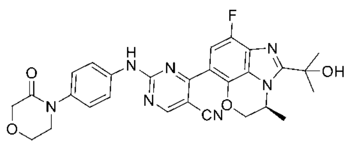,

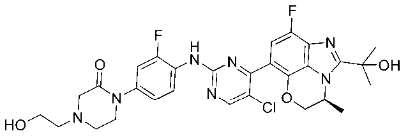
,
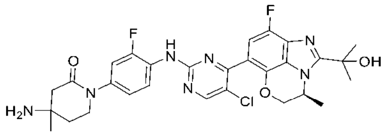
,
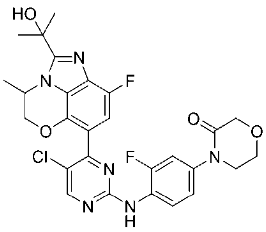
,
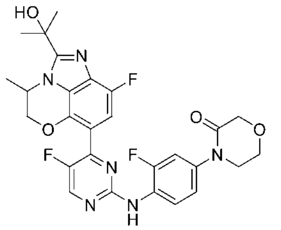
,
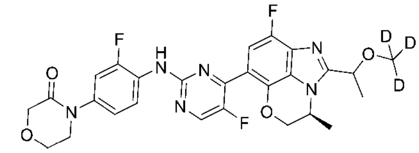
,

or

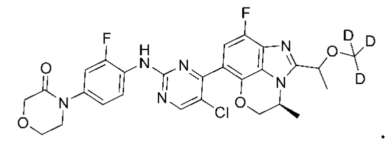
.

In a fourth aspect, the present disclosure provides an isotopically substituted form of the compound or the pharmaceutically acceptable salt thereof according to the first, second, or third aspect; preferably, the isotopically substituted form is deuterium-substituted.

The present disclosure further provides a preparation method for a compound represented by formula I or a pharmaceutically acceptable salt or isotopically substituted form thereof, comprising a step of reacting a compound represented by formula A with a compound represented by formula B,

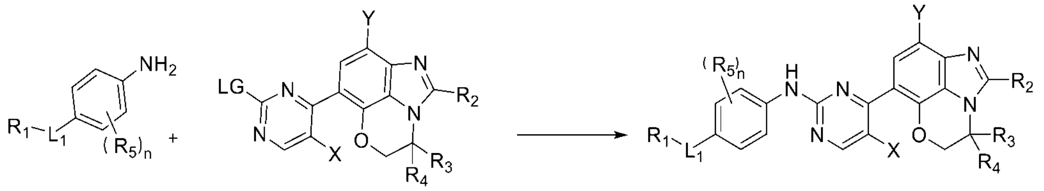

A B I

wherein $R_1$, $R_2$, $R_3$, $R_4$, $R_5$, X, Y, $L_1$, and n are as defined in formula I;
wherein LG is selected from the group consisting of leaving groups, such as halogen, sulfonate, and borate.

In another aspect, the present disclosure further provides a compound represented by formula B or a pharmaceutically acceptable salt thereof,

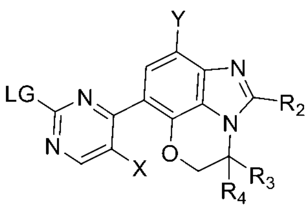

B

wherein $R_2$, $R_3$, $R_4$, X, and Y are as defined in formula I;
wherein LG is selected from the group consisting of leaving groups, such as halogen, sulfonate, and borate.

In another aspect, the present disclosure further provides a compound represented by formula A or a pharmaceutically acceptable salt thereof,

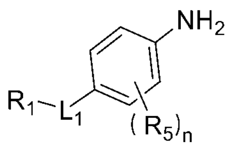

A

wherein $R_1$, $R_5$, $L_1$, and n are as defined in formula I.

In some embodiments, formula A and formula B are intermediates.

In a fifth aspect, the present disclosure provides a pharmaceutical composition comprising a therapeutically effective amount of at least one of the compound or the pharmaceutically acceptable salt thereof according to the first, second, or third aspect or the isotopically substituted form according to the fourth aspect, and a pharmaceutically acceptable excipient.

In some embodiments, a unit dose of the pharmaceutical composition is 0.001 mg-1000 mg.

In some embodiments, based on the total weight of the composition, the pharmaceutical composition comprises 0.01-99.99% of the aforementioned compound or pharmaceutically acceptable salt thereof. In certain embodiments, the pharmaceutical composition comprises 0.1-99.9% of the aforementioned compound or pharmaceutically acceptable salt thereof. In certain embodiments, the pharmaceutical composition comprises 0.5%-99.5% of the aforementioned compound or pharmaceutically acceptable salt thereof. In certain embodiments, the pharmaceutical composition comprises 1%-99% of the aforementioned compound or pharmaceutically acceptable salt thereof. In certain embodiments, the pharmaceutical composition comprises 2%-98% of the aforementioned compound or pharmaceutically acceptable salt thereof.

In some embodiments, based on the total weight of the composition, the pharmaceutical composition comprises 0.01%-99.99% of the pharmaceutically acceptable excipient. In certain embodiments, the pharmaceutical composition comprises 0.1%-99.9% of the pharmaceutically acceptable excipient. In certain embodiments, the pharmaceutical composition comprises 0.5%-99.5% of the pharmaceutically acceptable excipient. In certain embodiments, the pharmaceutical composition comprises 1%-99% of the pharmaceutically acceptable excipient. In certain embodiments, the pharmaceutical composition comprises 2%-98% of the pharmaceutically acceptable excipient.

The present disclosure further provides use of the compound represented by formula I or formula II or the pharmaceutically acceptable salt thereof or the isotopically substituted form according to the fourth aspect or the pharmaceutical composition according to the fifth aspect in the manufacture of a medicament for treating or preventing a disease or disorder associated with abnormal activity of a serine/threonine kinase.

The present disclosure further provides use of the compound represented by formula I or formula II or the pharmaceutically acceptable salt thereof or the isotopically substituted form according to the fourth aspect or the pharmaceutical composition according to the fifth aspect in the manufacture of a medicament for treating or preventing a disease or disorder associated with abnormal activity of CDK2.

The present disclosure further provides use of the compound represented by formula I or formula II or the pharmaceutically acceptable salt thereof or the isotopically substituted form according to the fourth aspect or the pharmaceutical composition according to the fifth aspect in the manufacture of a medicament for treating or preventing a disease or disorder associated with abnormal activity of CDK4.

The present disclosure further provides use of the compound represented by formula I or formula II or the pharmaceutically acceptable salt thereof or the isotopically substituted form according to the fourth aspect or the pharmaceutical composition according to the fifth aspect in the manufacture of a medicament for treating or preventing a disease or disorder, wherein the disease or disorder is selected from the group consisting of proliferative diseases, inflammatory diseases, autoinflammatory diseases, autoimmune diseases, and infectious diseases.

The present disclosure further provides use of the compound represented by formula I or formula II or the pharmaceutically acceptable salt thereof or the isotopically substituted form according to the fourth aspect or the pharmaceutical composition according to the fifth aspect for treating or preventing a disease or disorder associated with abnormal activity of a serine/threonine kinase.

The present disclosure further provides use of the compound represented by formula I or formula II or the pharmaceutically acceptable salt thereof or the isotopically substituted form according to the fourth aspect or the pharmaceutical composition according to the fifth aspect for treating or preventing a disease or disorder associated with abnormal activity of CDK2.

The present disclosure further provides use of the compound represented by formula I or formula II or the

pharmaceutically acceptable salt thereof or the isotopically substituted form according to the fourth aspect or the pharmaceutical composition according to the fifth aspect for treating or preventing a disease or disorder associated with abnormal activity of CDK4.

The present disclosure further provides use of the compound represented by formula I or formula II or the pharmaceutically acceptable salt thereof or the isotopically substituted form according to the fourth aspect or the pharmaceutical composition according to the fifth aspect for treating or preventing a disease or disorder, wherein the disease or disorder is selected from the group consisting of proliferative diseases, inflammatory diseases, autoinflammatory diseases, autoimmune diseases, and infectious diseases.

The present disclosure further provides a method for treating or preventing a disease or disorder associated with abnormal activity of a serine/threonine kinase, comprising administering to a patient a therapeutically or prophylactically effective amount of the compound represented by formula I or formula II or the pharmaceutically acceptable salt thereof or the isotopically substituted form according to the fourth aspect or the pharmaceutical composition according to the fifth aspect.

The present disclosure further provides a method for treating or preventing a disease or disorder associated with abnormal activity of CDK2, comprising administering to a patient a therapeutically or prophylactically effective amount of the compound represented by formula I or formula II or the pharmaceutically acceptable salt thereof or the isotopically substituted form according to the fourth aspect or the pharmaceutical composition according to the fifth aspect.

The present disclosure further provides a method for treating or preventing a disease or disorder associated with abnormal activity of CDK4, comprising administering to a patient a therapeutically or prophylactically effective amount of the compound represented by formula I or formula II or the pharmaceutically acceptable salt thereof or the isotopically substituted form according to the fourth aspect or the pharmaceutical composition according to the fifth aspect.

The present disclosure further provides a method for treating or preventing a disease or disorder, comprising administering to a patient a therapeutically or prophylactically effective amount of the compound represented by formula I or formula II or the pharmaceutically acceptable salt thereof or the isotopically substituted form according to the fourth aspect or the pharmaceutical composition according to the fifth aspect, wherein the disease or disorder is selected from the group consisting of proliferative diseases, inflammatory diseases, auto inflammatory diseases, autoimmune diseases, and infectious diseases.

In some embodiments, the disease or disorder is a proliferative disease.

In some embodiments, the proliferative disease is selected from the group consisting of breast cancer (e.g., triple-negative breast cancer), intestinal cancer, lung cancer, pancreatic cancer, prostate cancer, Ewing sarcoma, osteoma, neuroblastoma, cervical cancer, ovarian cancer, gastric cancer, and liver cancer.

In some embodiments, the breast cancer is ER-negative, PR-negative, and Her2-positive breast cancer.

In some embodiments, the breast cancer is CDK4/6 inhibitor-resistant breast cancer.

In some embodiments, the breast cancer is CDK4/6 inhibitor-resistant ER-positive breast cancer.

In some embodiments, the lung cancer is non-small cell lung cancer.

The pharmaceutically acceptable salts of the compounds described in the present disclosure may be selected from the group consisting of inorganic salts and organic salts.

The compounds of the present disclosure may have particular geometric or stereoisomeric forms. The present disclosure contemplates all such compounds, including cis and trans isomers, (-)- and (+)-enantiomers, (*R*)- and (*S*)-enantiomers, diastereomers, (*D*)-isomer, (*L*)-isomer, and racemic mixtures and other mixtures thereof, such as enantiomerically or diastereomerically enriched mixtures, all of which fall within the scope of the present disclosure. Additional asymmetric carbon atoms may be present in substituents such as alkyl. All these isomers and mixtures thereof are included within the scope of the present disclosure. The compounds of the present disclosure containing asymmetric carbon atoms may be separated in an optically active pure form or in a racemic form. The optically active pure form may be isolated from a racemic mixture or synthesized using chiral starting materials or chiral reagents.

Optically active (*R*)- and (*S*)-isomers, and *D*- and *L*-isomers may be prepared by chiral synthesis, chiral reagents, or other conventional techniques. If one enantiomer of a certain compound of the present disclosure is desired, it may be prepared by asymmetric synthesis or derivatization with a chiral auxiliary, wherein the resulting mixture of diastereomers is separated and the auxiliary group is cleaved to provide the pure desired enantiomer. Alternatively, when the molecule contains a basic functional group (e.g., amino) or an acidic functional group (e.g., carboxyl), salts of diastereomers are formed with an appropriate optically active acid or base, diastereomeric resolution is then performed by conventional methods well-known in the art, and pure enantiomers are then recovered. In addition, the separation of enantiomers and diastereomers is generally accomplished by chromatography using a chiral stationary phase, optionally in combination with chemical derivatization (e.g., carbamate formation from amines). In the chemical structures of the compounds of the present disclosure, the bond "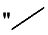" indicates an unspecified configuration; that is, if chiral isomers exist in the chemical structures, the bond "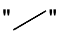" may be "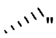" or "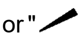", or includes both the configurations "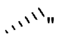" and "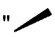" simultaneously. In

the chemical structures of the compounds of the present disclosure, the bond "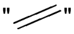" does not specify a configuration; that is, the configuration of the bond "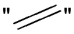" may be an E configuration or a Z configuration, or includes both the E configuration and the Z configuration simultaneously.

The compounds and intermediates of the present disclosure may also exist in different tautomeric forms, and all such forms are included within the scope of the present disclosure. The term "tautomer" or "tautomeric form" refers to structural isomers of different energies that can interconvert via a low-energy barrier. For example, proton tautomers (also known as proton transfer tautomers) include interconversion via proton migration, such as keto-enol and imine-enamine, and lactam-lactim isomerization. All tautomeric forms fall within the scope of the present disclosure. The names of the compounds do not exclude any tautomers.

The present disclosure also includes some isotopically labeled compounds of the present disclosure that are identical to those recited herein but have one or more atoms replaced with an atom having an atomic mass or mass number different from the atomic mass or mass number usually found in nature. Examples of isotopes that can be incorporated into the compounds of the present disclosure include isotopes of hydrogen, carbon, nitrogen, oxygen, phosphorus, sulfur, fluorine, iodine, and chlorine, such as $^{2}H$, $^{3}H$, $^{11}C$, $^{13}C$, $^{14}C$, $^{13}N$, $^{15}N$, $^{15}O$, $^{17}O$, $^{18}O$, $^{31}P$, $^{32}P$, $^{35}S$, $^{18}F$, $^{123}I$, $^{125}I$, and $^{36}Cl$.

Unless otherwise specified, when a position is specifically designated as deuterium (D), the position shall be understood to be deuterium having an abundance that is at least 1000 times greater than the natural abundance of deuterium (which is 0.015%) (i.e., at least 10% deuterium incorporation). The compounds of examples comprise deuterium having an abundance that is greater than at least 1000 times the natural abundance, at least 2000 times the natural abundance, at least 3000 times the natural abundance, at least 4000 times the natural abundance, at least 5000 times the natural abundance, at least 6000 times the natural abundance, or higher times the natural abundance. The present disclosure also includes various deuterated forms of the compound of formula (I). Each available hydrogen atom linked to a carbon atom may be independently replaced with a deuterium atom. Those skilled in the art are able to synthesize the deuterated forms of the compound of formula (I) with reference to the relevant literature. Commercially available deuterated starting materials can be used in preparing the deuterated forms of the compound of formula (I), or they can be synthesized using conventional techniques with deuterated reagents, including but not limited to deuterated borane, tri-deuterated borane in tetrahydrofuran, deuterated lithium aluminum hydride, deuterated iodoethane, deuterated iodomethane, etc.

"Optionally" or "optional" means that the event or circumstance subsequently described may, but does not necessarily, occur; this description includes an instance where the event or circumstance occurs or does not occur. For example, "$C_{1-6}$ alkyl optionally substituted with halogen or cyano" means that the halogen or cyano may, but does not necessarily, exist; this description includes an instance where the alkyl is substituted with halogen or cyano and an instance where the alkyl is not substituted with halogen or cyano.

"Pharmaceutical composition" refers to a mixture containing one or more of the compounds or the physiologically and pharmaceutically acceptable salts or pro-drugs thereof described herein, and other chemical components, as well as other components such as physiologically and pharmaceutically acceptable carriers and excipients. The pharmaceutical composition is intended to promote administration to an organism and facilitate the absorption of the active ingredient so that it can exert its biological activity. "Pharmaceutically acceptable excipient" includes, but is not limited to, any auxiliary, carrier, glidant, sweetener, diluent, preservative, dye/colorant, flavoring agent, surfactant, wetting agent, dispersant, suspending agent, stabilizer, isotonic agent, solvent, or emulsifier that has been approved by the U.S. Food and Drug Administration (FDA) as acceptable for use in humans or livestock animals.

"Effective amount" or "therapeutically effective amount" as described in the present disclosure includes an amount sufficient to ameliorate or prevent a symptom or disorder of a medical disorder. An effective amount also means an amount sufficient to allow or facilitate diagnosis. The effective amount for a particular patient or veterinary subject may vary depending on factors such as the disorder to be treated, the general health of the patient, the method, route, and dose of administration, and the severity of side effects. An effective amount may be the maximum dose or administration regimen to avoid significant side effects or toxic effects.

The term "alkyl" refers to a saturated aliphatic hydrocarbon group, including linear and branched groups of 1 to 20 carbon atoms; alkyl groups containing 1 to 6 or 1 to 3 carbon atoms are preferred. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, various branched isomers thereof, etc. Alkyl may be substituted or unsubstituted, and when it is substituted, the substituent may be substituted at any available point of attachment.

The term "cycloalkyl" refers to a saturated or partially unsaturated monocyclic or polycyclic hydrocarbon substituent, and the cycloalkyl ring contains 3 to 20 carbon atoms, preferably 3 to 8 carbon atoms, more preferably 3 to 7 carbon atoms or 3-6 carbon atoms. Non-limiting examples of monocyclic cycloalkyl groups include cyclopropyl, cyclobutyl, cyclopentyl, cyclopentenyl, cyclohexyl, cyclohexenyl, cyclohexadienyl, etc. Polycyclic cycloalkyl groups include spiro-ring, fused-ring, and bridged-ring cycloalkyl groups. Cycloalkyl may be substituted or unsubstituted.

The term "heterocycloalkyl" refers to a saturated or partially unsaturated monocyclic heterocyclic (i.e., monocyclic heterocycloalkyl) or polycyclic heterocyclic system (i.e., polycyclic heterocycloalkyl), and it contains in the ring(s) at least one (e.g., 1, 2, 3, or 4) heteroatom selected from the group consisting of nitrogen, oxygen, and sulfur (the nitrogen may be optionally oxidized to form a nitrogen oxide; the sulfur may be optionally substituted with oxo to form a sulfoxide or sulfone, but -O-O-, -O-S-, or -S-S- is excluded) and has 3 to 20 (e.g., 3, 4, 5, 6, 7, 8, 9, 10, 11, 12, 13, 14, 15, 16, 17, 18, 19, or 20) ring atoms (i.e., 3- to 20-membered heterocyclyl). Preferably, the heterocyclyl is a heterocyclyl group having 3 to 12 ring atoms (i.e., 3- to 12-membered heterocyclyl) or a heterocyclyl group having 4 to 11 ring atoms (i.e., 4- to 11-membered heterocyclyl); further preferably, the heterocyclyl is a heterocyclyl group having 3 to 8 ring atoms (i.e., 3- to 8-membered heterocyclyl); more preferably, the heterocyclyl is a heterocyclyl group having 3 to 6 ring atoms (i.e., 3- to 6-membered heterocyclyl); most preferably, the heterocyclyl is a heterocyclyl group having 5 or 6 ring atoms (i.e., 5- or 6-membered heterocyclyl).

The polycyclic heterocyclyl includes spiroheterocyclyl, fused heterocyclyl, and bridged heterocyclyl.

Non-limiting examples of the monocyclic heterocycloalkyl include: pyrrolidinyl, tetrahydropyranyl, 1,2,3,6-tetrahydropyridyl, piperidinyl, piperazinyl, morpholinyl, thiomorpholinyl, homopiperazinyl, etc.

Non-limiting examples of "polycyclic heterocycloalkyl" include:

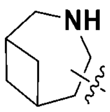, 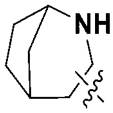, 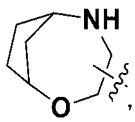, 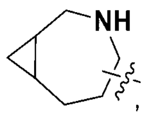, 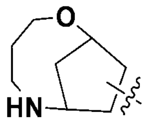, 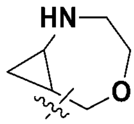,

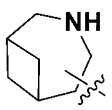, 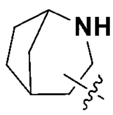, 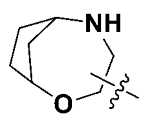, 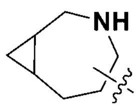, 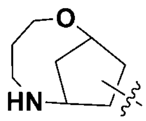, 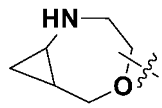,

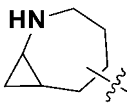, 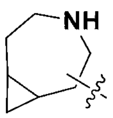, 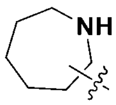, 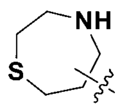, 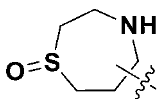, 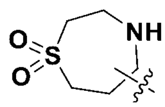,

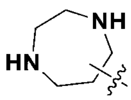, 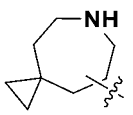, 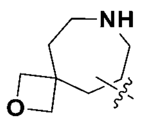, 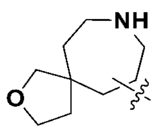, 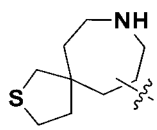 and 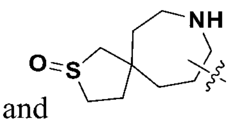.

The heterocycloalkyl ring may be fused to an aryl or heteroaryl ring, wherein the ring attached to the parent structure is heterocycloalkyl; its non-limiting examples include:

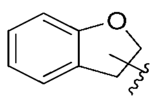, 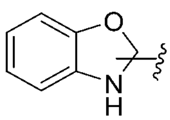, 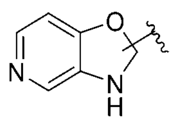, 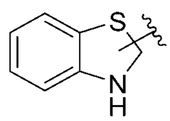,

etc.

Heterocycloalkyl may be optionally substituted or unsubstituted.

The term "alkoxy" refers to -O-(alkyl), wherein the alkyl is as defined above. Non-limiting examples of alkoxy groups include methoxy, ethoxy, propoxy, and butoxy. Alkoxy may be optionally substituted or unsubstituted.

The term "aryl" refers to a 6- to 14-membered, preferably 6- to 12-membered, all-carbon monocyclic or fused polycyclic (i.e., rings sharing a pair of adjacent carbon atoms) group having a conjugated $\pi$-electron system, such as phenyl and naphthyl. The aryl ring may be fused to a heteroaryl, heterocycloalkyl, or cycloalkyl ring, wherein the ring attached to the parent structure is the aryl ring; its non-limiting examples include:

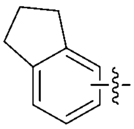 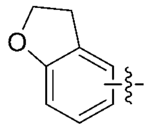 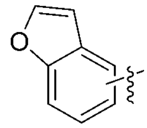 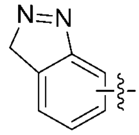 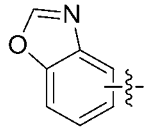 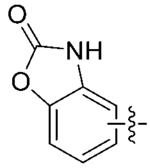

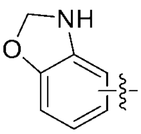 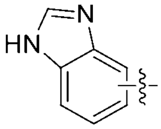 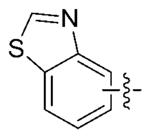 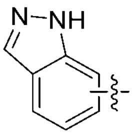 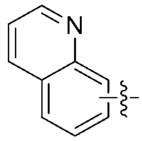 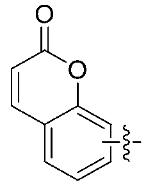

and

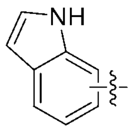.

Aryl may be substituted or unsubstituted.

The term "heteroaryl" refers to a heteroaromatic system containing 1 to 4 heteroatoms and 5 to 14 ring atoms, wherein the heteroatoms are selected from the group consisting of oxygen, sulfur, and nitrogen. Heteroaryl is preferably 6- to 12-membered, more preferably 6- to 10-membered, and more preferably 5- or 6-membered. Its non-limiting examples include imidazolyl, furanyl, thienyl, thiazolyl, pyrazolyl, oxazolyl, isoxazolyl, pyrrolyl, tetrazolyl, pyridyl, pyrimidinyl, thiadiazole, pyrazinyl, triazolyl, indazolyl, benzimidazolyl,

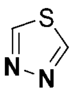, 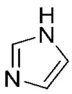, 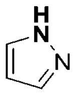,

etc.

The heteroaryl ring may be fused to an aryl, heterocycloalkyl, or cycloalkyl ring, wherein the ring attached to the parent structure is the heteroaryl ring; its non-limiting examples include:

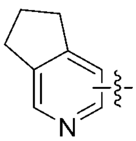 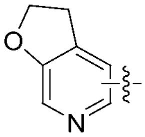 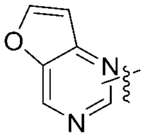 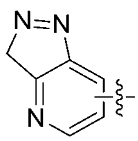 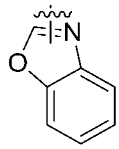

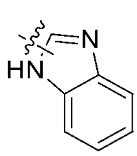 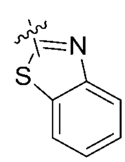 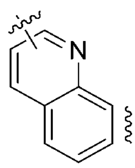 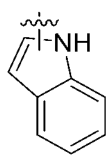.

Heteroaryl may be optionally substituted or unsubstituted.

The term "hydroxy" refers to the -OH group.

The term "halogen" refers to fluorine, chlorine, bromine, or iodine.

The term "cyano" refers to -CN.

The term "amino" refers to $-NH_2$.

The term "nitro" refers to $-NO_2$.

The term "oxo" refers to the =O substituent.

"Substituted" means that one or more, preferably up to 5, and more preferably 1 to 3, hydrogen atoms in the group

are independently substituted with a corresponding number of substituents. When the substituent is a ketone or oxo (i.e., =O), two (2) hydrogen atoms on the atom are replaced.

## DETAILED DESCRIPTION

The present disclosure is further described below with reference to examples, but these examples are not intended to limit the scope of the present disclosure.

Experimental methods without specific conditions indicated in the examples of the present disclosure were generally conducted under conventional conditions, or conditions recommended by the manufacturers of the starting materials or commercial products. Reagents without specific sources indicated were commercially available conventional reagents.

The structures of compounds were determined by nuclear magnetic resonance (NMR) spectroscopy and/or mass spectrometry (LCMS). NMR shifts (δ) are given in $10^{-6}$ (ppm). NMR analysis was performed using a Bruker AVANCE-400 nuclear magnetic resonance instrument, with dimethyl sulfoxide-D6 (DMSO-$d_6$), chloroform-D ($CDCl_3$), and methanol-D4 ($CD_3OD$) as solvents and tetramethylsilane (TMS) as an internal standard. The spatial configurations of the optical isomers (isomers) of compounds can be further confirmed by measuring single-crystal parameters.

HPLC analysis was performed using a Waters ACQUITY ultra high performance LC, Shimadzu LC-20A systems, Shimadzu LC-2010HT series, or Agilent 1200 LC high performance liquid chromatograph (ACQUITY UPLC BEH C18 1.7 μm 2.1 × 50 mm chromatography column, Ultimate XB-C18 3.0 × 150 mm chromatography column, or Xtimate C18 2.1 × 30 mm chromatography column).

MS analysis was performed using a Waters SQD2 mass spectrometer in positive/negative ion scan mode with a mass scan range of 100-1200.

The thin-layer chromatography silica gel plates used were Yantai Huanghai HSGF254 or Qingdao GF254 silica gel plates. The silica gel plates used in the thin-layer chromatography (TLC) had a layer thickness of 0.15 mm-0.2 mm, and those used in thin-layer chromatography separation and purification had a layer thickness of 0.4 mm-0.5 mm.

The flash column purification system used was Combiflash Rf150 (TELEDYNE ISCO) or Isolara one (Biotage).

The normal-phase column chromatography generally used a 100-200 mesh, 200-300 mesh, or 300-400 mesh Yantai Huanghai silica gel as the carrier, or used a Changzhou Santai pre-fill ultrapure normal-phase silica gel column (40-63 μm, 60 Å, 12 g, 25 g, 40 g, 80 g, or other specifications).

The reversed-phase column chromatography generally used a Changzhou Santai pre-fill ultrapure C18 silica gel column (20-45 μm, 100 Å, 40 g, 80 g, 120 g, 220 g, or other specifications).

The high-pressure column purification system used was Waters AutoP equipped with a Waters XBridge BEH C18 OBD Prep Column, 130 Å, 5 μm, 19 mm × 150 mm or Atlantis T3 OBD Prep Column, 100 Å, 5 μm, 19 mm × 150 mm.

Known starting materials in the present disclosure may be synthesized using or according to methods known in the art, or may be purchased from Shanghai Titan Scientific, ABCR GmbH & Co. KG, Acros Organics, Aldrich Chemical Company, Accela ChemBio Inc., Darui Chemicals, and other companies.

In the examples, reactions can all be performed in a nitrogen atmosphere unless otherwise specified.

An argon atmosphere or a nitrogen atmosphere means that the reaction flask is connected to a balloon containing about 1 L of argon or nitrogen.

A hydrogen atmosphere means that the reaction flask is connected to a balloon containing about 1 L of hydrogen.

Pressurized hydrogenation reactions were performed using a Parr 3916EKX hydrogenation instrument and a Qinglan QL-500 hydrogen generator, or an HC2-SS hydrogenation instrument.

Hydrogenation reactions generally involved 3 cycles of vacuumization and hydrogen filling.

Microwave reactions were performed using a CEM Discover-S 908860 microwave reactor.

In the examples, solutions were aqueous solutions unless otherwise specified.

In the examples, the temperature of a reaction was room temperature, i.e., 20 °C-30 °C, unless otherwise specified.

The reaction processes in the examples were monitored using thin-layer chromatography (TLC). The developing solvents used for reactions, the eluent systems of column chromatography used for compound purification, and the developing solvent systems of thin-layer chromatography included: A: a dichloromethane/methanol system, B: a n-hexane/ethyl acetate system, C: a petroleum ether/ethyl acetate system, D: a petroleum ether/ethyl acetate/methanol system, and E: a petroleum ether/tetrahydrofuran system. The volume ratio of the solvents was adjusted based on the polarity of the compound, or by adding a small amount of basic or acidic reagents such as triethylamine and acetic acid.

The normal-phase column chromatography of the purification processes in the examples could use a silica gel column. The eluent systems included, but were not limited to, A: a dichloromethane/methanol system, and B: a petroleum ether/ethyl acetate system. The volume ratio of the solvents was adjusted based on the polarity of the compound, or by adding a small amount of a basic or acidic reagent such as triethylamine or acetic acid.

The chromatography columns used in the C18 reversed-phase chromatography of the purification processes in

the examples were C18 columns. The eluent systems included, but were not limited to, an acetonitrile/water system. The ratio of the solvents was adjusted as needed based on the polarity of the actual compound, or by adding a small amount of a basic or acidic reagent such as ammonium formate or ammonia water or formic acid.

The solvents used for trituration in the purification processes in the examples included, but were not limited to, dichloromethane, methanol, petroleum ether, ethyl acetate, ethanol, acetonitrile, water, etc. Trituration was performed using a single solvent or a combination of multiple solvents.

**Example 1**

(S)-4-(4-((5-Chloro-4-(8-fluoro-2-(2-hydroxypropan-2-yl)-3-methyl-3,4-dihydro-5-oxa-1,2a-diazaacenaphthylen-6-yl) pyrimidin-2-yl)amino)-3-fluorophenyl)morpholin-3-one **(1)**

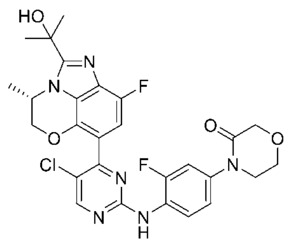

**1**

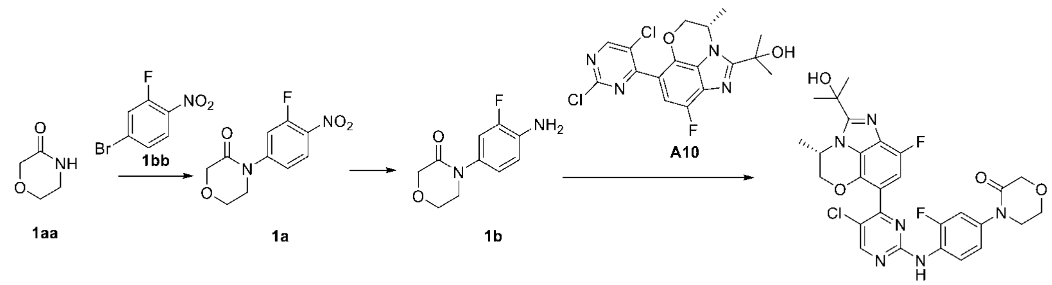


**1**

Step 1: 4-(3-fluoro-4-nitrophenyl)morpholin-3-one **(1a)**

**1bb**, namely 4-bromo-2-fluoro-1-nitrobenzene, (5 g, 22.7 mmol) was added to dioxane (40 mL), and **1aa**, namely morpholin-3-one, (2.76 g, 27.3 mmol), cesium carbonate (14.8 g, 45.4 mmol), and XantPhos Pd G3 (645 mg, 0.68 mmol) were added at room temperature. The mixture was purged with nitrogen three times and left to react at 110 °C. The reaction mixture was concentrated, and the residue was separated and purified by column chromatography (petroleum ether/ethyl acetate = 1:1) to give compound **1a** (1.82 g, yield: 33.4%).

Step 2: 4-(4-amino-3-fluorophenyl)morpholin-3-one **(1b)**

Compound **1a** (1.82 g, 7.58 mmol) was added to ethanol (12 mL) and water (3 mL), and iron powder (1.70 g, 30.3 mmol) and ammonium chloride (1.62 g, 30.3 mmol) were added. The mixture was left to react at 80 °C and filtered while hot. The filtrate was poured into water (50 mL) and extracted with ethyl acetate (15 mL × 2). The organic phases were combined, dried, filtered, triturated (petroleum ether/ethyl acetate = 3:1), and filtered to give compound **1b** (1.15 g, yield: 72.3%).

MS(ESI) m/z = 210.2$[M+H]^+$.

Step 3: (S)-4-(4-((5-chloro-4-(8-fluoro-2-(2-hydroxypropan-2-yl)-3-methyl-3,4-dihydro-5-oxa-1,2a-diazaacenaphthylen-6-yl)pyrimidin-2-yl)amino)-3-fluorophenyl)morpholin-3-one **(1)**

Compound **1b** (32 mg, 0.15 mmol) was added to dioxane (4 mL), and compound A10 (50 mg, 0.12 mmol, obtained by synthesis using the method in Patent No. WO2022166799 and resolution), cesium carbonate (78 g, 0.24 mmol), and XantPhos Pd G3 (9 mg, 0.01 mmol) were added at room temperature. The mixture was purged with nitrogen

and left to react in a microwave reactor at 110°C for 1 h. The reaction mixture was filtered and concentrated, and the residue was purified by C18 reversed-phase chromatography (acetonitrile:water = 4:1) to give compound **1** (21.6 mg, yield: 30.0%).

MS(ESI) m/z = 571.2[M+H]$^+$.

$^1$H NMR (400 MHz, DMSO-$d_6$) δ 9.57 (s, 1H), 8.55 (s, 1H), 7.72 (t, 1H), 7.39 (dd, 1H), 7.25 - 7.17 (m, 1H), 6.99 (d, 1H), 5.82 (s, 1H), 5.26-5.22 (m, 1H), 4.50 (d, 1H), 4.28 - 4.18 (m, 3H), 3.97 (dd, 2H), 3.75 (dd, 2H), 1.67 (s, 3H), 1.62 (s, 3H), 1.46 (d, *J* = 6.6 Hz, 3H).

**Example 2**

(S)-4-(3-Fluoro-4-((5-fluoro-4-(8-fluoro-3-methyl-3,4-dihydro-5-oxa-1,2a-diazaacenaphthylen-6-yl)pyrimidin-2-yl)amino)phenyl)morpholin-3-one (2)

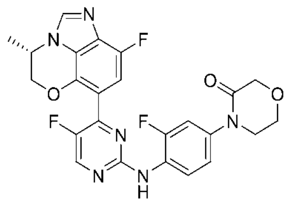

2

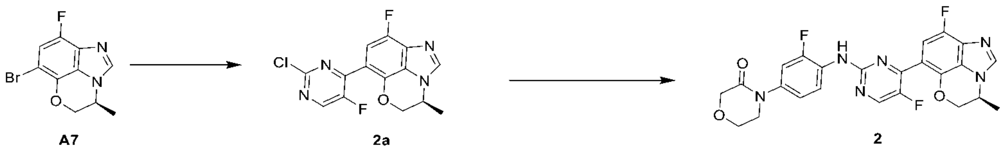

A7 2a 2

Step 1: (S)-6-(2-chloro-5-fluoropyrimidin-4-yl)-8-fluoro-3-methyl-3,4-dihydro-5-oxa-1,2a-diazaacenaphthylene **(2a)**

**A7** (27 mg, 0.1 mmol, obtained by synthesis using the method in Patent No. WO2022166799 and resolution) was dissolved in 1,4-dioxane (1 mL), and bis(pinacolato)diboron (37.5 mg, 0.15 mmol), potassium acetate (20 mg, 0.2 mmol), and Pd(dppf)$Cl_2$ (7.0 mg, 0.01 mmol) were added in a nitrogen atmosphere. The mixture was heated to 100 °C and left to react for 120 min. The reaction mixture was cooled to room temperature, and 2,4-dichloro-5-fluoropyrimidine (16.5 mg, 0.1 mmol), sodium carbonate (21 mg, 0.2 mmol), and two drops of water were added. The mixture was purged with nitrogen three times, then heated to 110 °C, and left to react for 1.5 h. After the reaction was complete, the reaction mixture was concentrated. Water (5 mL) was added to the reaction mixture, and extraction was performed with ethyl acetate (10 mL × 2). The organic phases were combined, dried, and concentrated, and the residue was separated by column chromatography to give compound **2a** (17 mg, yield: 52.6%). MS(ESI) m/z = 323.2[M+H]$^+$.

Step 2: (S)-4-(3-fluoro-4-((5-fluoro-4-(8-fluoro-3-methyl-3,4-dihydro-5-oxa-1,2a-diazaacenaphthylen-6-yl)pyrimidin-2-yl)amino)phenyl)morpholin-3-one **(2)**

Compound **2a** (17 mg, 0.05 mmol) was dissolved in 1,4-dioxane (1 mL), and 4-(4-amino-3-fluorophenyl) morpholin-3-one **(1b)** (11 mg, 0.05 mmol), cesium carbonate (32 mg, 0.1 mmol), and Xantphos Pd G3 (5 mg, 0.005 mmol) were sequentially added. In a nitrogen atmosphere, the mixture was left to react in a microwave reactor at 110 °C. After the reaction mixture was filtered, the residue was separated by C18 reversed-phase chromatography to give compound **2** (4 mg, yield: 16.1%).

MS(ESI) m/z = 497.3[M+H]$^+$.

$^1$H NMR (400 MHz, DMSO) δ 9.37 (s, 1H), 8.61 - 8.50 (m, 2H), 7.78 (t, 1H), 7.38 (dd, 1H), 7.25 - 7.18 (m, 1H), 7.15 (d, 1H), 4.75-4.73 (m, 1H), 4.58 (dd, 1H), 4.20 (s, 2H), 4.16 (dd, 1H), 3.97 (dd, , 2H), 3.75 (dd, 2H), 1.56 (d, 3H).

**Example 3**

(S)-4-((5-Fluoro-4-(8-fluoro-2-(2-hydroxypropan-2-yl)-3-methyl-3,4-dihydro-5-oxa-1,2a-diazaacenaphthylen-6-yl)pyrimidin-2-yl)amino)-N-methylbenzamide (3)

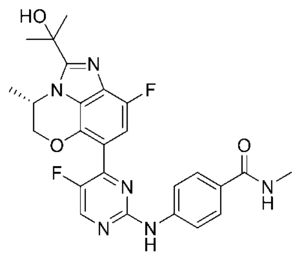

**3**

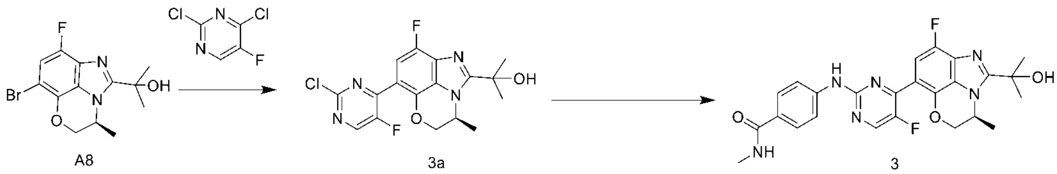

A8

3a

3

Step 1: (S)-2-(6-(2-chloro-5-fluoropyrimidin-4-yl)-8-fluoro-3-methyl-3,4-dihydro-5-oxa-1,2a-diazaacenaphthylen-2-yl) propan-2-ol **(3a)**

**A8** (33 mg, 0.1 mmol, obtained by synthesis using the method in Patent No. WO2022166799 and resolution) was dissolved in 1,4-dioxane (1 mL), and bis(pinacolato)diboron (37.5 mg, 0.15 mmol), potassium acetate (20 mg, 0.2 mmol), and Pd(dppf)$Cl_2$ (7.0 mg, 0.01 mmol) were added in a nitrogen atmosphere. The mixture was stirred at 100 °C for 120 min. The reaction mixture was cooled to room temperature, and 2,4-dichloro-5-fluoropyrimidine (16.5 mg, 0.1 mmol), sodium carbonate (21 mg, 0.2 mmol), and two drops of water were added. The mixture was purged with nitrogen three times and then left to react at 110 °C for 1.5 h. After the reaction was complete, the reaction mixture was concentrated. Water (5 mL) was added to the reaction mixture, and extraction was performed with ethyl acetate (10 mL × 2). The organic phases were combined, dried, and concentrated, and the residue was separated by column chromatography to give compound **3a** (21.5 mg, yield: 56.5%).

MS(ESI) m/z = 381.2$[M+H]^+$.

Step 2: (S)-4-((5-fluoro-4-(8-fluoro-2-(2-hydroxypropan-2-yl)-3-methyl-3,4-dihydro-5-oxa-1,2a-diazaacenaphthylen-6-yl)pyrimidin-2-yl)amino)-N-methylbenzamide **(3)** The target compound **3** was synthesized by using the method in Example 2 and replacing 4-(4-amino-3-fluorophenyl)morpholin-3-one with 4-amino-*N*-methylbenzamide.

MS(ESI) m/z = 495.3$[M+H]^+$.

$^1$H NMR (400 MHz, DMSO) δ 10.08 (s, 1H), 8.66 (d, 1H), 8.24-8.22 (m, 1H), 7.90 - 7.84 (m, 2H), 7.81 - 7.73 (m, 2H), 7.19 (d, 1H), 5.85 (s, 1H), 5.27-5.25 (m, 1H), 4.55 (d, 1H), 4.33 - 4.25 (m, 1H), 2.76 (d, 3H), 1.69 (s, 3H), 1.63 (s, 3H), 1.50 (d, 3H).

## Example 4

(S)-4-(3-Fluoro-4-((5-fluoro-4-(8-fluoro-2-(2-hydroxypropan-2-yl)-3-methyl-3,4-dihydro-5-oxa-1,2a-diazaacenaphthylen-6-yl)pyrimidin-2-yl)amino)phenyl)morpholin-3-one **(4)**

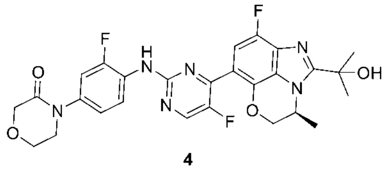

**4**

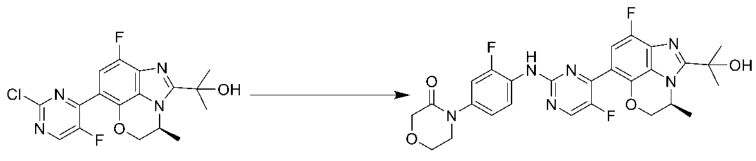

3a

4

The target compound **4** was obtained by using a synthesis method similar to that of Example 3 and replacing 4-amino-N-methylbenzamide with 4-(4-amino-3-fluorophenyl)morpholin-3-one.

MS(ESI) m/z = 555.3[M+H]$^+$.

$^1$H NMR (400 MHz, DMSO) δ 9.36 (s, 1H), 8.55 (d, 1H), 7.79 (t, 1H), 7.38 (dd, 1H), 7.21-7.17 (m, 1H), 7.11 (d, 1H), 5.83 (s, 1H), 5.28 - 5.20 (m, 1H), 4.57 - 4.49 (m, 1H), 4.24 (d, 1H), 4.21 (s, 2H), 3.97 (dd, 2H), 3.78 - 3.71 (m, 2H), 1.67 (s, 3H), 1.62 (s, 3H), 1.47 (d, 3H).

## Example 5

(S)-4-((5-Chloro-4-(8-fluoro-2-(2-hydroxypropan-2-yl)-3-methyl-3,4-dihydro-5-oxa-1,2a-diazaacenaphthylen-6-yl)pyrimidin-2-yl)amino)-N-methylbenzenesulfonamide (5)

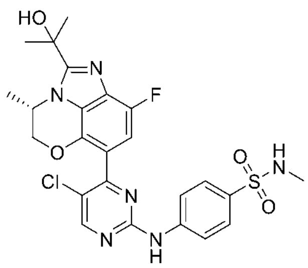

5

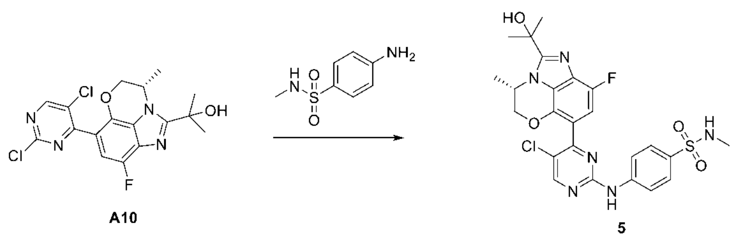

A10

5

4-Amino-N-methylbenzenesulfonamide (28.6 mg, 0.15 mmol) was added to dioxane (4 mL), and A10 (50 mg, 0.13 mmol), cesium carbonate (82 g, 0.25 mmol), and XantPhos Pd G3 (7 mg, 0.007 mmol) were added at room temperature. The mixture was purged with nitrogen and left to react in a microwave reactor at 100 °C for 1 h. After the reaction mixture was filtered and concentrated, the residue was purified by C18 reversed-phase chromatography to give compound 5 (24 mg, yield: 34.9%).

MS(ESI) m/z = 547.2[M+H]$^+$.

$^1$H NMR (400 MHz, DMSO-$d_6$) δ 10.41 (s, 1H), 8.71 (s, 1H), 8.01 - 7.92 (m, 2H), 7.72 - 7.64 (m, 2H), 7.24 (q, 1H), 7.09 (d, 1H), 5.83 (s, 1H), 5.27 (q, 1H), 4.56 - 4.48 (m, 1H), 4.28 (dd, 1H), 2.38 (d, 3H), 1.69 (s, 3H), 1.63 (s, 3H), 1.48 (d, 3H).

## Example 6

(S)-4-(3-Fluoro-4-((5-fluoro-4-(8-fluoro-2,3-dimethyl-3,4-dihydro-5-oxa-1,2a-diazaacenaphthylen-6-yl)pyrimidin-2-yl)amino)phenyl)morpholin-3-one (6)

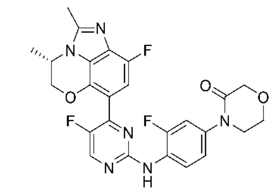

6

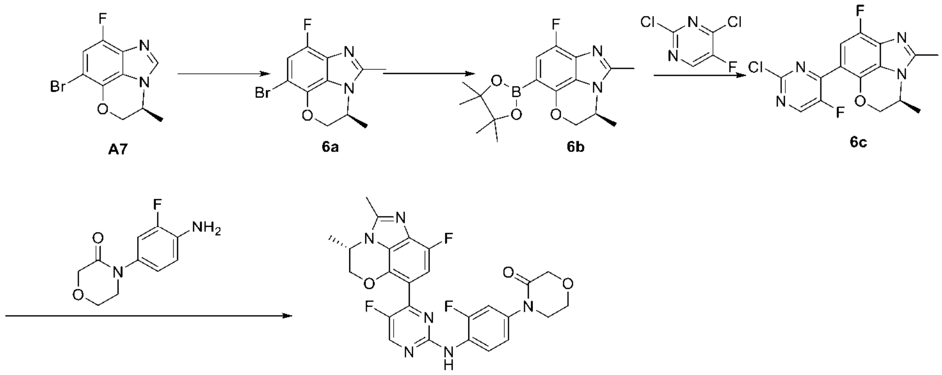

A7 6a 6b 6c

6

Step 1: (S)-6-bromo-8-fluoro-2,3-dimethyl-3,4-dihydro-5-oxa-1,2a-diazaacenaphthylene **(6a)**

Compound **A7** (110 mg, 0.41 mmol) was added to tetrahydrofuran (8 mL). The mixture was purged with nitrogen three times, and LDA (0.35 mL, 0.61 mmol, 2.0 M in THF) was added at -78 °C. After 1 h of stirring, iodomethane (115 mg, 0.81 mmol) was added at the same temperature. After 1 h of reaction at -78 °C, saturated ammonium chloride (50 mL) was added, and the aqueous phase was extracted with ethyl acetate (10 mL × 3). The organic phases were combined and concentrated, and the residue was separated by column chromatography to give compound **6a** (104 mg, yield: 81.0%).

MS(ESI) m/z = 285.2$[M+H]^+$.

Step 2: (S)-8-fluoro-2,3-dimethyl-6-(4,4,5,5-tetramethyl-1,3,2-dioxaborolan-2-yl)-3,4-dihydro-5-oxa-1,2a-diazaacenaphthylene **(6b)**

Compound **6a** (100 mg, 0.35 mmol) was dissolved in 1,4-dioxane (3 mL), and bis(pinacolato)diboron (134 mg, 0.54 mmol), potassium acetate (103 mg, 1.05 mmol), and Pd(dppf)$Cl_2$ (14.5 mg, 0.02 mmol) were sequentially added. In a nitrogen atmosphere, the mixture was left to react in a microwave reactor at 110 °C for 1 h. After the reaction mixture was filtered and concentrated, the residue was separated by column chromatography to give compound **6b** (50 mg, yield: 15.5%).

MS(ESI) m/z = 333.3$[M+H]^+$.

Step 3: (S)-6-(2-chloro-5-fluoropyrimidin-4-yl)-8-fluoro-2,3-dimethyl-3,4-dihydro-5-oxa-1,2a-diazaacenaphthylene **(6c)**

Compound **6b** (50.0 mg, 0.15 mmol) was dissolved in 1,4-dioxane (3 mL) and water (0.05 mL), and 2,4-dichloro-5-fluoropyrimidine (25.0 mg, 0.15 mmol), sodium carbonate (32.0 mg, 0.30 mmol), and Pd(dppf)$Cl_2$ (2.00 mg, 0.003 mmol) were sequentially added. The mixture was purged with nitrogen three times and left to react at 110 °C for 18 h. After the reaction mixture was filtered and concentrated, the residue was separated and purified by column chromatography to give compound **6c** (50 mg, yield: 35.0%).

MS(ESI) m/z = 337.1$[M+H]^+$.

Step 4: (S)-4-(3-fluoro-4-((5-fluoro-4-(8-fluoro-2,3-dimethyl-3,4-dihydro-5-oxa-1,2a-diazaacenaphthylen-6-yl)pyrimidin-2-yl)amino)phenyl)morpholin-3-one **(6)**

Compound **6c** (50.0 mg, 0.15 mmol) was dissolved in 1,4-dioxane (3 mL), and 4-(4-amino-3-fluorophenyl) morpholin-3-one **(1b)** (37.0 mg, 0.18 mmol), cesium carbonate (96.0 mg, 0.30 mmol), and XantPhos Pd G3 (3.00 mg, 0.003 mmol) were sequentially added. In a nitrogen atmosphere, the mixture was left to react in a microwave reactor at 110 °C for 1 h. After the reaction mixture was filtered and concentrated, the residue was purified by C18 reversed-phase chromatography to give compound **6** (3.60 mg, yield: 4.8%).

MS(ESI) m/z = 511.1[M+H]+.

**Example 7**

(S)-4-((5-Chloro-4-(8-fluoro-2-(2-hydroxypropan-2-yl)-3-methyl-3,4-dihydro-5-oxa-1,2a-diazaacenaphthylen-6-yl)pyrimidin-2-yl)amino)-3-fluoro-N-methylbenzenesulfonamide **(7)**

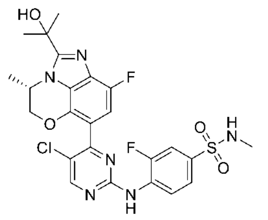

**7**

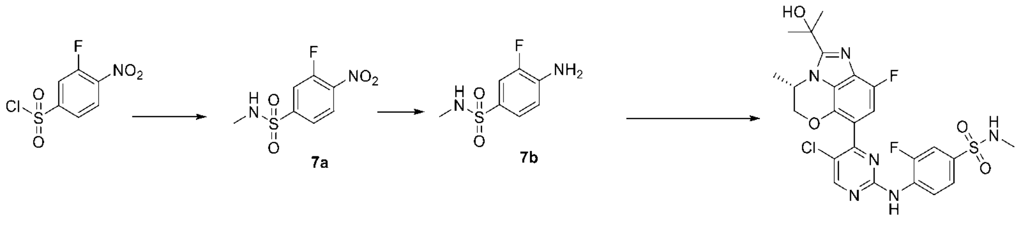

7a 7b 7

Step 1: 3-fluoro-N-methyl-4-nitrobenzenesulfonamide **(7a)**

3-Fluoro-4-nitrobenzenesulfonyl chloride (300 mg, 1.25 mmol) was added to dichloromethane (10 mL) and water (3 mL), and methylamine hydrochloride (127 mg, 1.88 mmol) and potassium carbonate (173 g, 1.25 mmol) were added under ice-bath conditions. The mixture was left to react for 1 h. The reaction mixture was poured into water (50 mL) and extracted with dichloromethane (15 mL × 3). The organic phases were combined and concentrated, and the residue was separated by column chromatography to give compound **7a** (234 mg, yield: 80.1%).

Step 2: 4-amino-3-fluoro-N-methylbenzenesulfonamide **(7b)**

Compound **7a** (234 mg, 1.00 mmol) was added to ethanol (12 mL) and water (3 mL), and iron powder (224 mg, 4.00 mmol) and ammonium chloride (218 mg, 4.00 mmol) were added. The mixture was left to react at 80 °C for 2 h and then filtered while hot. The filtrate was poured into water (50 mL) and extracted with ethyl acetate (15 mL × 3). The organic phases were combined, dried, filtered, concentrated, and triturated to give compound **7b** (163 mg, yield: 80.2%).
MS(ESI) m/z = 205.2[M+H]+.

Step 3: (S)-4-((5-chloro-4-(8-fluoro-2-(2-hydroxypropan-2-yl)-3-methyl-3,4-dihydro-5-oxa-1,2a-diazaacenaphthylen-6-yl)pyrimidin-2-yl)amino)-3-fluoro-N-methylbenzenesulfonamide (7)

Compound **7b** (25.7 mg, 0.13 mmol) was added to dioxane (4 mL), and **A10** (50 mg, 0.13 mmol), cesium carbonate (85 mg, 0.26 mmol), and XantPhos Pd G3 (6 mg, 0.006 mmol) were added at room temperature. In a nitrogen atmosphere, the mixture was left to react in a microwave reactor at 100 °C for 1 h. After the reaction mixture was filtered and concentrated, the residue was purified by C18 reversed-phase chromatography to give compound **7** (30 mg, yield: 42.2%).
MS(ESI) m/z = 565.2[M+H]+.
$^1$H NMR (400 MHz, DMSO-$d_6$) δ 9.90 (s, 1H), 8.67 (s, 1H), 8.18 (t, 1H), 7.63 - 7.53 (m, 2H), 7.45 (s, 1H), 7.04 (d, 1H), 5.82 (s, 1H), 5.26 (q, 1H), 4.56 - 4.48 (m, 1H), 4.26 (dd, 1H), 2.43 (s, 3H), 1.68 (s, 3H), 1.62 (s, 3H), 1.47 (d, 3H).

**Example 8**

(4-((5-Chloro-4-((S)-8-fluoro-2-(2-hydroxypropan-2-yl)-3-methyl-3,4-dihydro-5-oxa-1,2a-diazaacenaphthylen-6-yl)pyrimidin-2-yl)amino)-3-fluorophenyl)(cyclopropyl)sulfonimide **(8)**

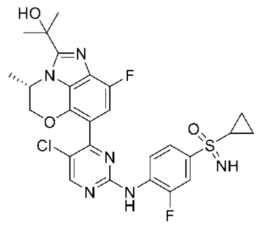

8

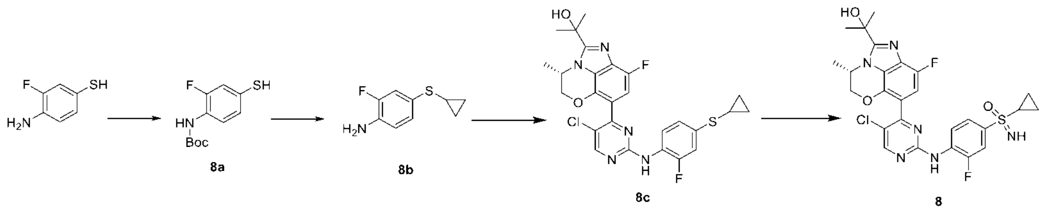

8a 8b 8c 8

Step 1: tert-butyl (2-fluoro-4-mercaptophenyl)carbamate **(8a)**

4-Amino-3-fluorobenzene-1-thiol (143 mg, 1.0 mmol) was dissolved in ethanol (5 mL), and DMAP (12.2 mg, 0.10 mmol) and di-tert-butyl dicarbonate (310 mg, 1.5 mmol) were added. The mixture was left to react at room temperature for 60 min. After the reaction was complete, the reaction mixture was concentrated. Water (5 mL) was added to the reaction mixture, and extraction was performed with ethyl acetate (10 mL × 2). The organic phases were combined, dried, and concentrated, and the residue was separated by column chromatography to give compound **8a** (200 mg, yield: 82.3%).

Step 2: 4-(cyclopropylthio)-2-fluoroaniline **(8b)**

Compound **8a** (120 mg, 0.5 mmol) was dissolved in tert-butanol (2.5 mL), and potassium tert-butoxide (112 mg, 1.0 mmol) was added. Bromocyclopropane (120 mg, 1.0 mmol) was slowly added with stirring. In a nitrogen atmosphere, the mixture was left to react in a microwave reactor at 120 °C for 2 h. After the reaction mixture was filtered, water (5 mL) was added, and extraction was performed with ethyl acetate (10 mL × 2). The organic phases were combined, dried, and concentrated, and the residue was directly used in the next step.
MS(ESI) m/z = 184.3[M+H]$^+$.

Step 3: (S)-2-(6-(5-chloro-2-((4-(cyclopropylthio)-2-fluorophenyl)amino)pyrimidin-4-yl)-8-fluoro-3-methyl-3,4-dihydro-5-oxa-1,2a-diazaacenaphthylen-2-yl)propan-2-ol **(8c)**

**8c** was synthesized by using a synthesis method similar to that of Example 1 and replacing 4-(4-amino-3-fluorophenyl)morpholin-3-one with compound **8b.**
MS(ESI) m/z = 544.2[M+H]$^+$.

Step 4: (4-((5-chloro-4-((S)-8-fluoro-2-(2-hydroxypropan-2-yl)-3-methyl-3,4-dihydro-5-oxa-1,2a-diazaacenaphthylen-6-yl)pyrimidin-2-yl)amino)-3-fluorophenyl)(cyclopropyl)sulfonimide **(8)**

Compound **8c** (27 mg, 0.05 mmol) was dissolved in ethanol (1 mL), and iodobenzene diacetate (80 mg, 0.25 mmol) and ammonium acetate (38 mg, 0.5 mmol) were added. The mixture was left to react at room temperature for 1 h. After the reaction was complete, the reaction mixture was purified and separated by C18 reversed-phase chromatography to give the target compound **8** (10 mg, yield: 34.8%).
MS(ESI) m/z = 575.2[M+H]$^+$.
$^1$H NMR (400 MHz, DMSO) δ 9.89 (s, 1H), 8.66 (s, 1H), 8.13 (t, 1H), 7.72 - 7.63 (m, 2H), 7.04 (d, 1H), 5.83 (s, 1H), 5.26 (d, 1H), 4.52 (d, 1H), 4.30-4.26 (m, 1H), 2.70-2.66 (m, 1H), 1.68 (s, 3H), 1.62 (s, 3H), 1.47 (d, 3H), 1.11-1.07 (m, 1H), 1.01 - 0.90 (m, 2H), 0.89-0.79 (m, 2H).

**Example 9**

4-((5-Chloro-4-((S)-8-fluoro-2-(2-hydroxypropan-2-yl)-3-methyl-3,4-dihydro-5-oxa-1,2a-diazaacenaphthylen-6-yl)pyrimidin-2-yl)amino)-3-fluoro-N-methylbenzenesulfonimide **(9)**

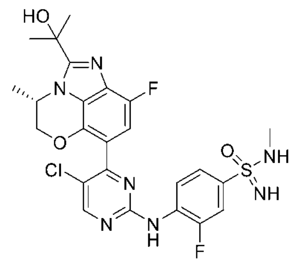

**9**

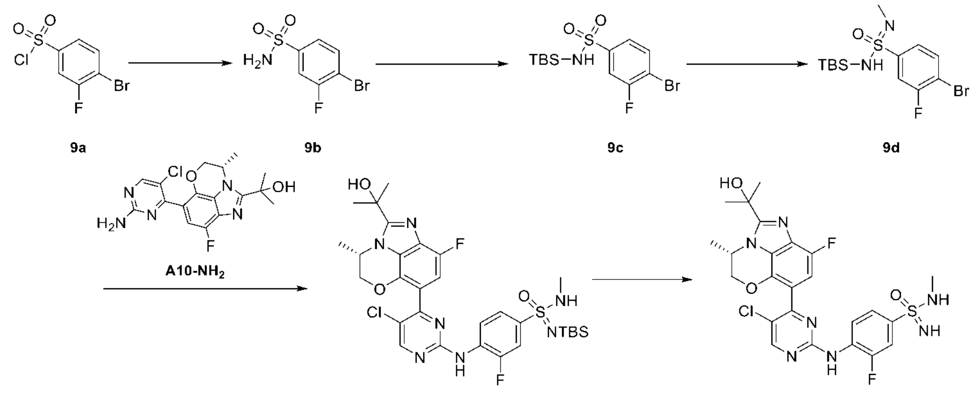

9a 9b 9c 9d A10-NH$_2$ 9e 9

Steps for synthesis of intermediate A10-NH$_2$

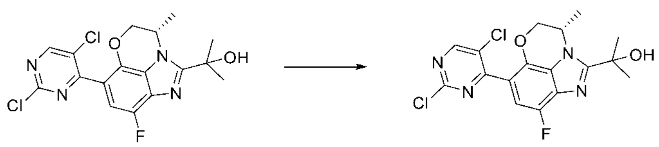

A10 A10-NH$_2$

Step 1: 4-bromo-3-fluorobenzene-1-sulfonamide **(9b)**

4-Bromo-3-fluorobenzenesulfonyl chloride (9a) (1.0 g, 3.65 mmol) was added to acetonitrile (10 mL), and the mixture was cooled to 0 °C. Ammonia water (1.42 mL, 10.97 mmol) was added, and the mixture was stirred at 0 °C for 30 min. The reaction mixture was poured into water (20 mL) and extracted with ethyl acetate (20 mL × 3). The organic phases were combined, dried, and filtered. The filtrate was concentrated, and the residue was separated by column chromatography to give compound **9b** (0.9 g, yield: 96.8%).

Step 2: 4-bromo-N-(tert-butyldimethylsilyl)-3-fluorobenzenesulfonamide **(9c)**

Compound **9b** (500 mg, 1.96 mmol) was added to anhydrous tetrahydrofuran (10 mL). In a nitrogen atmosphere, the mixture was cooled to 0 °C, and 60% sodium hydride (197.1 mg, 4.92 mmol) was added in batches. After 30 min of reaction, tertbutyldimethylsilyl chloride (357.5 mg, 2.36 mmol) was added. The mixture was warmed to room temperature and stirred for 3 h. Water (10 mL) was added to the reaction mixture, and extraction was performed with ethyl acetate (10 mL × 3). The organic phases were combined, washed with saturated brine (10 mL), and dried. The filtrate was

concentrated, and the residue was separated by column chromatography to give compound **9c** (600 mg, yield: 82.8%).
MS(ESI) m/z = 368.1[M+H]$^+$.

Step 3: 4-bromo-N-(tert-butyldimethylsilyl)-3-fluoro-N'-methylbenzenesulfonimide **(9d)**

Compound **9c** (150 mg, 0.41 mmol) was dissolved in chloroform (10 mL). In a nitrogen atmosphere, triethylamine (0.23 mL, 1.63 mmol) was added at room temperature. After 30 min of stirring at room temperature, triphenylphosphine dichloride (272.1 mg, 0.81 mmol) was added. After 30 min of reaction at room temperature, a 30% solution of methylamine in ethanol (462.8 mg, 4.48 mmol) was added. The mixture was left to react at room temperature for 16 h. Water (20 mL) was added to the reaction mixture, and extraction was performed with dichloromethane (10 mL × 3). The organic phases were combined, washed with saturated brine (10 mL), and dried. The filtrate was concentrated, and the residue was separated by column chromatography to give compound **9d** (80 mg, yield: 51.5%).
MS(ESI) m/z = 381.0[M+H]$^+$.

Step 4: (S)-2-(6-(2-amino-5-chloropyrimidin-4-yl)-8-fluoro-3-methyl-3,4-dihydro-5-oxa-1,2a-diazaacenaphthylen-2-yl) propan-2-ol **(A10-NH$_2$)**

A10 was dissolved in a 7 mol/L solution of ammonia in methanol (2 mL), and the mixture was left to react at 70 °C for 3 h. After the reaction mixture was concentrated, the residue was separated by column chromatography to give **A10-NH$_2$** (30 mg, yield: 63.1%).
MS(ESI) m/z = 378.1[M+H]$^+$.

Step 5: N'-(tert-butyldimethylsilyl)-4-((5-chloro-4-((S)-8-fluoro-2-(2-hydroxypropan-2-yl)-3-methyl-3,4-dihydro-5-oxa-1,2a-diazaacenaphthylen-6-yl)pyrimidin-2-yl)amino)-3-fluoro-N-methylbenzenesulfonimide **(9e)**

Compound **9d** (10 mg, 0.03 mmol) was dissolved in 1,4-dioxane (1.5 mL), and A10-NH$_2$ (9.9 mg, 0.03 mmol), cesium carbonate (17.1 mg, 0.05 mmol), and Brettphos Pd G3 (4.8 mg, 0.005 mmol) were sequentially added. In a nitrogen atmosphere, the mixture was left to react in a microwave reactor at 100 °C for 1 h. After the reaction mixture was concentrated, the residue was separated by column chromatography to give compound **9e** (10 mg, yield: 56.2%).
MS(ESI) m/z = 678.2[M+H]$^+$.

Step 6: 4-((5-chloro-4-((S)-8-fluoro-2-(2-hydroxypropan-2-yl)-3-methyl-3,4-dihydro-5-oxa-1,2a-diazaacenaphthylen-6-yl)pyrimidin-2-yl)amino)-3-fluoro-N-methylbenzenesulfonimide **(9)**

Compound **9e** (25 mg, 0.03 mmol) was dissolved in tetrahydrofuran (2 mL), and tetrabutylammonium fluoride (0.18 mL, 0.18 mmol) was added at room temperature. The mixture was left to react at room temperature for 1 h. Water (10 mL) was added to the reaction mixture, and extraction was performed with ethyl acetate (5 mL × 3). The organic phases were combined, washed with saturated brine (10 mL), and dried. The filtrate was concentrated, and the residue was purified by C18 reversed-phase chromatography to give compound **9** (7.1 mg, yield: 34.1%).
MS(ESI) m/z = 564.2[M+H]$^+$.

**Example 10**

(S)-4-(4-((5-Chloro-4-(8-fluoro-2-(2-hydroxypropan-2-yl)-3-methyl-3,4-dihydro-5-oxa-1,2a-diazaacenaphthylen-6-yl) pyrimidin-2-yl)amino)phenyl)morpholin-3-one **(10)**

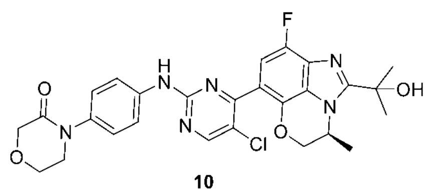

**10**

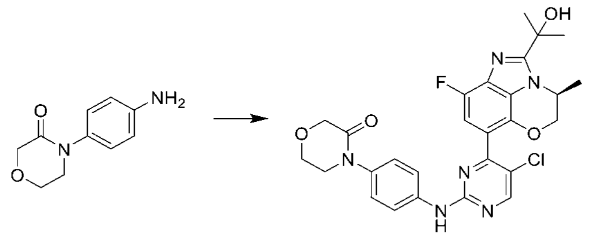

10

The target compound **10** was obtained by using a synthesis method similar to that of Example 1 and using 4-(4-aminophenyl)morpholin-3-one (commercially available, CAS: 438056-69-0) and **A10** as the starting materials.

MS(ESI) m/z = 553.4[M+H]+.

$^1$H NMR (400 MHz, DMSO) δ 9.96 (s, 1H), 8.55 (s, 1H), 7.73 - 7.66 (m, 2H), 7.25 - 7.17 (m, 2H), 6.99 (d, 1H), 5.76 (s, 1H), 5.21-5.19 (m, 1H), 4.44 (d, 1H), 4.20-4.16 (m, 1H), 4.11 (s, 2H), 3.89 (dd, 2H), 3.62 (dd, 2H), 1.61 (s, 3H), 1.56 (s, 3H), 1.41 (d, *J* = 6.6 Hz, 3H).

**Example 11**

(S)-4-((5-Chloro-4-(8-fluoro-2-(2-hydroxypropan-2-yl)-3-methyl-3,4-dihydro-5-oxa-1,2a-diazaacenaphthylen-6-yl)pyrimidin-2-yl)amino)-N-methylbenzamide **(11)**

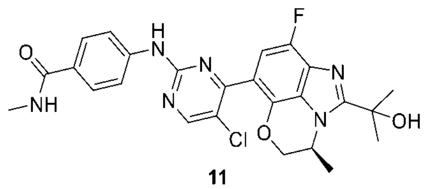

11

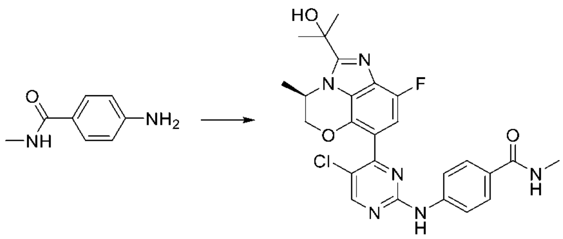

11

The target compound **11** was obtained by using a synthesis method similar to that of Example 1 and using 4-amino-N-methylbenzamide and **A10** as the starting materials.

MS(ESI) m/z = 511.4[M+H]+.

$^1$H NMR (400 MHz, DMSO) δ 10.22 (s, 1H), 8.67 (s, 1H), 8.28-8.25 (m, 1H), 7.84 (d, 2H), 7.76 (d, 2H), 7.08 (d, 1H), 5.84 (s, 1H), 5.35 - 5.23 (m, 1H), 4.52 (d, 1H), 4.27 (dd, 1H), 2.76 (d, 3H), 1.69 (s, 3H), 1.63 (s, 3H), 1.48 (d, 3H).

**Example 12**

1-(4-((5-Chloro-4-((S)-8-fluoro-2-(2-hydroxypropan-2-yl)-3-methyl-3,4-dihydro-5-oxa-1,2a-diazaacenaphthylen-6-yl) pyrimidin-2-yl)amino)-3-fluorophenyl)-4-(dimethylamino)piperidin-2-one **(12)**

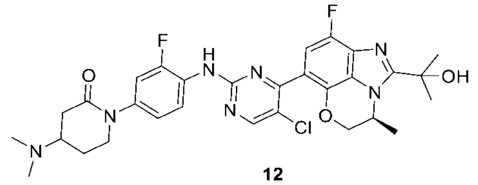

12

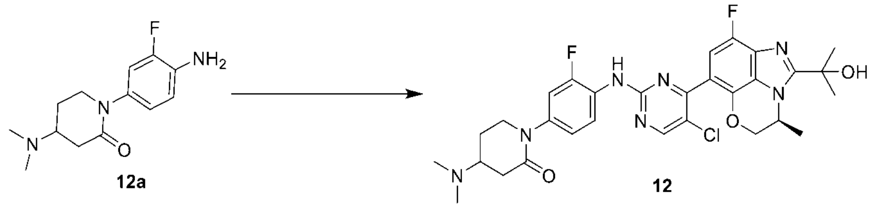

12a 12

The target compound **12** was obtained by referring to the synthesis method of Example 1 and replacing 4-(4-amino-3-fluorophenyl)morpholin-3-one with 1-(4-amino-3-fluorophenyl)-4-(dimethylamino)-2-piperidone (synthesized using a method similar to that in Patent No. CN114364675A).

MS(ESI) m/z = 612.2[M+H]$^+$.

**Example 13**

(S)-4-(8-Fluoro-2-(2-hydroxypropan-2-yl)-3-methyl-3,4-dihydro-5-oxa-1,2a-diazaacenaphthylen-6-yl)-2-((2-fluoro-4-(3-oxomorpholino)phenyl)amino)pyrimidine-5-carbonitrile **(13)**

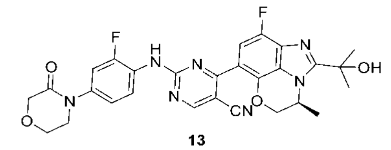

13

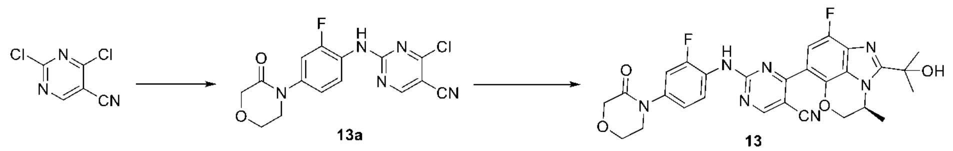

13a 13

Step 1: 4-chloro-2-((2-fluoro-4-(3-oxomorpholino)phenyl)amino)pyrimidine-5-carbonitrile **(13a)**

2,4-Dichloropyrimidine-5-carbonitrile (250 mg, 1.44 mmol) was added to tert-butanol (5 mL) and 1,2-dichloroethane (5 mL), and zinc dichloride (207 mg, 1.52 mmol) and triethylamine (58.6 mg, 0.52 mmol) were added under ice-bath conditions. The mixture was purged with nitrogen three times and stirred under ice-bath conditions for 1 h, and 4-(4-amino-3-fluorophenyl)morpholin-3-one **(1b)** (101 mg, 0.48 mmol) was added. The mixture was left to react at 80 °C for 18 h. Water (50 mL) was added, and the aqueous phase was extracted with ethyl acetate (10 mL × 3). The organic phases were combined and concentrated, and the residue was separated by column chromatography to give compound **13a** (119 mg, yield: 71.1%).

MS(ESI) m/z = 348.1[M+H]$^+$.

Step 2: (S)-4-(8-fluoro-2-(2-hydroxypropan-2-yl)-3-methyl-3,4-dihydro-5-oxa-1,2a-diazaacenaphthylen-6-yl)-2-((2-fluoro-4-(3-oxomorpholino)phenyl)amino)pyrimidine-5-carbonitrile **(13)**

Compound **13a** (33.0 mg, 0.10 mmol) was dissolved in 1,4-dioxane (3 mL) and water (3 drops), and **A8** (75.0 mg, 0.20 mmol), sodium carbonate (21.2 mg, 0.2 mmol), and Pd(dppf)$Cl_2$ (4.00 mg, 0.005 mmol) were sequentially added. In a nitrogen atmosphere, the mixture was left to react in a microwave reactor at 100 °C for 1.5 h. After the reaction mixture was filtered and concentrated, the residue was purified by C18 reversed-phase chromatography to give compound **13** (14.2 mg, yield: 26.7%).

MS(ESI) m/z = 562.3[M+H]$^+$.

$^1$H NMR (400 MHz, DMSO-$d_6$) δ 10.27 (s, 1H), 8.88 (s, 1H), 7.65 (s, 1H), 7.46 (dd, 1H), 7.30-7.26 (m, 1H), 7.09 (d, 1H), 5.83 (s, 1H), 5.28 (d, 1H), 4.55 (d, 1H), 4.29 (d, 1H), 4.22 (s, 2H), 3.98 (dd, 2H), 3.81 - 3.73 (m, 2H), 1.65 (d, 6H), 1.48 (d, *J* = 6.6 Hz, 3H).

**Example 14**

(4-((5-Chloro-4-((S)-8-fluoro-2-(2-hydroxypropan-2-yl)-3-methyl-3,4-dihydro-5-oxa-1,2a-diazaacenaphthylen-6-yl)pyrimidin-2-yl)amino)-3-fluorophenyl)(methyl)sulfonimide **(14)**

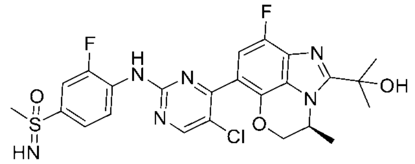

**14**

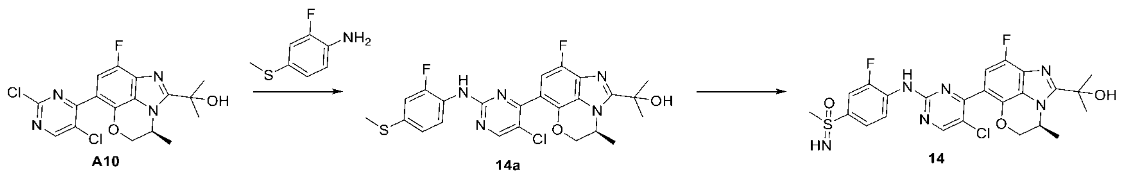

A10 14a 14

Step 1: (S)-2-(6-(5-chloro-2-((2-fluoro-4-(methylthio)phenyl)amino)pyrimidin-4-yl)-8-fluoro-3-methyl-3,4-dihydro-5-oxa-1,2a-diazaacenaphthylen-2-yl)propan-2-ol **(14a)** Compound **A10** (55 mg, 0.14 mmol) was dissolved in 1,4-dioxane (1 mL), and 2-fluoro-4-methylthioaniline (23.9 mg, 0.15 mmol), cesium carbonate (90 mg, 0.28 mmol), and XantPhos Pd G3 (13.1 mg, 0.014 mmol) were sequentially added. The mixture was left to react in a microwave reactor at 110 °C for 1 h. After the reaction was complete, the reaction mixture was cooled to room temperature, and the residue was separated by column chromatography to give compound **14a** (65 mg, yield: 90.6%).

Step 2: (4-((5-chloro-4-((S)-8-fluoro-2-(2-hydroxypropan-2-yl)-3-methyl-3,4-dihydro-5-oxa-1,2a-diazaacenaphthylen-6-yl)pyrimidin-2-yl)amino)-3-fluorophenyl)(methyl)sulfonimide **(14)**

Compound **14a** (60 mg, 0.12 mmol) was dissolved in ethanol (1 mL), and iodobenzene diacetate (111.9 mg, 0.35 mmol) and ammonium acetate (44.6 mg, 0.58 mmol) were sequentially added. The mixture was left to react overnight at room temperature. After the reaction was complete, the reaction mixture was separated by C18 reversed-phase chromatography to give compound **14** (11 mg, yield: 17.3%).

MS(ESI) m/z = 549.27[M+H]$^+$.

$^1$H NMR (400 MHz, DMSO$d_6$) δ 9.91 (s, 1H), 8.66 (s, 1H), 8.14 (t, 1H), 7.76 - 7.69 (m, 2H), 7.04 (d, 1H), 5.83 (s, 1H), 5.285.24 (m, 1H), 4.52 (d, J = 11.4 Hz, 1H), 4.26 (d, 2H), 3.08 (d, 3H), 1.65 (d, 6H), 1.47 (d, 3H).

**Example 15**

(3-Fluoro-4-((5-fluoro-4-((S)-8-fluoro-2-(2-hydroxypropan-2-yl)-3-methyl-3,4-dihydro-5-oxa-1,2a-diazaacenaphthylen-6-yl)pyrimidin-2-yl)amino)phenyl)(methyl)sulfonimide **(15)**

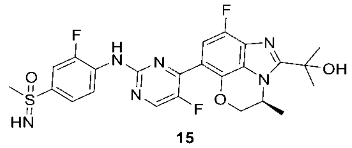

**15**

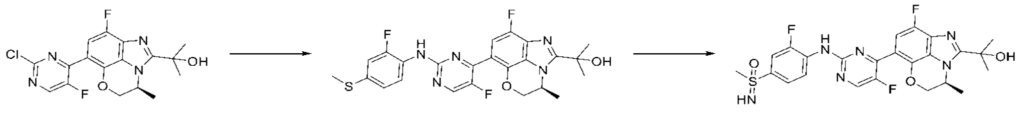

3a 15a 15

The target compound **15** was obtained by using a synthesis method similar to that of Example 14 and replacing compound A10 with compound 3a.

MS(ESI) m/z = 533.22[M+H]+.

$^1$H NMR (400 MHz, DMSO$d_6$) δ 9.71 (s, 1H), 8.66 (d, 1H), 8.29 - 8.24 (m, 1H), 7.76 - 7.68 (m, 2H), 7.16 (d, 1H), 5.85 (s, 1H), 5.27 (q, 1H), 4.58 - 4.52 (m, 1H), 4.31 - 4.21 (m, 2H), 3.09 (s, 3H), 1.68 (s, 3H), 1.62 (s, 3H), 1.48 (d, 3H).

### Example 16

(S)-3-Fluoro-4-((5-fluoro-4-(8-fluoro-2-(2-hydroxypropan-2-yl)-3-methyl-3,4-dihydro-5-oxa-1,2a-diazaacenaphthylen-6-yl)pyrimidin-2-yl)amino)-N-methylbenzenesulfonamide **(16)**

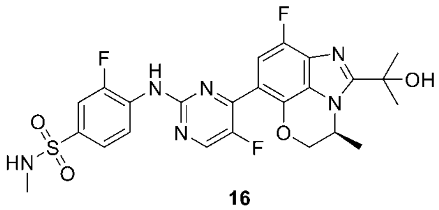

**16**

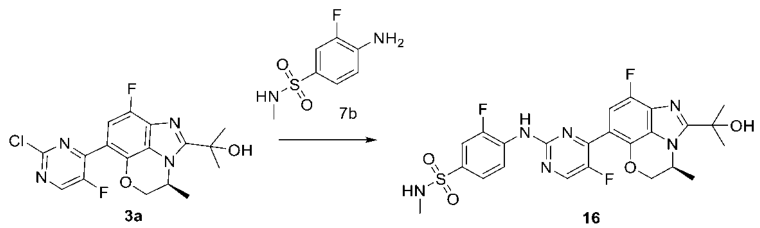

3a 7b 16

The target compound **16** was obtained by using a synthesis method similar to that of Example 7 and replacing compound A10 with compound 3a.

MS(ESI) m/z = 549.21[M+H]+.

$^1$H NMR (400 MHz, DMSO DMSO-$d_6$) δ 9.73 (s, 1H), 8.67 (d, 1H), 8.35 - 8.27 (m, 1H), 7.60-7.56 (m, 2H), 7.44 (d, 1H), 7.16 (d, 1H), 5.84 (s, 1H), 5.27 (q, 1H), 4.59 - 4.52 (m, 1H), 4.27 (dd, 1H), 2.43 (d, 3H), 1.68 (s, 3H), 1.62 (s, 3H), 1.48 (d, 3H).

### Example 17

(4-((5-Fluoro-4-((S)-8-fluoro-2-(2-hydroxypropan-2-yl)-3-methyl-3,4-dihydro-5-oxa-1,2a-diazaacenaphthylen-6-yl)pyrimidin-2-yl)amino)phenyl)(methyl)sulfonimide **(17)**

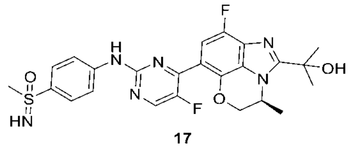

17

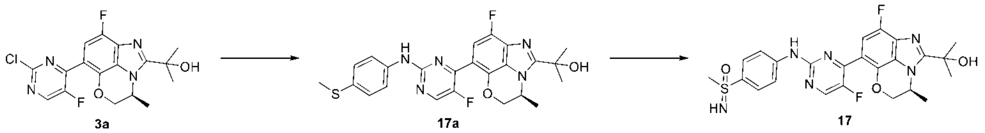

3a 17a 17

The target compound **17** was obtained by using a synthesis method similar to that of Example 14, replacing compound A10 with compound 3a, and replacing 2-fluoro-4-methylthioaniline with 4-methylthioaniline.

MS(ESI) m/z = 515.22[M+H]$^+$.

1H NMR (400 MHz, DMSO-$d_6$) δ 9.73 (s, 1H), 8.67 (d, 1H), 8.35 - 8.27 (m, 1H), 7.62-7.56 (m, 2H), 7.44 (d, 1H), 7.16 (d, 1H), 5.84 (s, 1H), 5.27 (q, 1H), 4.59 - 4.52 (m, 1H), 4.27 (dd, 1H), 2.43 (d, 3H), 1.68 (s, 3H), 1.62 (s, 3H), 1.48 (d, 3H).

**Example 18**

(S)-4-(4-((5-Fluoro-4-(8-fluoro-2-(2-hydroxypropan-2-yl)-3-methyl-3,4-dihydro-5-oxa-1,2a-diazaacenaphthylen-6-yl) pyrimidin-2-yl)amino)phenyl)morpholin-3-one **(18)**

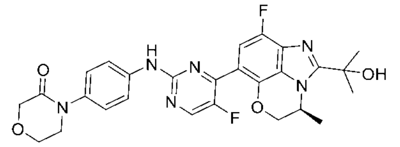

18

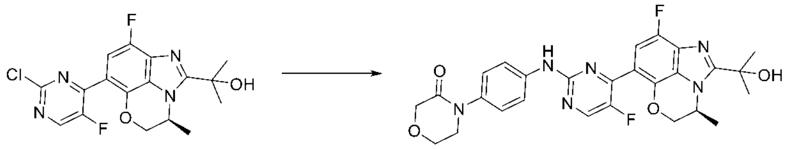

3a 18

The target compound **18** was obtained by using a synthesis method similar to that of Example 1, replacing compound A10 with compound 3a, and replacing compound 1b with 4-(4-aminophenyl)morpholin-3-one.

MS(ESI) m/z = 537.22[M+H]$^+$.

$^1$H NMR (400 MHz, DMSO-$d_6$) δ 9.89 (s, 1H), 8.61 (d, 1H), 7.84 - 7.76 (m, 2H), 7.32 - 7.25 (m, 2H), 7.17 (d, 1H), 5.84 (s, 1H), 5.26 (q, 1H), 4.57 - 4.52 (m, 1H), 4.27 (dd, 1H), 4.18 (s, 2H), 3.96 (dd, 2H), 3.73 - 3.66 (m, 2H), 1.68 (s, 3H), 1.63 (s, 3H), 1.49 (d, 3H).

**Example 19**

(4-((5-Chloro-4-((S)-8-fluoro-2-(2-hydroxypropan-2-yl)-3-methyl-3,4-dihydro-5-oxa-1,2a-diazaacenaphthylen-6-yl)pyrimidin-2-yl)amino)phenyl)(methyl)sulfinamide **(19)**

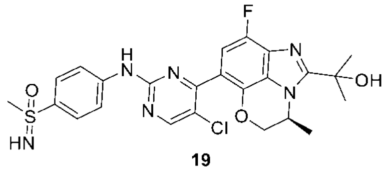

19

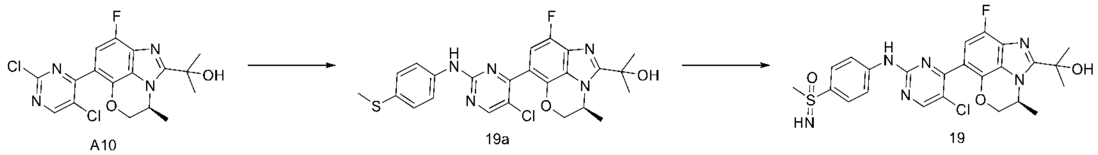

A10 19a 19

The target compound **19** was obtained by using a synthesis method similar to that of Example 14 and replacing 2-fluoro-4-methylthioaniline with 4-methylthioaniline.

MS(ESI) m/z = 531.17[M+H]$^+$.

$^1$H NMR (400 MHz, DMSO-$d_6$) δ 10.41 (s, 1H), 8.71 (s, 1H), 8.01 - 7.93 (m, 2H), 7.85 - 7.77 (m, 2H), 7.08 (d, 1H), 5.83 (s, 1H), 5.30 - 5.23 (m, 1H), 4.55 - 4.49 (m, 1H), 4.28 (dd, 1H), 4.01 (s, 1H), 3.02 (s, 3H), 1.68 (s, 3H), 1.63 (s, 3H), 1.48 (d, 3H).

**Example 20**

(R)-4-(4-((5-Chloro-4-(8-fluoro-2-(2-hydroxypropan-2-yl)-3-methyl-3,4-dihydro-5-oxa-1,2a-diazaacenaphthylen-6-yl) pyrimidin-2-yl)amino)-3-fluorophenyl)morpholin-3-one **(20)**

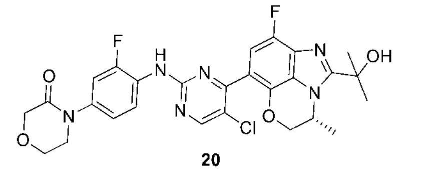

**20**

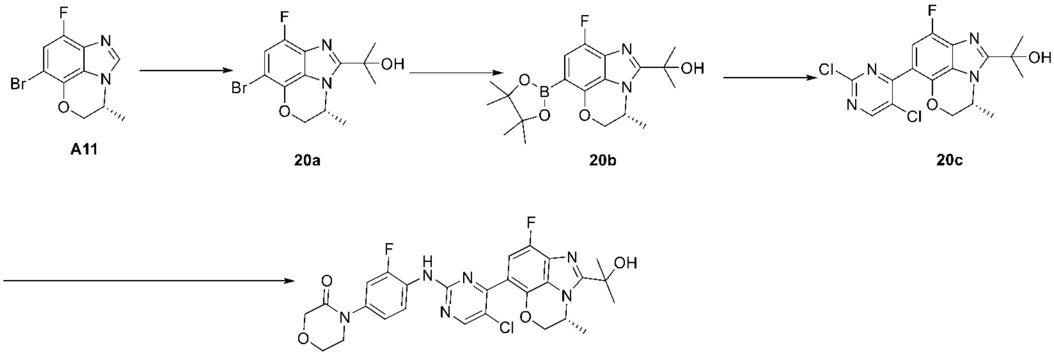

**A11** **20a** **20b** **20c**

**20**

Step 1: (R)-2-(6-bromo-8-fluoro-3-methyl-3,4-dihydro-5-oxa-1,2a-diazaacenaphthylen-2-yl)propan-2-ol **(20a)**

Compound A11 **(A7** enantiomer, obtained by synthesis using a method similar to that in Patent No. WO2022166799 and resolution) was added to tetrahydrofuran (25 mL). The mixture was purged with nitrogen three times, and LDA (5.55 mL, 11.1 mmol, 2.0 M in THF) was added at -78 °C. The mixture was stirred for 1 h, and acetone (1.24 g, 22.1 mmol) was added. The mixture was left to react for 1 h, and saturated ammonium chloride (100 mL) was added. The aqueous phase was extracted with ethyl acetate (20 mL × 3). The organic phases were combined and concentrated, and the residue was separated by column chromatography to give compound **20a** (2.17 g, yield: 90.1%).

MS(ESI) m/z = 329.2[M+H]$^+$.

Step 2: (R)-2-(8-fluoro-3-methyl-6-(4,4,5,5-tetramethyl-1,3,2-dioxaborolan-2-yl)-3,4-dihydro-5-oxa-1,2a-diazaacenaphthylen-2-yl)propan-2-ol **(20b)**

Compound **20a** (2.17 g, 6.59 mmol) was dissolved in 1,4-dioxane (20 mL), and bis(pinacolato)diboron (5.02 g, 19.8 mmol), potassium acetate (1.94 g, 19.8 mmol), and $PdCl_2(PPh_3)_2$ (232 mg, 0.33 mmol) were sequentially added. The mixture was purged with nitrogen three times and left to react at 110 °C for 18 h. After the reaction mixture was filtered and concentrated, the residue was separated by column chromatography to give compound **20b** (1.96 g, yield: 79.2%).

MS(ESI) m/z = 377.3[M+H]$^+$.

Step 3: (R)-2-(6-(2,5-dichloropyrimidin-4-yl)-8-fluoro-3-methyl-3,4-dihydro-5-oxa-1,2a-diazaacenaphthylen-2-yl)propan-2-ol **(20c)**

Compound **20b** (1.45 g, 3.85 mmol) was dissolved in 1,4-dioxane (8 mL) and water (0.05 mL), and 2,4,5-trichloropyrimidine (0.71 g, 3.85 mmol), sodium carbonate (0.82 mg, 7.71 mmol), and PdC12(dppf) (138 mg, 0.19 mmol)

were sequentially added. The mixture was purged with nitrogen three times and left to react at 80 °C for 18 h. After the reaction mixture was filtered and concentrated, the residue was separated by column chromatography to give compound **20c** (1.60 g, yield: 104.0%).

MS(ESI) m/z = 397.2[M+H]$^+$.

Step 4: (R)-4-(4-((5-chloro-4-(8-fluoro-2-(2-hydroxypropan-2-yl)-3-methyl-3,4-dihydro-5-oxa-1,2a-diazaacenaphthylen-6-yl)pyrimidin-2-yl)amino)-3-fluorophenyl)morpholin-3-one **(20)**

Compound **20c** (83.0 mg, 0.21 mmol) was dissolved in 1,4-dioxane (4 mL), and 4-(4-amino-3-fluorophenyl) morpholin-3-one **(1b)** (44.0 mg, 0.21 mmol), cesium carbonate (137 mg, 0.42 mmol), and XantPhos Pd G3 (10.0 mg, 0.01 mmol) were sequentially added. In a nitrogen atmosphere, the mixture was left to react in a microwave reactor at 100 °C for 1.5 h. After the reaction mixture was filtered and concentrated, the residue was purified by C18 reversed-phase chromatography to give compound **20** (38.0 mg, yield: 31.8%).

MS(ESI) m/z = 571.2[M+H]$^+$.

$^1$H NMR (400 MHz, DMSO-$d_6$) δ 9.56 (s, 1H), 8.55 (s, 1H), 7.72 (t, 1H), 7.39 (dd, 1H), 7.21 (dd, 1H), 6.99 (d, 1H), 5.82 (s, 1H), 5.24 (q, 1H), 4.50 (d, 1H), 4.24 (dd, 1H), 4.20 (s, 2H), 3.97 (dd, 2H), 3.74 (dd, 2H), 1.67 (s, 3H), 1.62 (s, 3H), 1.46 (d, 3H).

**Example 21**

4-(3-Fluoro-4-((5-fluoro-4-((3S)-8-fluoro-2-(1-hydroxyethyl)-3-methyl-3,4-dihydro-5-oxa-1,2a-diazaacenaphthylen-6-yl) pyrimidin-2-yl)amino)phenyl)morpholin-3-one **(21)**

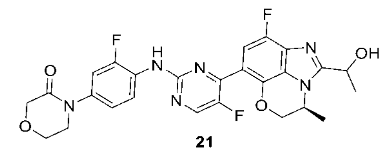

21

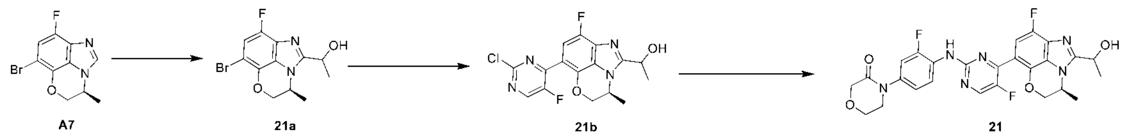

A7 21a 21b 21

Step 1 : 1-((S)-6-bromo-8-fluoro-3-methyl-3,4-dihydro-5-oxa-1,2a-diazaacenaphthylen-2-yl)ethan-1-ol **(21a)**

Compound **A7** (270 mg, 1.0 mmol) was added to anhydrous THF (10 mL). In a nitrogen atmosphere, the mixture was cooled to -78 °C, and LDA (0.6 mL, 2 N in THF) was slowly added. After 60 min of reaction, acetaldehyde (66 mg, 1.5 mmol) was added. The mixture was stirred for another 60 min with the temperature maintained. After the reaction was complete, a saturated ammonium chloride solution (5 mL) was added to quench the reaction, and ethyl acetate (15 mL × 2) was added for extraction. The organic phases were combined, dried, and concentrated, and the residue was separated by column chromatography to give compound **21a** (250 mg, yield: 79.6%).

MS(ESI) m/z = 315.1[M+H]$^+$.

Step 2: 1-((S)-6-(2-chloro-5-fluoropyrimidin-4-yl)-8-fluoro-3-methyl-3,4-dihydro-5-oxa-1,2a-diazaacenaphthylen-2-yl) ethan-1-ol **(21b)**

Compound **21a** (31 mg, 0.1 mmol) was dissolved in 1,4-dioxane (2 mL), and bis(pinacolato)diboron (37.5 mg, 0.15 mmol), potassium acetate (20 mg, 0.2 mmol), and Pd(dppf)$Cl_2$ (7.0 mg, 0.01 mmol) were added in a nitrogen atmosphere. The mixture was stirred at 100 °C for 120 min. Subsequently, the reaction mixture was cooled to room temperature, and 2,4-dichloro-5-fluoropyrimidine (16.5 mg, 0.1 mmol), sodium carbonate (21 mg, 0.2 mmol), a small amount of Pd(dppf)$Cl_2$, and two drops of water were added. The mixture was purged with nitrogen three times and left to react at 110 °C for 1.5 h. After the reaction was complete, the reaction mixture was concentrated. Water (5 mL) was added to the reaction mixture, and extraction was performed with ethyl acetate (10 mL × 2). The organic phases were combined, dried, and concentrated, and the residue was separated by column chromatography to give compound **21b** (18 mg, yield:

49.2%).

MS(ESI) m/z = 367.2[M+H]$^+$.

Step 3: 4-(3-fluoro-4-((5-fluoro-4-((3S)-8-fluoro-2-(1-hydroxyethyl)-3-methyl-3,4-dihydro-5-oxa-1,2a-diazaacenaphthylen-6-yl)pyrimidin-2-yl)amino)phenyl)morpholin-3-one **(21)**

Compound **21b** (18 mg, 0.05 mmol) was dissolved in 1,4-dioxane (1 mL), and 4-(4-amino-3-fluorophenyl) morpholin-3-one **(1b)** (11 mg, 0.05 mmol), cesium carbonate (32 mg, 0.1 mmol), and Xantphos Pd G3 (5 mg, 0.005 mmol) were sequentially added. In a nitrogen atmosphere, the mixture was left to react in a microwave reactor at 110 °C for 1.5 h. After the reaction mixture was filtered, the residue was separated by C18 reversed-phase chromatography to give compound **21** (7 mg, yield: 25.9%).

MS(ESI) m/z = 541.3[M+H]$^+$.

$^1$H NMR (400 MHz, DMSO-$d_6$) δ 9.36 (s, 1H), 8.55 (d, 1H), 7.79 (t, 1H), 7.38 (dd, 1H), 7.25 (dd, 1H), 7.14 (dd, 1H), 5.94-5.86 (m, 1H), 5.17 - 5.05 (m, 2H), 4.60 - 4.48 (m, 1H), 4.33 - 4.17 (m, 1H), 4.21 (s, 2H), 3.97 (dd, 2H), 3.75 (dd, 2H), 1.61 (t, 3H), 1.46 (dd, 3H).

**Example 22**

4-(3-Fluoro-4-((5-fluoro-4-((3S)-8-fluoro-2-(1-methoxyethyl)-3-methyl-3,4-dihydro-5-oxa-1,2a-diazaacenaphthylen-6-yl)pyrimidin-2-yl)amino)phenyl)morpholin-3-one **(22)**

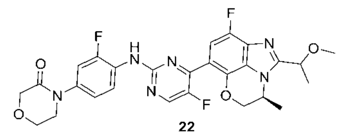

22

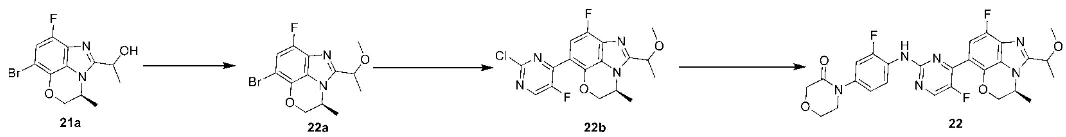

21a 22a 22b 22

Step 1: (3S)-6-bromo-8-fluoro-2-(1-methoxyethyl)-3-methyl-3,4-dihydro-5-oxa-1,2a-diazaacenaphthylene **(22a)**

Compound **21a** (314 mg, 1.0 mmol) was added to anhydrous THF (10 mL). In a nitrogen atmosphere, the mixture was cooled to 0 °C, and 60% sodium hydride (60 mg, 1.5 mmol) was added in batches. After 30 min of reaction, iodomethane (170 mg, 1.2 mmol) was added. The mixture was warmed to room temperature and stirred for 30 min. The reaction mixture was poured into water (20 mL) and extracted with ethyl acetate (15 mL × 2). The organic phases were combined and dried. The reaction mixture was concentrated, and the residue was separated by column chromatography to give compound **22a** (250 mg, yield: 76.2%).

MS(ESI) m/z = 329.2[M+H]$^+$.

Remaining steps: The target compound 22 was obtained by using a synthesis method similar to that of Example 21 and replacing compound 21a with compound 22a. MS(ESI) m/z = 555.3[M+H]$^+$.

$^1$H NMR (400 MHz, DMSO-$d_6$) δ 9.37 (s, 1H), 8.56 (d, 1H), 7.80 (t, 1H), 7.38 (dd, 1H), 7.21 (dd, 1H), 7.14 (d, 1H), 5.09 - 4.94 (m, 1H), 4.92-4.88 (m, 1H), 4.58-4.55 (m, 1H), 4.32-4.29 (m, 1H), 4.21 (s, 2H), 3.97 (dd, 2H), 3.79 - 3.71 (m, 2H), 3.30 (s, 3H), 1.61 (dd, 3H), 1.44 (dd, 3H).

**Example 23**

(S)-1-(4-((5-Chloro-4-(8-fluoro-2-(2-hydroxypropan-2-yl)-3-methyl-3,4-dihydro-5-oxa-1,2a-diazaacenaphthylen-6-yl) pyrimidin-2-yl)amino)3-fluorophenyl)piperazin-2-one **(23)**

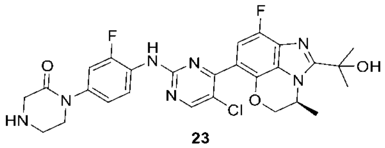

23

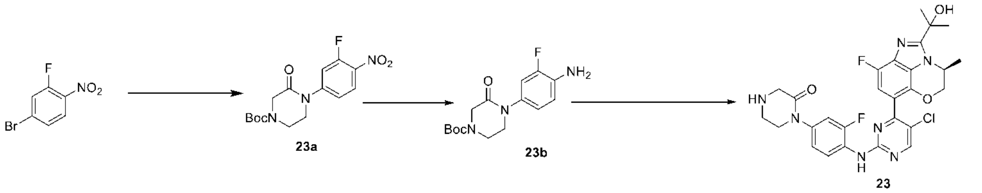

Step 1: tert-butyl 4-(3-fluoro-4-nitrophenyl)-3-oxopiperazine-1-carboxylate **(23a)**

In a nitrogen atmosphere, tert-butyl 3-oxopiperazine-1-carboxylate (200 mg, 1.0 mmol) was dissolved in 1,4-dioxane (10 mL), and 4-bromo-2-fluoro-1-nitrobenzene (220 mg, 1.0 mmol), cesium carbonate (650 mg, 2 mmol), and Xantphos Pd G3 (45 mg, 0.05 mmol) were sequentially added. The mixture was left to react in a microwave reactor at 110 °C for 1.5 h. After the reaction was complete, the reaction mixture was concentrated, and the residue was separated by column chromatography to give compound **23a** (180 mg, yield: 53.1%).

MS(ESI) m/z = 240.2[M-100+H]$^+$.

Step 2: tert-butyl 4-(4-amino-3-fluorophenyl)-3-oxopiperazine-1-carboxylate (23b)

Compound **23a** (170 mg, 0.5 mmol) was dissolved in ethanol:water (4 mL:1 mL) at room temperature, and ammonium chloride (53 mg, 1.0 mmol) was added. The mixture was heated to 60 °C, and iron powder (280 mg, 5 mmol) was added. The mixture was left to react for 2 h. After the reaction was complete, the reaction mixture was filtered through diatomaceous earth while hot, and the filtrate was concentrated under reduced pressure. Water (5 mL) was added, and extraction was performed with ethyl acetate (15 mL × 2). The organic phases were combined and concentrated to give compound **23b** (140 mg, yield: 93.3%).

MS(ESI) m/z = 310.2[M-100+H]$^+$.

Step 3: (S)-1-(4-((5-chloro-4-(8-fluoro-2-(2-hydroxypropan-2-yl)-3-methyl-3,4-dihydro-5-oxa-1,2a-diazaacenaphthylen-6-yl)pyrimidin-2-yl)amino)3-fluorophenyl)piperazin-2-one **(23)**

Compound **23b** (60 mg, 0.2 mmol) was dissolved in 1,4-dioxane (3 mL), and compound **A10** (80 mg, 0.2 mmol), cesium carbonate (130 mg, 0.4 mmol), and Xantphos Pd G3 (18 mg, 0.02 mmol) were sequentially added. In a nitrogen atmosphere, the mixture was left to react in a microwave reactor at 110 °C for 1.5 h. The reaction mixture was filtered and then concentrated, and hydrochloric acid (2 N in EA, 1 mL) was added. The mixture was stirred at room temperature for 1 h. After concentration, the residue was separated by C18 reversed-phase chromatography to give the target product **23** (20 mg, yield: 17.5%).

MS(ESI) m/z = 570.3[M+H]$^+$.

$^1$H NMR (400 MHz, DMSO-$d_6$) δ 9.56 (s, 1H), 8.54 (d, 1H), 7.70 (t, 1H), 7.33-7.31 (m, 1H), 7.19 - 7.10 (m, 1H), 6.99 (d, 1H), 5.83 (s, 1H), 5.24-5.22 (m, 1H), 4.50 (d, 1H), 4.24 (d, 1H), 3.84 - 3.67 (m, 2H), 3.60-3.58 (m, 2H), 3.00-2.98 (m, 2H), 1.65 (d, 6H), 1.46 (d, 3H), 1.24 (s, 1H).

**Example 24**

(S)-4-(4-((5-Chloro-4-(8-fluoro-2-(2-hydroxypropan-2-yl)-3-methyl-3,4-dihydro-5-oxa-1,2a-diazaacenaphthylen-6-yl) pyrimidin-2-yl)amino)2-fluorophenyl)morpholin-3-one **(24)**

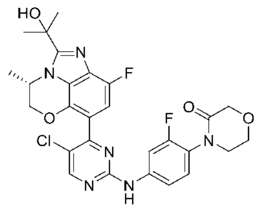

24

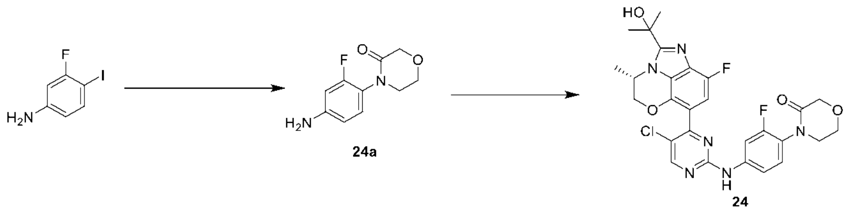

Step 1: 4-(4-amino-2-fluorophenyl)morpholin-3-one **(24a)**

3-Fluoro-4-iodoaniline (237 mg, 1.0 mmol) was dissolved in NMP (10 mL), and morpholin-3-one (101 mg, 1.0 mmol), cuprous iodide (38 mg, 0.2 mmol), potassium phosphate (454 mg, 2 mmol), and DMEDA (35 mg, 0.4 mmol) were sequentially added. In a nitrogen atmosphere, the mixture was left to react in a microwave reactor at 130 °C for 2 h. After the reaction was complete, the reaction mixture was concentrated, and the residue was separated by column chromatography to give compound **24a** (200 mg, yield: 95.2%).

MS(ESI) m/z = 211.1[M+H]$^+$.

Step 2: (S)-4-(4-((5-chloro-4-(8-fluoro-2-(2-hydroxypropan-2-yl)-3-methyl-3,4-dihydro-5-oxa-1,2a-diazaacenaphthylen-6-yl)pyrimidin-2-yl)amino)2-fluorophenyl)morpholin-3-one **(24)**

Compound **24a** (21 mg, 0.1 mmol) was dissolved in 1,4-dioxane (1 mL), and compound A10 (40 mg, 0.1 mmol), cesium carbonate (65 mg, 0.2 mmol), and Xantphos Pd G3 (9 mg, 0.01 mmol) were sequentially added. In a nitrogen atmosphere, the mixture was left to react in a microwave reactor at 110 °C for 1.5 h. The reaction mixture was filtered, and the residue was separated by C18 reversed-phase chromatography to give the target product **24** (10 mg, yield: 17.5%).

MS(ESI) m/z = 571.3[M+H]$^+$.

$^1$H NMR (400 MHz, DMSO-$d_6$) δ 10.28 (s, 1H), 8.68 (s, 1H), 7.92 (dd, 1H), 7.49 (dd, 1H), 7.35-7.33 (m, 1H), 7.08 (d, 1H), 5.83 (s, 1H), 5.26 (d, 1H), 4.51 (d, 1H), 4.27 (d, 1H), 4.21 (s, 2H), 3.97 (dd, 2H), 3.63 (dd, 2H), 1.65 (d, 6H), 1.48 (d, 3H).

**Example 25**

(S)-4-(2-Chloro-4-((5-chloro-4-(8-fluoro-2-(2-hydroxypropan-2-yl)-3-methyl-3,4-dihydro-5-oxa-1,2a-diazaacenaphthylen-6-yl)pyrimidin-2-yl)amino)phenyl)morpholin-3-one **(25)**

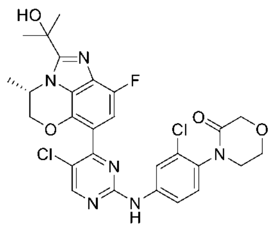

25

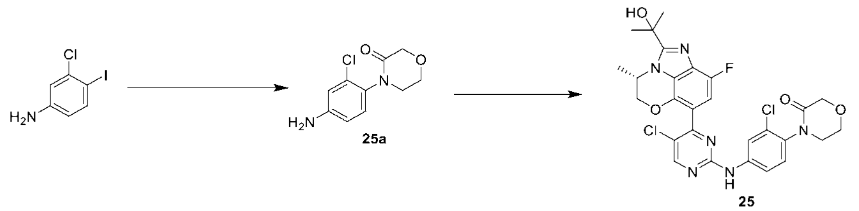

25a

25

The target compound **25** was obtained by using a synthesis method similar to that of Example 24 and using 3-chloro-4-iodoaniline as the starting material.

MS(ESI) m/z = 587.2[M+H]$^+$.

$^1$H NMR (400 MHz, DMSO-$d_6$) δ 10.27 (s, 1H), 8.69 (s, 1H), 8.10 (d, 1H), 7.71 (dd, 1H), 7.36 (d, 1H), 7.08 (d, 1H), 5.83 (s, 1H), 5.26 (d, 1H), 4.51 (d, 1H), 4.27 (d,1H), 4.20 (s, 2H), 3.97 (dd, 2H), 3.60 (dd, 2H), 1.68 (s, 3H), 1.63 (s, 3H), 1.48 (d, 3H).

Example **26**

(S)-4-(3-Fluoro-4-((5-fluoro-4-(8-fluoro-2-(methoxymethyl)-3-methyl-3,4-dihydro-5-oxa-1,2a-diazaacenaphthylen-6-yl) pyrimidin-2-yl)amino)phenyl)morpholin-3-one **(26)**

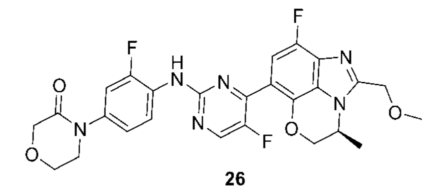

26

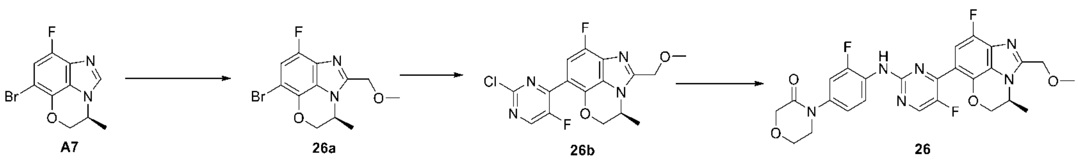

A7 26a 26b 26

Step 1: (S)-6-bromo-8-fluoro-2-(methoxymethyl)-3-methyl-3,4-dihydro-5-oxa-1,2a-diazaacenaphthylene **(26a)**

Compound **A7** (160 mg, 0.59 mmol) was added to tetrahydrofuran (5 mL), and LDA (0.33 mL, 0.66 mmol, 2.0 M in THF) was added at -78 °C in a nitrogen atmosphere. The mixture was stirred for 1 h, and bromomethyl methyl ether (89 mg, 0.71 mmol) was added. The mixture was left to react for 1 h. Saturated ammonium chloride (50 mL) was added. Liquid separation was performed, and the aqueous phase was extracted with ethyl acetate (10 mL × 3). The organic phases were combined and concentrated, and the residue was separated by column chromatography to give the product **26a** (100 mg, yield: 53.7%).

MS(ESI) m/z = 315.2[M+H]$^+$.

Step 2: (S)-6-(2-chloro-5-fluoropyrimidin-4-yl)-8-fluoro-2-(methoxymethyl)-3-methyl-3,4-dihydro-5-oxa-1,2a-diazaacenaphthylene **(26b)**

Compound **26a** (117 mg, 0.37 mmol) was dissolved in 1,4-dioxane (3 mL), and bis(pinacolato)diboron (283 mg, 1.11 mmol), potassium acetate (109 mg, 1.11 mmol), and Pd(dppf)$Cl_2$ (26 mg, 0.1 mmol) were sequentially added. The mixture was left to react at 110 °C for 2 h in a nitrogen atmosphere. 2,4-Dichloro-5-fluoropyrimidine (87 mg, 0.52 mmol), sodium carbonate (76.0 mg, 0.72 mmol), and Pd(dppf)$Cl_2$ (26 mg, 0.1 mmol) were sequentially added. The mixture was left to react at 110 °C for 2 h in a nitrogen atmosphere. After the reaction mixture was filtered and concentrated, the residue was separated by column chromatography to give the product **26b** (100 mg, yield: 55.0%).

MS(ESI) m/z = 367.1 [M+H]$^+$.

Step 3: (S)-4-(3-fluoro-4-((5-fluoro-4-(8-fluoro-2-(methoxymethyl)-3-methyl-3,4-dihydro-5-oxa-1,2a-diazaacenaphthylen-6-yl)pyrimidin-2-yl)amino)phenyl)morpholin-3-one **(26)**

Compound **26b** (50.0 mg, 0.14 mmol) was dissolved in 1,4-dioxane (2 mL), and 4-(4-amino-3-fluorophenyl) morpholin-3-one **(1b)** (29.0 mg, 0.14 mmol), cesium carbonate (96.0 mg, 0.30 mmol), and XantPhos Pd G3 (5.0 mg, 0.005 mmol) were sequentially added. In a nitrogen atmosphere, the mixture was left to react in a microwave reactor at 110 °C for 1 h. After the reaction mixture was filtered and concentrated, the residue was purified by C18 reversed-phase chromatography to give a compound product (20 mg, yield: 27.1%).

MS(ESI) m/z = 541.3$[M+H]^+$.

$^1H$ NMR (400 MHz, DMSO) δ 9.36 (s, 1H), 8.57 (d, 1H), 7.80 (t, 1H), 7.39 (dd, 1H), 7.25 - 7.18 (m, 1H), 7.15 (d, 1H), 4.98-4.92 (m, 1H), 4.85 - 4.71 (m, 2H), 4.52 (dd, 1H), 4.31 (dd, 1H), 4.21 (s, 2H), 3.97 (dd, 2H), 3.75 (dd, 2H), 3.38 (s, 3H), 1.46 (d, 3H).

**Example 27**

(S)-4-(3-Fluoro-4-((5-fluoro-4-(8-fluoro-2-isopropyl-3-methyl-3,4-dihydro-5-oxa-1,2a-diazaacenaphthylen-6-yl)pyrimidin-2-yl)amino)phenyl)morpholin-3-one **(27)**

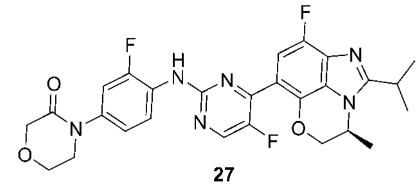

**27**

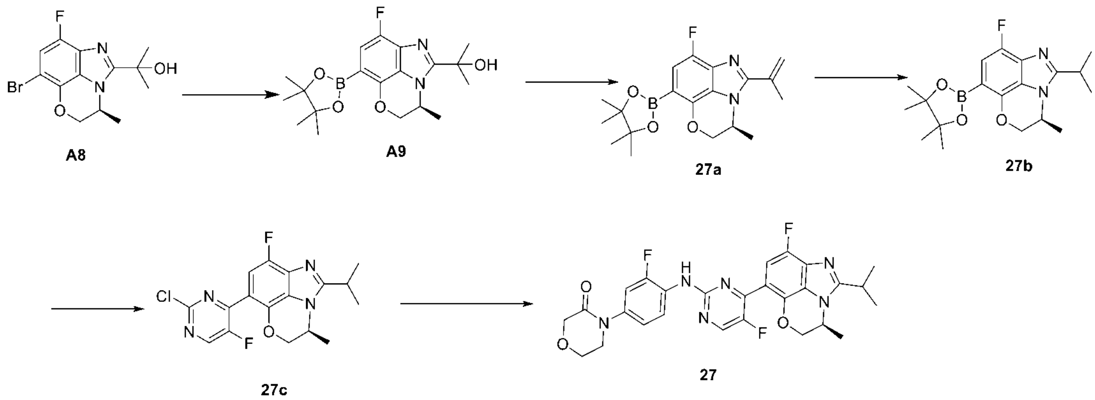

A8 A9 27a 27b 27c 27

Step 1: (S)-2-(8-fluoro-3-methyl-6-(4,4,5,5-tetramethyl-1,3,2-dioxaborolan-2-yl)-3,4-dihydro-5-oxa-1,2a-diazaacenaphthylen-2-yl)propan-2-ol **(A9)**

Compound **A8** (328 mg, 1.0 mmol) was dissolved in dioxane (10 mL), and bis(pinacolato)diboron (635 mg, 2.50 mmol), potassium acetate (250 mg, 2.5 mmol), and Pd(dppf)$Cl_2$ (65 mg, 0.05 mmol) were sequentially added. In a nitrogen atmosphere, the mixture was left to react in a microwave reactor at 110 °C for 1 h. The reaction mixture was directly used in the next step.

MS(ESI) m/z = 377.4$[M+H]^+$.

Step 2: (S)-8-fluoro-3-methyl-2-(prop-1-en-2-yl)-6-(4,4,5,5-tetramethyl-1,3,2-dioxaborolan-2-yl)-3,4-dihydro-5-oxa-1,2a-diazaacenaphthylene **(27a)**

Compound **A9** (200 mg, 0.53 mmol) was added to tetrahydrofuran (5 mL), and the Burgess reagent (138 mg, 0.58 mmol) was added at room temperature. The mixture was stirred at room temperature for 2 h in a nitrogen atmosphere. Water (50 mL) was added, and the aqueous phase was extracted with ethyl acetate (10 mL × 3). The organic phases were combined and concentrated, and the residue was separated by column chromatography to give compound **27a** (248 mg, yield: 130.0%).

MS(ESI) m/z = 359.2[M+H]$^+$.

Step 3: (S)-8-fluoro-2-isopropyl-3-methyl-6-(4,4,5,5-tetramethyl-1,3,2-dioxaborolan-2-yl)-3,4-dihydro-5-oxa-1,2a-diazaacenaphthylene **(27b)**

Compound **27a** (248 mg, 0.69 mmol) was dissolved in methanol (5 mL), and 10% Pd/C (24 mg) was added. The mixture was left to react at room temperature for 16 h in a hydrogen atmosphere. After the reaction mixture was filtered and concentrated, the residue was separated by column chromatography to give compound **27b** (226 mg, yield: 91.0%).

MS(ESI) m/z = 361.3[M+H]$^+$.

Step 4: (S)-6-(2-chloro-5-fluoropyrimidin-4-yl)-8-fluoro-2-isopropyl-3-methyl-3,4-dihydro-5-oxa-1,2a-diazaacenaphthylene **(27c)**

Compound **27b** (226 mg, 0.63 mmol) was dissolved in 1,4-dioxane (8 mL) and water (0.05 mL), and 2,4-dichloro-5-fluoropyrimidine (110 mg, 0.66 mmol), sodium carbonate (134 mg, 1.26 mmol), and PdC12(dppf) (21.0 mg, 0.03 mmol) were sequentially added. The mixture was left to react at 100 °C for 5 h in a nitrogen atmosphere. After the reaction mixture was filtered and concentrated, the residue was separated by column chromatography to give compound **27c** (120 mg, yield: 52.4%).

MS(ESI) m/z = 365.2[M+H]$^+$.

Step 5: (S)-4-(3-fluoro-4-((5-fluoro-4-(8-fluoro-2-isopropyl-3-methyl-3,4-dihydro-5-oxa-1,2a-diazaacenaphthylen-6-yl)pyrimidin-2-yl)amino)phenyl)morpholin-3-one **(27)** Compound **27c** (120 mg, 0.33 mmol) was dissolved in 1,4-dioxane (4 mL), and 4-(4-amino-3-fluorophenyl)morpholin-3-one **(1b)** (69.0 mg, 0.33 mmol), cesium carbonate (214 mg, 0.66 mmol), and XantPhos Pd G3 (19.0 mg, 0.02 mmol) were sequentially added. In a nitrogen atmosphere, the mixture was left to react in a microwave reactor at 100 °C for 1 h. After the reaction mixture was filtered and concentrated, the residue was purified by C18 reversed-phase chromatography to give compound **27** (70.0 mg, yield: 39.5%).

MS(ESI) m/z = 539.3[M+H]$^+$.

$^1$H NMR (400 MHz, DMSO-$d_6$) δ 9.35 (s, 1H), 8.54 (d, 1H), 7.79 (t, 1H), 7.38 (dd, 1H), 7.23-7.21 (m, 1H), 7.10 (d, 1H), 5.00 - 4.90 (m, 1H), 4.55 (dd, 1H), 4.28 (dd, 1H), 4.21 (s, 2H), 4.01 - 3.93 (m, 2H), 3.78 - 3.71 (m, 2H), 3.27-3.25(m, 1H) 1.44 - 1.33 (m, 9H).

### Example 28

(S)-4-(3-Fluoro-4-((5-fluoro-4-(8-fluoro-2-(1-hydroxycyclobutyl)-3-methyl-3,4-dihydro-5-oxa-1,2a-diazaacenaphthylen-6-yl)pyrimidin-2-yl)amino)phenyl)morpholin-3-one **(28)**

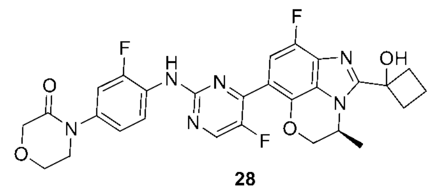

**28**

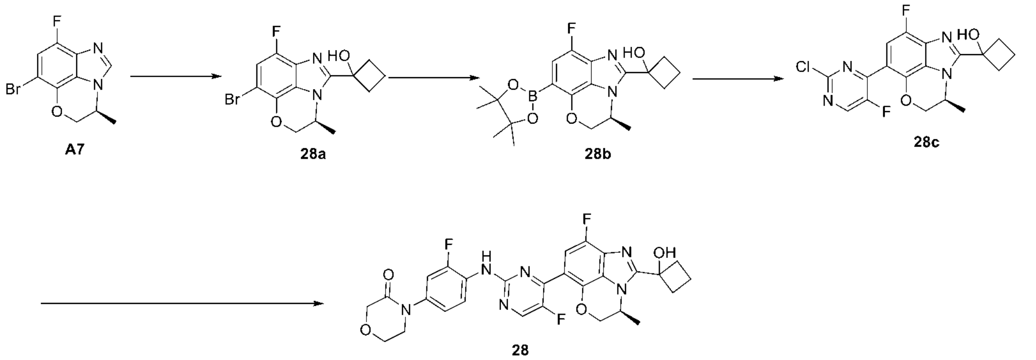

A7

28a

28b

28c

28

Step 1 : (S)-1-(6-bromo-8-fluoro-3-methyl-3,4-dihydro-5-oxa-1,2a-diazaacenaphthylen-2-yl)cyclobutan-1-ol **(28a)**

Compound **A7** (150 mg, 0.55 mmol) was added to tetrahydrofuran (6 mL), and LDA (0.33 mL, 0.66 mmol, 2.0 M in THF) was added at -78 °C in a nitrogen atmosphere. After 1 h of stirring, cyclobutanone (155 mg, 2.21 mmol) was added. The mixture was left to react for 1 h. Saturated ammonium chloride (50 mL) was added, and the aqueous phase was extracted with ethyl acetate (10 mL × 3). The organic phases were combined and concentrated, and the residue was separated and purified by column chromatography to give compound **28a** (135 mg, yield: 71.1%).
MS(ESI) m/z = 341.1$[M+H]^+$.

Step 2: (S)-1-(8-fluoro-3-methyl-6-(4,4,5,5-tetramethyl-1,3,2-dioxaborolan-2-yl)-3,4-dihydro-5-oxa-1,2a-diazaacenaphthylen-2-yl)cyclobutan-1-ol **(28b)**

Compound **28a** (135 mg, 0.40 mmol) was dissolved in 1,4-dioxane (4 mL), and bis(pinacolato)diboron (305 mg, 1.20 mmol), potassium acetate (118 mg, 1.20 mmol), and $PdCl_2$(dppf) (14.5 mg, 0.02 mmol) were sequentially added. In a nitrogen atmosphere, the mixture was left to react in a microwave reactor at 110 °C for 1 h. The reaction mixture was directly used in the next step.
MS(ESI) m/z = 389.3$[M+H]^+$.

Step 3: (S)-1-(6-(2-chloro-5-fluoropyrimidin-4-yl)-8-fluoro-3-methyl-3,4-dihydro-5-oxa-1,2a-diazaacenaphthylen-2-yl) cyclobutan-1-ol **(28c)**

Water (0.05 mL), 2,4-dichloro-5-fluoropyrimidine (67.0 mg, 0.40 mmol), sodium carbonate (42.0 mg, 0.40 mmol), and PdCl2(dppf) (14.5 mg, 0.02 mmol) were sequentially added to the reaction mixture of step 2, and the mixture was left to react at 90 °C for 18 h in a nitrogen atmosphere. After the reaction mixture was filtered and concentrated, the residue was separated by column chromatography to give compound **28c** (172 mg, two-step yield: 109.2%).
MS(ESI) m/z = 393.2$[M+H]^+$.

Step 4: (S)-4-(3-fluoro-4-((5-fluoro-4-(8-fluoro-2-(1-hydroxycyclobutyl)-3-methyl-3,4-dihydro-5-oxa-1,2a-diazaacenaphthylen-6-yl)pyrimidin-2-yl)amino)phenyl)morpholin-3-one **(28)**

Compound **28c** (66.0 mg, 0.17 mmol) was dissolved in 1,4-dioxane (3 mL), and 4-(4-amino-3-fluorophenyl) morpholin-3-one **(1b)** (37.0 mg, 0.18 mmol), cesium carbonate (110 mg, 0.34 mmol), and XantPhos Pd G3 (3.00 mg, 0.003 mmol) were sequentially added. In a nitrogen atmosphere, the mixture was left to react in a microwave reactor at 100 °C for 1.5 h. After the reaction mixture was filtered and concentrated, the residue was purified by C18 reversed-phase chromatography to give compound **28** (22.0 mg, yield: 23.3%).
MS(ESI) m/z = 567.2$[M+H]^+$.
$^1H$ NMR (400 MHz, DMSO-$d_6$) δ 9.35 (s, 1H), 8.55 (d, 1H), 7.80 (t, 1H), 7.38 (dd, 1H), 7.23-7.19 (m, 1H), 7.12 (d, 1H), 6.39 (s, 1H), 5.03 (q, 1H), 4.50 (dd, 1H), 4.30 - 4.18 (m, 3H), 4.01 - 3.90 (m, 2H), 3.79 - 3.71 (m, 2H), 2.78-2.74(m, 2H), 2.42-2.38 (m, 2H),1.8-1.84 (m, 2H), 1.43 (d, 3H).

**Example 29**

(S)-2-(4-((5-Chloro-4-(8-fluoro-2-(2-hydroxypropan-2-yl)-3-methyl-3,4-dihydro-5-oxa-1,2a-diazaacenaphthylen-6-yl) pyrimidin-2-yl)amino)phenylacetonitrile **(29)**

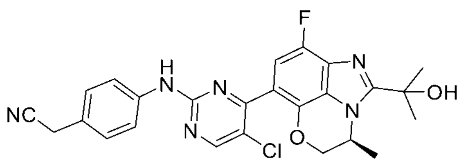

**29**

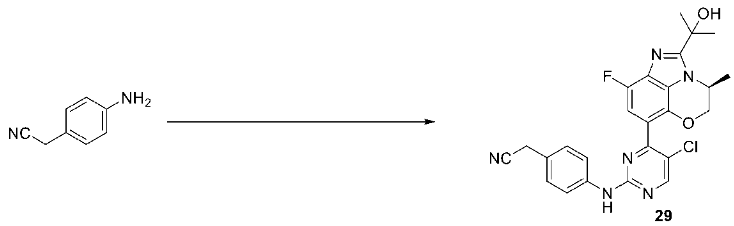

29

The target compound **29** was synthesized by using a method similar to that of Example 1 and using p-aminophenylacetonitrile as the starting material.

MS(ESI) m/z = 511.2$[M+H]^+$.

**Example 30**

(S)-3-(4-((5-Chloro-4-(8-fluoro-2-(2-hydroxypropan-2-yl)-3-methyl-3,4-dihydro-5-oxa-1,2a-diazaacenaphthylen-6-yl) pyrimidin-2-yl)amino)3-fluorophenyl)oxazolidin-2-one **(30)**

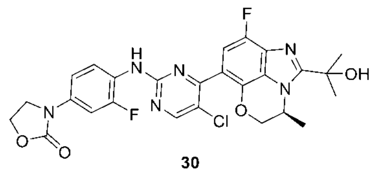

30

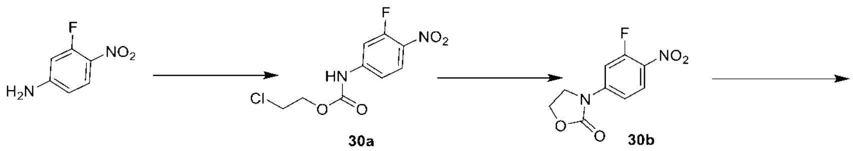

30a 30b

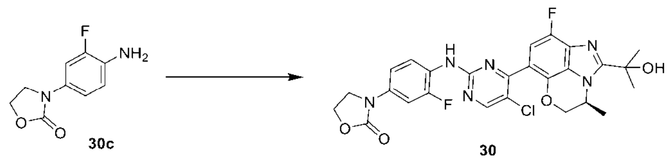

30c 30

Step 1: 2-chloroethyl (3-fluoro-4-nitrophenyl)carbamate **(30a)**

3-Fluoro-4-nitroaniline (1 g, 6.4 mmol) was added to anhydrous THF (10 mL), and 2-chloroethyl formate (1 g, 7.6 mmol) and pyridine (0.6 mL, 7.6 mmol) were added under ice-bath conditions. The mixture was left to react under ice-bath conditions for 1 h. The reaction mixture was poured into water (50 mL) and extracted with dichloromethane (15 mL × 3). After concentration, the residue was directly used in the next step.

Step 2: 3-(3-fluoro-4-nitrophenyl)oxazolidin-2-one **(30b)**

Compound 30**a** (1.68 g, 6.4 mmol) was added to acetonitrile (20 mL), and potassium carbonate (1.3 g, 9.6 mmol) was added. The mixture was left to react at 80 °C for 2 h. The reaction mixture was filtered, and the filtrate was concentrated, poured into water (5 mL), and extracted with ethyl acetate (15 mL × 3). The organic phases were combined, dried, filtered, triturated, and filtered to give compound **30b** (1.1 g, yield: 87.6%).

Remaining steps: The target compound 30 was obtained by using a synthesis method similar to that of Example 7 and replacing compound 7b with compound 30b.

MS(ESI) m/z = 557.2$[M+H]^+$.

**Example 31**

(S)-(4-((5-Chloro-4-(8-fluoro-2-(2-hydroxypropan-2-yl)-3-methyl-3,4-dihydro-5-oxa-1,2a-diazaacenaphthylen-6-yl)pyrimidin-2-yl)amino)-3-fluorophenyl)(4-ethylpiperazin-1-yl)methanone **(31)**

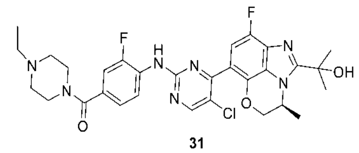

31

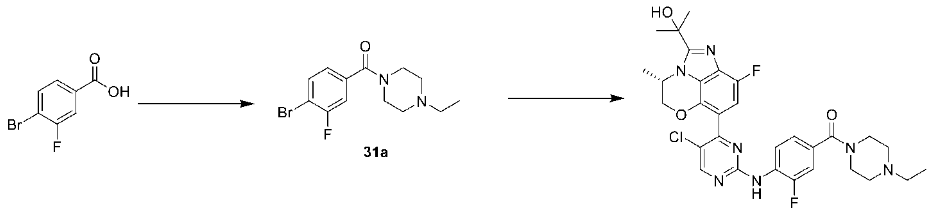

31a

31

Step 1: (4-bromo-3-fluorophenyl)(4-ethylpiperazin-1-yl)methanone **(31a)**

4-Bromo-3-fluorobenzoic acid (220 mg, 1.0 mmol) was added to DMF (10 mL), and 1-ethylpiperazine (115 mg, 1.0 mmol), TBTU (321 mg, 1.0 mmol), and DIEA (258 mg, 2.0 mmol) were added at room temperature. The mixture was left to react at room temperature for 2 h in a nitrogen atmosphere. The reaction mixture was poured into water (5 mL) and extracted with ethyl acetate (10 mL × 2). The organic phases were combined and concentrated, and the residue was separated by column chromatography to give the product, compound **31a** (250 mg, yield: 79.3%).

MS(ESI) m/z = 315.3[M+H]$^+$.

Step 2: (S)-(4-((5-chloro-4-(8-fluoro-2-(2-hydroxypropan-2-yl)-3-methyl-3,4-dihydro-5-oxa-1,2a-diazaacenaphthylen-6-yl)pyrimidin-2-yl)amino)-3-fluorophenyl)(4-ethylpiperazin-1-yl)methanone **(31)**

The target compound **31** was obtained by using the synthesis method of Example 9 and using compound **31a** as the starting material.

MS(ESI) m/z = 612.4[M+H]$^+$.

**Example 32**

(S)-(4-((5-Chloro-4-(8-fluoro-2-(2-hydroxypropan-2-yl)-3-methyl-3,4-dihydro-5-oxa-1,2a-diazaacenaphthylen-6-yl)pyrimidin-2-yl)amino)-2-fluorophenyl)(4-ethylpiperazin-1-yl)methanone **(32)**

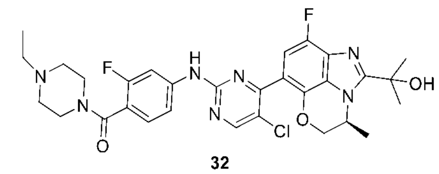

32

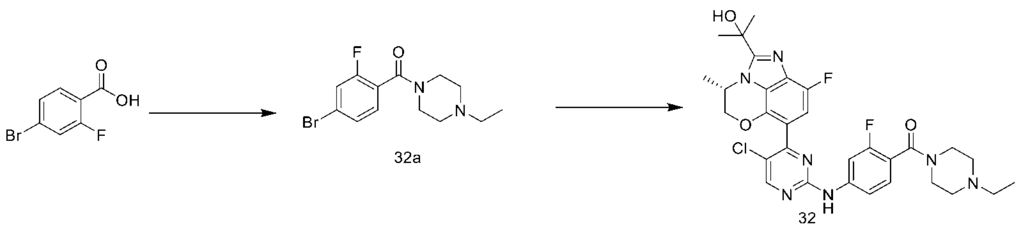

32a

32

The target compound **32** was obtained by using a synthesis method similar to that of Example 31 and replacing 4-bromo-3-fluorobenzoic acid with 4-bromo-2-fluorobenzoic acid.

MS(ESI) m/z = 612.4[M+H]+.

**Example 33**

4-(4-((5-Chloro-4-((3S)-8-fluoro-2-(1-methoxyethyl)-3-methyl-3,4-dihydro-5-oxa-1,2a-diazaacenaphthylen-6-yl)pyrimidin-2-yl)amino)-3-fluorophenyl)morpholin-3-one (**33**)

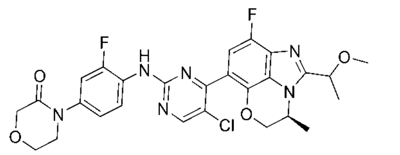

33

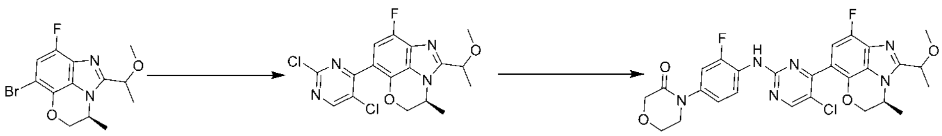

22a 33a 33

The target compound **33** was obtained by using a synthesis method similar to that of Example 22 and replacing 2,4-dichloro-5-fluoropyrimidine with 2,4,5-trichloropyrimidine.

MS(ESI) m/z = 571.3[M+H]+.

**Example 34**

1-(4-((5-Chloro-4-((S)-8-fluoro-2-(2-hydroxypropan-2-yl)-3-methyl-3,4-dihydro-5-oxa-1,2a-diazaacenaphthylen-6-yl) pyrimidin-2-yl)amino)-3-fluorophenyl)-3-(dimethylamino)pyrrolidin-2-one (**34**)

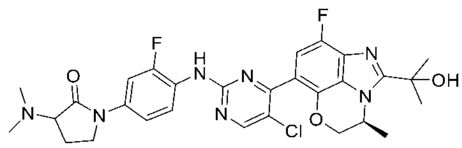

34

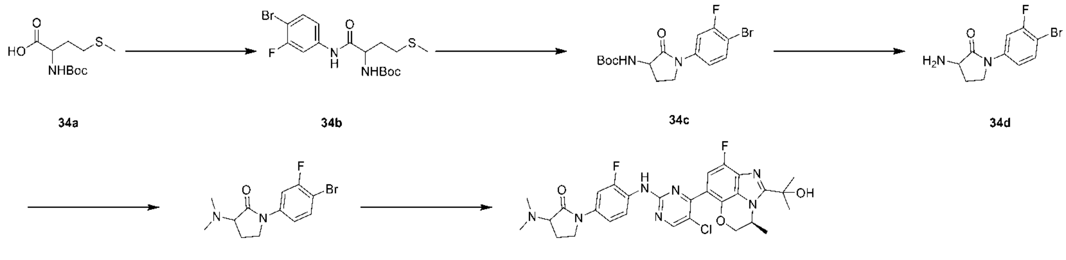

34a 34b 34c 34d

34e 34

Step 1: tert-butyl (1-((4-bromo-3-fluorophenyl)amino)-4-(methylthio)-1-oxobutan-2-yl)carbamate (**34b**)

Compound **34a** (500 mg, 2.005 mmol), 4-bromo-3-fluoroaniline (381.04 mg, 2.005 mmol), N,N,N',N'-tetramethyl-O-(7-azabenzotriazol-1-yl)uronium hexafluorophosphate (1143.76 mg, 3.008 mmol), and N,N-diisopropylethylamine (0.994 mL, 6.016 mmol) were dissolved in N,N-dimethylformamide (5 mL) at room temperature, and the mixture was left to react at room temperature for 1 h in a nitrogen atmosphere. The reaction mixture was poured into a saturated

ammonium chloride solution (50 mL) and extracted with ethyl acetate (20 mL × 3). The organic phases were combined, washed with saturated brine (20 mL), dried over anhydrous sodium sulfate, filtered, and concentrated under reduced pressure. The residue was purified by column chromatography to give **34b** as a brown oil (400 mg, yield: 29.8%).

MS(ESI) m/z = 421.2[M+H]+.

Step 2: tert-butyl (1-(4-bromo-3-fluorophenyl)-2-oxopyrrolidin-3-yl)carbamate (**34c**)

Compound **34b** (100 mg, 0.237 mmol) was dissolved in iodomethane (5 mL) at room temperature, and the mixture was left to react overnight at room temperature in a nitrogen atmosphere. The reaction mixture was concentrated under reduced pressure, and the residue was dissolved in anhydrous tetrahydrofuran (10 mL). The system was cooled to 0 °C in a nitrogen atmosphere, and lithium bis(trimethylsilyl)amide (0.237 mL, 0.237 mmol) was then slowly added dropwise to the reaction system. The system was warmed to room temperature until the reaction was complete. The reaction mixture was poured into a saturated ammonium chloride solution (20 mL) and extracted with ethyl acetate (10 mL × 3). The organic phases were combined, washed with saturated brine (20 mL), dried over anhydrous sodium sulfate, filtered, and concentrated under reduced pressure. The residue was purified by column chromatography to give compound **34c** (50 mg, yield: 56.4%).

MS(ESI) m/z = 373.1[M+H]+.

Step 3: 3-amino-1-(4-bromo-3-fluorophenyl)pyrrolidin-2-one (**34d**)

Compound **34c** (50 mg, 0.134 mmol) was dissolved in a solution of hydrochloric acid in 1,4-dioxane (4 N in dioxane, 5 mL) at room temperature, and the mixture was left to react at room temperature for 2 h. The reaction mixture was directly concentrated under reduced pressure to give crude **34d** (50 mg, yield: 120.56%).

MS(ESI) m/z = 273.1[M+H]+.

Step 4: 1-(4-bromo-3-fluorophenyl)-3-(dimethylamino)pyrrolidin-2-one (**34e**)

Compound **34d** (50 mg, 0.183 mmol), formaldehyde (16.49 mg, 0.549 mmol), and acetic acid (0.052 mL, 0.915 mmol) were dissolved in methanol (5 mL) at room temperature. After 30 min of stirring at room temperature, sodium cyanoborohydride (23.01 mg, 0.366 mmol) was added, and the mixture was stirred at room temperature for another hour. The reaction mixture was poured into water (20 mL) and extracted with ethyl acetate (10 mL × 3). The organic phases were combined, washed with saturated brine (20 mL), dried over anhydrous sodium sulfate, filtered, and concentrated under reduced pressure. The residue was purified by column chromatography to give **34e** (25 mg, yield: 45.3%).

MS(ESI) m/z = 301.1[M+H]+.

Step 5: 1-(4-((5-chloro-4-((S)-8-fluoro-2-(2-hydroxypropan-2-yl)-3-methyl-3,4-dihydro-5-oxa-1,2a-diazaacenaphthylen-6-yl)pyrimidin-2-yl)amino)-3-fluorophenyl)-3-(dimethylamino)pyrrolidin-2-one (**34**)

Compound **34e** (25 mg, 0.066 mmol), **A10** (20 mg, 0.066 mmol), cesium carbonate (54.09 mg, 0.166 mmol), and Brettphos Pd G3 (1.50 mg, 0.002 mmol) were dissolved in 1,4-dioxane (3 mL) at room temperature. In a nitrogen atmosphere, the mixture was left to react in a microwave reactor at 100 °C for 1 h. The reaction mixture was poured into water (10 mL) and extracted with ethyl acetate (10 mL × 3). The organic phases were combined, washed with saturated brine (20 mL), dried over anhydrous sodium sulfate, filtered, and concentrated under reduced pressure. The residue was purified by C18 reversed-phase chromatography to give the target compound **34** (2.38 mg, yield: 6.0%).

MS(ESI) m/z = 598.3[M+H]+.

**Example 35**

(4-((5-Chloro-4-((S)-8-fluoro-2-(2-hydroxypropan-2-yl)-3-methyl-3,4-dihydro-5-oxa-1,2a-diazaacenaphthylen-6-yl)pyrimidin-2-yl)amino)2-fluorophenyl)((S)-3-(dimethylamino)pyrrolidin-1-yl)methanone (**35**)

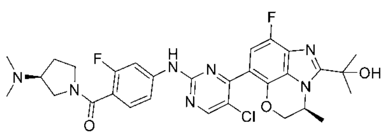

35

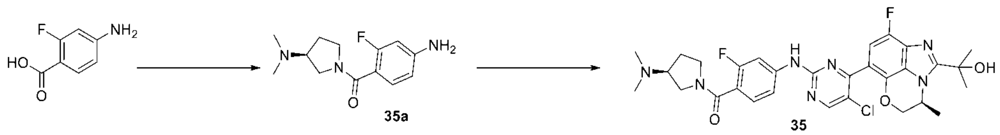


Step 1: (S)-(4-amino-2-fluorophenyl)(3-(dimethylamino)pyrrolidin-1-yl)methanone (**35a**)

4-Bromo-2-fluorobenzoic acid (100 mg, 0.46 mmol) was added to DMF (4 mL), and (3S)-3-(dimethylamino) tetrahydropyrrole (63.0 mg, 0.55 mmol), TBTU (220 mg, 0.68 mmol), and DIEA (118 mg, 0.91 mmol) were added at room temperature. The mixture was left to react at room temperature for 2 h in a nitrogen atmosphere. The reaction mixture was poured into water (50 mL) and extracted with ethyl acetate (10 mL × 2). After concentration, the residue was separated by column chromatography to give the product **35a** (91 mg, yield: 63.0%).

MS(ESI) m/z = 252.2[M+H]$^+$.

Step 2: (4-((5-chloro-4-((S)-8-fluoro-2-(2-hydroxypropan-2-yl)-3-methyl-3,4-dihydro-5-oxa-1,2a-diazaacenaphthylen-6-yl)pyrimidin-2-yl)amino)2-fluorophenyl)((S)-3-(dimethylamino)pyrrolidin-1-yl)methanone (**35**)

Compound **35a** (45 mg, 0.14 mmol) was added to dioxane (2.5 mL), and compound **A10** (53 mg, 0.14 mmol), cesium carbonate (91 g, 0.28 mmol), and XantPhos Pd G3 (9 mg, 0.01 mmol) were added at room temperature. In a nitrogen atmosphere, the mixture was left to react in a microwave reactor at 110 °C for 1.5 h. After the reaction mixture was filtered and concentrated, the residue was purified by C18 reversed-phase chromatography to give compound **35** (38.0 mg, yield: 44.1%).

MS(ESI) m/z = 612.2[M+H]$^+$.

$^1$H NMR (400 MHz, DMSO-$d_6$) δ 10.38 (s, 1H), 8.71 (d, 1H), 7.94-7.90 (m, 1H), 7.53-7.51 (m, 1H), 7.36-7.34 (m, 1H), 7.09 (dd, 1H), 5.83 (s, 1H), 5.26 (q, 1H), 4.51 (d, 1H), 4.28 (dd, 1H), 3.74 - 3.55 (m, 2H), 3.43-3.41 (m, 2H), 3.21 - 3.06 (m, 1H), 2.77 - 2.59 (m, 2H), 2.17 (s, 3H), 2.07 (s, 3H), 1.66 (d, 6H), 1.48 (d, 3H).

The compounds of Examples 36-42 below were synthesized using the method of Example 35.

**Example 36**

(2-Chloro-4-((5-chloro-4-((S)-8-fluoro-2-(2-hydroxypropan-2-yl)-3-methyl-3,4-dihydro-5-oxa-1,2a-diazaacenaphthylen-6-yl)pyrimidin-2-yl)amino)phenyl)((S)-3-(dimethylamino)pyrrolidin-1-yl)methanone (**36**)

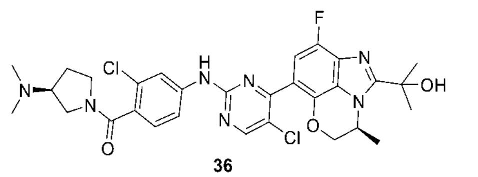

36

MS(ESI) m/z = 628.3[M+H]$^+$.

$^1$H NMR (400 MHz, DMSO) δ 10.31 (s, 1H), 8.70 (d, 1H), 8.06 (dd, 1H), 7.74-7.70 (m, 1H), 7.30 (dd, 1H), 7.08 (dd, 1H), 5.83 (s, 1H), 5.26 (q, 1H), 4.55 - 4.48 (m, 1H), 4.27 (dd, 1H), 3.78 - 3.58 (m, 2H), 3.32 - 3.10 (m, 2H), 2.99 (dd, 1H), 2.72 (dd, 1H), 2.17 (s, 3H), 2.07 (s, 3H), 1.80 - 1.65 (m, 1H), 1.68 (s, 3H), 1.63 (s, 3H), 1.48 (d, , 3H).

**Example 37**

(4-((5-Chloro-4-((S)-8-fluoro-2-(2-hydroxypropan-2-yl)-3-methyl-3,4-dihydro-5-oxa-1,2a-diazaacenaphthylen-6-yl)pyrimidin-2-yl)amino)-2-fluorophenyl)((R)-3-(dimethylamino)pyrrolidin-1-yl)methanone (**37**)

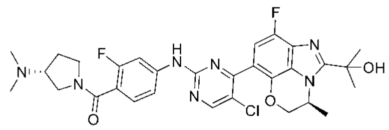

37

MS(ESI) m/z = 612.4[M+H]+.

$^{1}$H NMR (400 MHz, DMSO) δ 10.38 (s, 1H), 8.71 (d, 1H), 7.94-7.90 (m, 1H), 7.53-7.49 (m, 1H), 7.36-7.32 (m, 1H), 7.09 (dd, 1H), 5.80 (s, 1H), 5.28-5.24 (m, 1H), 4.55 - 4.47 (m, 1H), 4.32 - 4.24 (m, 1H), 3.74 - 3.55 (m, 2H), 3.46 - 3.27 (m, 2H), 3.21 - 3.06 (m, 1H), 2.77 - 2.60 (m, 1H), 2.17 (s, 3H), 2.07 (s, 3H), 1.80 - 1.64 (m, 1H), 1.68 (s, 3H), 1.63 (s, 3H), 1.48 (d, 3H).

**Example 38**

(2-Chloro-4-((5-chloro-4-((S)-8-fluoro-2-(2-hydroxypropan-2-yl)-3-methyl-3,4-dihydro-5-oxa-1,2a-diazaacenaphthylen-6-yl)pyrimidin-2-yl)amino)phenyl)((R)-3-(dimethylamino)pyrrolidin-1-yl)methanone (**38**)

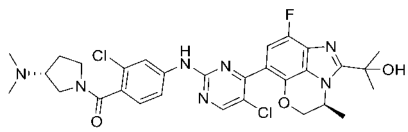

38

MS(ESI) m/z = 628.3[M+H]+.

$^{1}$H NMR (400 MHz, DMSO) δ 10.31 (s, 1H), 8.70 (d, 1H), 8.06 (dd, , 1H), 7.74-7.70 (m, 1H), 7.30 (dd, 1H), 7.08 (dd, 1H), 5.83 (s, 1H), 5.26 (q, 1H), 4.52 (d, 1H), 4.27 (d, 1H), 3.73-3.63 (m, 2H), 3.32 - 3.10 (m, 2H), 2.99 (dd, 1H), 2.72 (dd, 1H), 2.17 (s, 3H), 2.07 (s, 3H), 1.78 - 1.65 (m, 1H), 1.68 (s, 3H), 1.63 (s, 3H), 1.48 (d, *J* = 6.6 Hz, 3H).

**Example 39**

(4-((5-Chloro-4-((S)-8-fluoro-2-(2-hydroxypropan-2-yl)-3-methyl-3,4-dihydro-5-oxa-1,2a-diazaacenaphthylen-6-yl)pyrimidin-2-yl)amino)-3-fluorophenyl)((S)-3-(dimethylamino)pyrrolidin-1-yl)methanone (**39**)

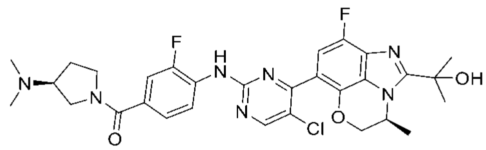

39

MS(ESI) m/z = 612.4[M+H]+.

**Example 40**

(4-((5-Chloro-4-((S)-8-fluoro-2-(2-hydroxypropan-2-yl)-3-methyl-3,4-dihydro-5-oxa-1,2a-diazaacenaphthylen-6-yl)pyrimidin-2-yl)amino)phenyl)((S)-3-(dimethylamino)pyrrolidin-1-yl)methanone (**40**)

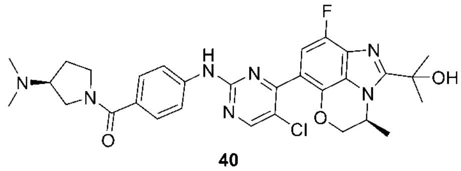

40

MS(ESI) m/z = 594.4[M+H]$^+$.

**Example 41**

(4-((5-Chloro-4-((S)-8-fluoro-2-(2-hydroxypropan-2-yl)-3-methyl-3,4-dihydro-5-oxa-1,2a-diazaacenaphthylen-6-yl)pyrimidin-2-yl)amino)-3-fluorophenyl)((R)-3-(dimethylamino)pyrrolidin-1-yl)methanone (**41**)

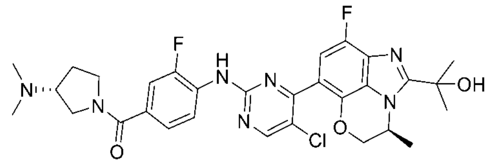

**41**

MS(ESI) m/z = 612.4[M+H]$^+$.

**Example 42**

(4-((5-Chloro-4-((S)-8-fluoro-2-(2-hydroxypropan-2-yl)-3-methyl-3,4-dihydro-5-oxa-1,2a-diazaacenaphthylen-6-yl)pyrimidin-2-yl)amino)phenyl)((R)-3-(dimethylamino)pyrrolidin-1-yl)methanone (**42**)

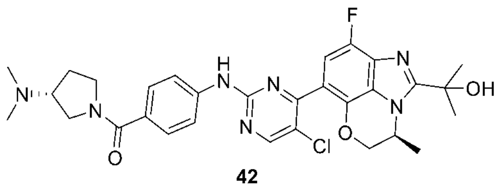

**42**

MS(ESI) m/z = 594.4[M+H]$^+$.

**Example 43**

(S)-2-(6-(5-Chloro-2-((2-fluoro-4-((4-methylpiperazin-1-yl)sulfonyl)phenyl)amino)pyrimidin-4-yl)-8-fluoro-3-methyl-3,4-dihydro-5-oxa-1,2a-diazaacenaphthylen-2-yl)propanol (**43**)

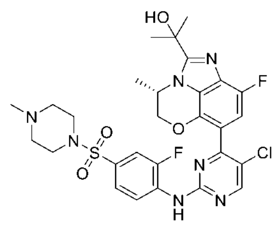

**43**

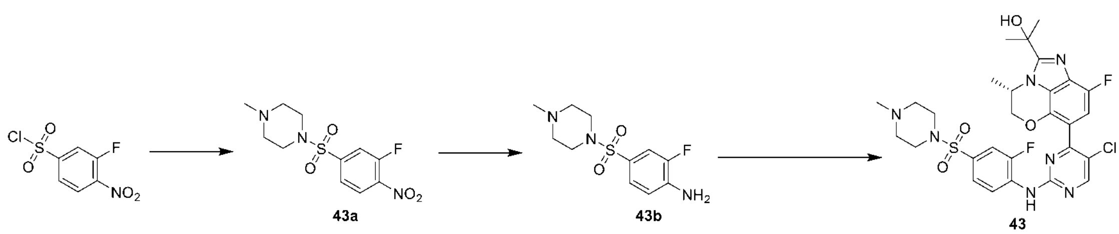

**43a** **43b** **43**

Step 1: 1-((3-fluoro-4-nitrophenyl)sulfonyl)-4-methylpiperazine (**43a**)

In a nitrogen atmosphere, 3-fluoro-4-nitrobenzenesulfonyl chloride (230 mg, 1.0 mmol) was dissolved in anhydrous dichloromethane (10 mL), and 4-methylpiperazine (150 mg, 1.5 mmol) was added. The mixture was left to react at room temperature for 1.5 h. The reaction mixture was concentrated, and the residue was separated by column chromatography to give compound **43a** (130 mg, yield: 42.8%).

MS(ESI) m/z = 304.2$[M+H]^+$.

Step 2: 2-fluoro-4-((4-methylpiperazin-1-yl)sulfonyl)aniline (**43b**)

Compound **43a** (150 mg, 0.5 mmol) was dissolved in ethanol:water (4 mL:1 mL) at room temperature, and ammonium chloride (53 mg, 1.0 mmol) was added. The mixture was heated to 60 °C, and iron powder (280 mg, 5 mmol) was added. The mixture was left to react for 2 h. After the reaction was complete, the reaction mixture was filtered through diatomaceous earth while hot, and the filtrate was concentrated. Water (5 mL) was added, and extraction was performed with ethyl acetate (15 mL × 2). The organic phases were combined and concentrated to give compound **43b** (70 mg, yield: 51.1%).

MS(ESI) m/z = 274.2$[M+H]^+$.

Step 3: (S)-2-(6-(5-chloro-2-((2-fluoro-4-((4-methylpiperazin-1-yl)sulfonyl)phenyl)amino)pyrimidin-4-yl)-8-fluoro-3-methyl-3,4-dihydro-5-oxa-1,2a-diazaacenaphthylen-2-yl)propanol (**43**)

Compound **43b** (55 mg, 0.2 mmol) was dissolved in 1,4-dioxane (3 mL), and compound **A10** (80 mg, 0.2 mmol), cesium carbonate (130 mg, 0.4 mmol), and Xantphos Pd G3 (18 mg, 0.02 mmol) were sequentially added. In a nitrogen atmosphere, the mixture was left to react in a microwave reactor at 110 °C for 1.5 h. The reaction mixture was filtered and concentrated under reduced pressure, and hydrochloric acid (2 N in EA) was directly added. The mixture was stirred at room temperature for 1 h and concentrated, and the residue was separated by preparative C18 reversed-phase chromatography to give the target product **43** (20 mg, yield: 15.9%).

MS(ESI) m/z = 634.3$[M+H]^+$.

**Example 44**

(S)-4-(4-((5-Chloro-4-(8-fluoro-2-(1-hydroxycyclopentyl)-3-methyl-3,4-dihydro-5-oxa-1,2a-diazaacenaphthylen-6-yl) pyrimidin-2-yl)amino)-3-fluorophenyl)morpholin-3-one (**44**)

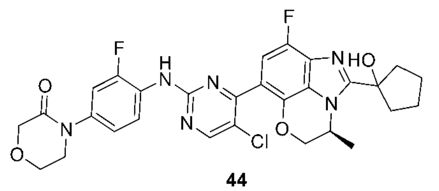

**44**

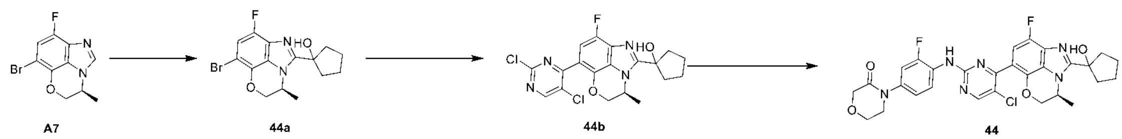

A7 44a 44b 44

The target compound **44** was obtained by referring to Example 28 and using A7 and cyclopentanone as the starting materials.

MS(ESI) m/z = 597.3$[M+H]^+$.

**Biological Assays**

The present disclosure is further described and explained below with reference to test examples. However, these examples are not intended to limit the scope of the present disclosure.

Test Example 1. Assay for Inhibitory Activity of Compounds of Present Disclosure Against Ovarian Cancer Cells (OVCAR3)

Table 1. Experimental materials and instruments

| Name | Manufacturer | Model/Cat. No. |
|---|---|---|
| RPMI 1640 culture medium | Gibco | 11875-093 |
| Serum | Gibco | 10091-148 |
| CTG Cell Viability Detection Reagent | MCE | HY-K0302-100ml |
| White culture plate, 384-well | Greiner | 781098 |
| Envision | PerkinElmer | 2015 |
| Plate shaker | Beijing Jiayuan | MB-100-2A |

1) Experimental steps

Ovarian cancer cells (OVCAR3) were cultured using an RPMI 1640 culture medium containing 10% FBS in a cell incubator (37 °C, 5% $CO_2$). On day one, the cells were plated in a 384-well plate at a cell concentration of 800 cells/well and cultured overnight in an incubator. On day two, the cells were treated with compounds. The highest compound concentration was 10 μM and was diluted 3-fold to obtain 9 concentrations, and the final concentration of DMSO was 0.1%. After the cells were cultured in the incubator for another 7 days, the cell viability was tested using a CTG Cell Viability Detection Reagent (MCE); the testing method was consistent with the method provided by the kit. Data were processed using GraphPad Prism 8, and $IC_{50}$ was calculated.

Y = Bottom + (Top - Bottom) / (1 + 10^((LogIC50 - X) × HillSlope)). Calculation formula:

X: the logarithm of the compound concentration; Y: inhibition%.

Table 2. The $IC_{50}$ (nM) of compounds of the present disclosure against OVCAR3

| Example No. | $IC_{50}$ (nM) |
|---|---|
| 1 | 671.7 |
| 2 | 47.5 |
| 3 | 197.2 |
| 4 | 347.6 |
| 5 | 89.9 |
| 6 | 80.2 |
| 7 | 669.5 |
| 9 | 752.1 |
| 10 | 230.5 |
| 11 | 673.1 |
| 12 | 643.3 |
| 13 | 900.8 |
| 15 | 635.7 |
| 16 | 222.5 |
| 17 | 153.6 |
| 18 | 102.9 |
| 19 | 275.8 |

(continued)

| Example No. | $IC_{50}$ (nM) |
|---|---|
| 21 | 259.7 |
| 22 | 824.7 |
| 24 | 255.1 |
| 25 | 409.3 |
| 26 | 517.1 |
| 27 | 572.5 |
| 28 | 137.2 |
| 29 | 335 |
| 32 | 426.4 |
| 35 | 201.5 |
| 36 | 961.2 |
| 38 | 690.2 |
| 40 | 363.6 |
| 42 | 674.2 |
| 44 | 574.3 |

Test Example 2. Assay for Inhibitory Activity of Compounds of Present Disclosure Against Triple-Negative Breast Cancer Cells (HCC1806)

1) Experimental materials and instruments

Table 3. Experimental materials and instruments

| Name | Manufacturer | Model/Cat. No. |
|---|---|---|
| RPMI 1640 culture medium | Gibco | 11875-093 |
| FBS | Gibco | 10091-148 |
| CTG Cell Viability Detection Reagent | MCE | HY-K0302-100ml |
| White culture plate, 384-well | Greiner | 781098 |
| Envision | PerkinElmer | 2015 |
| Plate shaker | Beijing Jiayuan | MB-100-2A |

2) Experimental steps

Triple-negative breast cancer cells (HCC1806, Nanjing Cobioer Biosciences Co., Ltd.) were cultured using an RPMI 1640 culture medium containing 10% FBS in a cell incubator (37 °C, 5% $CO_2$). On day one, the cells were plated in a 384-well plate at a cell concentration of 200 cells/well and cultured overnight in an incubator. On day two, the cells were treated with compounds. The highest compound concentration was 10 μM and was diluted 3-fold to obtain 9 concentrations, and the final concentration of DMSO was 0.2%. After the cells were cultured in the incubator for another 7 days, the cell viability was tested using a CTG Cell Viability Detection Reagent (MCE); the testing method was consistent with the method provided by the kit. Data were processed using XLfit, and $IC_{50}$ was calculated.

$Y = Bottom + (Top - Bottom) / (1 + 10^{(LogIC_{50} - X) \times HillSlope})$. Calculation formula:

X: the logarithm of the compound concentration; Y: inhibition%.

Table 4. The $IC_{50}$ (nM) of compounds of the present disclosure against HCC1806

| Example No. | HCC1806 $IC_{50}$ (nM) |
|---|---|
| 1 | 150.2 |
| 3 | 171.8 |
| 4 | 278.5 |
| 7 | 342.1 |
| 22 | 405.5 |
| 23 | 385.5 |
| 27 | 303.3 |
| 44 | 199.3 |
| PF-07220060 | 1593 |
| PF-07104091 | 936 |
| Note: PF-07220060 (WO2019207463A Example A94) and PF-07104091 (WO2020157652A Example13) were clinically selective CDK4 and CDK2 inhibitors by Pfizer, respectively. | |

In the experiment on this cell strain, Example 1 exhibited better inhibitory activity than the clinical molecules: the CDK4 inhibitor PF-07220060 and the CDK2 inhibitor PF-07104091.

Test Example 3. Assay for Activity of Compound of Present Disclosure Against Cyclin-Dependent Kinases (CDK2/CycE1 and CDK4/CycD1)

1) Experimental materials and instruments

Table 5. Experimental materials and instruments

| Name | Manufacturer | Model/Cat. No. |
|---|---|---|
| Kinase detector/microfluidic system | Perkin Elmer | EZ Reader |
| Centrifuge | Eppendorf | 5810R |
| Biochemical incubator | Shanghai Boxun | SPX-100B-Z |
| Echo | LABCYTE | C12527-07 |
| Automated microplate pipetting system | BioTek | PRC384U |
| Name of reagent | Manufacturer | Cat. No. |
| CDK2/CycE1 | Carna | 04-165 |
| CDK4/CycD 1 | Invitrogen | PR8064A |
| Peptide18 (CDK2/CycE1 substrate) | GL | 114202 |
| Peptide8 (CDK4/CycD1 substrate) | GL | 112396 |
| HEPES, pH7.5 | Gibco | 15630-080 |
| Brij-35 solution | Sigma | B4184 |
| EDTA | Gibco | 15575-038 |
| $MgCl_2$ | Sigma | M2670-500g |
| DTT | Sigma | D0632-10G |

2) Experimental steps

The test compounds (the highest concentration was 1 μM) were added using Echo 650 and serially diluted 3-fold to obtain a total of 9 concentration points. The assay plate was sealed and centrifuged at 1000 g for 1 min. 2.5× enzymes were prepared in a 1× kinase buffer (50 mM HEPES, pH 7.5, 0.0015% Brij-35, 1 M DTT), and the 2.5× enzymes (10 μL) were added to the 384-well assay plate. The plate was centrifuged at 1000 g for 30 s and left to stand at room temperature for 10 min. 2.5× substrate and ATP mixtures were prepared in the 1× kinase buffer, and the 2.5× substrate and ATP mixtures (10 μL) were added to start reactions. The assay plate was centrifuged at 1000 g for 30 s, sealed, and incubated at room temperature for 1 h. 25 μL of a stop solution reagent (100 mM HEPES, pH 7.5, 0.015% Brij-35, 0.2% Coating Reagent #3, 50 mM EDTA) was added.

Conversion rate data were read on a CaliperEZ Reader. The conversion rate data were copied from the CaliperEZ Reader. The conversion rates were converted to inhibition rate data.

$$\text{Inhibition\%} = (\text{max - conversion}) / (\text{max - min}) \times 100,$$

where "min" is the reading of a control sample well to which no enzyme was added for reaction; "max" is the reading of a control well to which DMSO was added.

$IC_{50}$ values were obtained by fitting using XLFit excel add-in version 5.4.0.8.

$$\text{Fitting formula: } Y = \text{Bottom} + (\text{Top} - \text{Bottom}) / (1 + (IC_{50} / X)^\wedge\text{HillSlope}).$$

Table 6. The $IC_{50}$ values of a compound of the present disclosure against CDK2/CycE1 and CDK4/CycD1

| Example No. | CDK2/CycE1 $IC_{50}$ (nM) | CDK4/CycD1 $IC_{50}$ (nM) |
|---|---|---|
| Example 1 | 9.8 | 3.8 |
| Comparative Example 1 | >1000 | 3.4 |
| Note: Example 1 of CN116217588A was synthesized according to the patent and used as Comparative Example 1 of the present application. | | |

Compound 1 exhibited relatively good inhibitory activity against CDK4/CycD1 and CDK2/CycE1. Example 1 exhibited stronger inhibitory activity against CDK2/CycE1 than Comparative Example 1 and thus potentially had the effect of reducing resistance to some CDK4/6 inhibitors. Example 1 may also have a wider range of indications.

Test Example 4. Assay for Inhibitory Activity of Compound of Present Disclosure Against Human Breast Cancer Cells (MDA-MB-231)

Table 7. Experimental materials and instruments

| Name | Manufacturer | Model/Cat. No. |
|---|---|---|
| DMEM culture medium | Gibco | 11965-092 |
| FBS | Gibco | 10091-148 |
| CTG Cell Viability Detection Reagent | MCE | HY-K0302-100ml |
| White culture plate, 384-well | Greiner | 781098 |
| Envision | PerkinElmer | 2015 |
| Plate shaker | Beijing Jiayuan | MB-100-2A |

Experimental steps

Human breast cancer cells (MDA-MB-231) were cultured using a DMEM culture medium containing 10% FBS in

a cell incubator (37 °C, 5% $CO_2$). On day one, the cells were plated in a 384-well plate at a cell concentration of 600 cells/well and cultured overnight in an incubator. On day two, the cells were treated with compounds. The highest compound concentration was 10 μM and was diluted 3-fold to obtain 9 concentrations, and the final concentration of DMSO was 0.2%. After the cells were cultured in the incubator for another 7 days, the cell viability was tested using a CTG Cell Viability Detection Reagent; the testing method was consistent with the method provided by the kit. Data were processed using XLFit, and $IC_{50}$ was calculated.

$$Y = Bottom + (Top - Bottom)/(1 + 10^\wedge((LogIC50 - X) \times HillSlope)).$$ Calculation formula:

X: the logarithm of the compound concentration; Y: % inhibition.

Table 8. The $IC_{50}$ (nM) of a compound of the present disclosure against MDA-MB-231

| Example No. | $IC_{50}$ (nM) |
|---|---|
| 1 | 116.8 |
| PF-07220060 | 567 |
| PF-07104091 | 1251.8 |

In the experiment on this cell strain, Example 1 exhibited better inhibitory activity than the clinical molecules: the CDK4 inhibitor PF-07220060 and the CDK2 inhibitor PF-07104091.

Test Example 5. Assay for Inhibitory Activity of Compound of Present Disclosure Against Human Non-Small Cell Lung Cancer Cells (A549)

Table 9. Experimental materials and instruments

| Name | Manufacturer | Model/Cat. No. |
|---|---|---|
| F12K culture medium | Gibco | 21127-022 |
| FBS | Gibco | 10091-148 |
| CTG Cell Viability Detection Reagent | MCE | HY-K0302-100ml |
| White culture plate, 384-well | Greiner | 781098 |
| Envision | PerkinElmer | 2015 |
| Plate shaker | Beijing Jiayuan | MB-100-2A |

Experimental steps

Human non-small cell lung cancer cells (A549) were cultured using an F12K culture medium containing 10% FBS in a cell incubator (37 °C, 5% $CO_2$). On day one, the cells were plated in a 384-well plate at a cell concentration of 300 cells/well and cultured overnight in an incubator. On day two, the cells were treated with compounds. The highest compound concentration was 10 μM and was diluted 3-fold to obtain 9 concentrations, and the final concentration of DMSO was 0.2%. After the cells were cultured in the incubator for another 5 days, the cell viability was tested using a CTG Cell Viability Detection Reagent; the testing method was consistent with the method provided by the kit. Data were processed using XLFit, and $IC_{50}$ was calculated.

$$Y = Bottom + (Top - Bottom) / (1 + 10^\wedge((LogIC50 - X) \times HillSlope)).$$ Calculation formula:

X: the logarithm of the compound concentration; Y: % inhibition.

Table 10. The $IC_{50}$ (nM) of a compound of the present disclosure against A549

| Example No. | $IC_{50}$ (nM) |
|---|---|
| 1 | 236.4 |

(continued)

| Example No. | $IC_{50}$ (nM) |
|---|---|
| PF-07220060 | 1874.4 |
| PF-07104091 | 1132 |

In the experiment on this cell strain, Example 1 exhibited better inhibitory activity than the clinical molecules: the CDK4 inhibitor PF-07220060 and the CDK2 inhibitor PF-07104091.

Test Example 6. Assay for Inhibitory Activity of Compound of Present Disclosure Against Human Prostate Cancer Cells (22RV1)

Table 11. Experimental materials and instruments

| Name | Manufacturer | Model/Cat. No. |
|---|---|---|
| RPMI 1640 culture medium | Gibco | 11875-093 |
| FBS | Gibco | 10091-148 |
| CTG Cell Viability Detection Reagent | MCE | HY-K0302-100ml |
| White culture plate, 384-well | Greiner | 781098 |
| Envision | PerkinElmer | 2015 |
| Plate shaker | Beijing Jiayuan | MB-100-2A |

Experimental steps

Human prostate cancer cells (22RV1) were cultured using an RPMI 1640 culture medium containing 10% FBS in a cell incubator (37 °C, 5% $CO_2$). On day one, the cells were plated in a 384-well plate at a cell concentration of 500 cells/well and cultured overnight in an incubator. On day two, the cells were treated with compounds. The highest compound concentration was 10 μM and was diluted 3-fold to obtain 9 concentrations, and the final concentration of DMSO was 0.2%. After the cells were cultured in the incubator for another 5 days, the cell viability was tested using a CTG Cell Viability Detection Reagent; the testing method was consistent with the method provided by the kit. Data were processed using XLFit, and $IC_{50}$ was calculated.

Calculation formula: Y = Bottom + (Top - Bottom) / (1 + 10^((LogIC50 - X) × HillSlope)).

X: the logarithm of the compound concentration; Y: % inhibition.

Table 12. The $IC_{50}$ (nM) of a compound of the present disclosure against 22RV1

| Example No. | $IC_{50}$ (nM) |
|---|---|
| 1 | 80.3 |
| PF-07104091 | 2703.7 |

In the experiment on this cell strain, Example 1 exhibited better inhibitory activity than the clinical molecule: the CDK2 inhibitor PF-07104091.

Test Example 7. Assay for Inhibitory Activity of Compound of Present Disclosure Against Human Breast Cancer Cells (SUM-149PT)

Table 13. Experimental materials and instruments

| Name | Manufacturer | Model/Cat. No. |
|---|---|---|
| DMEM culture medium | Gibco | 11965-092 |
| FBS | Gibco | 10091-148 |
| CTG Cell Viability Detection Reagent | MCE | HY-K0302-100ml |
| White culture plate, 384-well | Greiner | 781098 |
| Envision | PerkinElmer | 2015 |
| Plate shaker | Beijing Jiayuan | MB-100-2A |

Experimental steps

Human breast cancer cells (SUM-149PT) were cultured using a DMEM culture medium containing 10% FBS in a cell incubator (37 °C, 5% $CO_2$). On day one, the cells were plated in a 384-well plate at a cell concentration of 800 cells/well and cultured overnight in an incubator. On day two, the cells were treated with compounds. The highest compound concentration was 10 μM and was diluted 3-fold to obtain 9 concentrations, and the final concentration of DMSO was 0.2%. After the cells were cultured in the incubator for another 5 days, the cell viability was tested using a CTG Cell Viability Detection Reagent; the testing method was consistent with the method provided by the kit. Data were processed using XLFit, and $IC_{50}$ was calculated.

Y = Bottom + (Top - Bottom) / (1 + 10^((LogIC50 - X) × HillSlope)). Calculation formula:

X: the logarithm of the compound concentration; Y: % inhibition.

Table 14. The $IC_{50}$ (nM) of a compound of the present disclosure against SUM-149PT

| Example No. | $IC_{50}$ (nM) |
|---|---|
| 1 | 255.6 |
| PF-07104091 | 1336.3 |

In the experiment on this cell strain, Example 1 exhibited better inhibitory activity than the clinical molecule: the CDK2 inhibitor PF-07104091.

Test Example 8. Assay for Inhibitory Activity of Compound of Present Disclosure Against CDK4/6 Inhibitor-Resistant Human Breast Cancer Cells (MCF-7-Palbociclib-Resistant Cell Line)

1) Experimental materials and instruments

Table 15. Experimental materials and instruments

| **Name** | **Source** | **Model/Cat. No.** |
|---|---|---|
| DMEM | Gibco | 11965-092 |
| FBS | Gibco | 10091-148 |
| DMSO | SIGMA | D2650 |
| CTG Cell Viability Detection Reagent | MCE | HY-K0302-100ml |
| White culture plate, 384-well | Greiner | 781098 |
| Envision | PerkinElmer | 2104 |
| Plate shaker | Thermo | 88880024 |
| Palbociclib | MCE | HY-50767 |

2) Experimental steps

MCF-7/Palbo-R cell source: MCF7 cells (Nanjing Cobioer Biosciences Co., Ltd.) were treated with 1 μM palbociclib for a long time, and drug resistance was induced.

The palbociclib-resistant breast cancer cell line MCF-7/Palbo-R was cultured using a DMEM culture medium containing 10% FBS in a cell incubator (37 °C, 5% $CO_2$). On day one, the cells were plated in a 384-well plate at a cell concentration of 800 cells/well and cultured overnight in an incubator. On day two, the cells were treated with compounds. The highest compound concentration was 10 μM and was diluted 3-fold to obtain 9 concentrations, and the final concentration of DMSO was 0.2%. After the cells were cultured in the incubator for another 5 days, the cell viability was tested using a CellTiter-Glo luminescent cell viability assay kit; the testing method was consistent with the method provided by the kit. Data were processed using XLfit, and $IC_{50}$ was calculated.

The inhibition rates (IR) of the test compounds were calculated using the following formula: IR (%) = (1 - (RLU compound - RLU blank control) / (RLU vehicle control-RLU blank control)) × 100%.

Table 16. The $IC_{50}$ (nM) of a compound of the present disclosure against MCF-7/Palbo-R

| Example No. | $IC_{50}$ (nM) |
|---|---|
| 1 | 180 |
| Palbociclib | >2000 |
| PF-07220060 | 967 |
| PF-07104091 | 836 |

In the experiment on this cell strain, the molecule of Example 1 exhibited better inhibitory activity than the clinical molecules: the CDK4 inhibitor PF-07220060 and the CDK2 inhibitor PF-07104091.

## Claims

1. A compound represented by formula I or a pharmaceutically acceptable salt thereof,

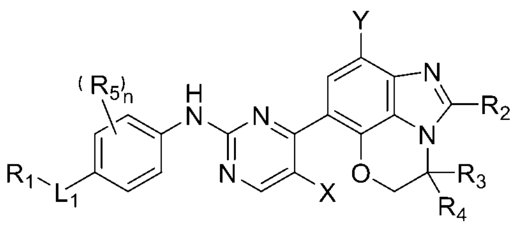

I

wherein:

$R_2$ is selected from the group consisting of hydrogen, $C_{1-6}$ alkyl, $C_{1-6}$ alkoxy, 3-8 membered cycloalkyl, and 3-12 membered heterocycloalkyl, and the $C_{1-6}$ alkyl, $C_{1-6}$ alkoxy, 3-8 membered cycloalkyl, and 3-12 membered heterocycloalkyl are optionally substituted with one or more $R_{21}$;

each $R_{21}$ is independently selected from the group consisting of deuterium, halogen, hydroxy, amino, $C_{1-3}$ alkoxy, 3-7 membered cycloalkyl, and 3-7 membered heterocycloalkyl, and the $C_{1-3}$ alkoxy, 3-7 membered cycloalkyl, and 3-7 membered heterocycloalkyl are optionally substituted with one or more substituents selected from the group consisting of hydroxy, halogen, and deuterium;

$R_3$ and $R_4$ are each independently selected from the group consisting of hydrogen, cyano, halogen, $C_{1-6}$ alkyl, 3-8 membered cycloalkyl, $C_{1-6}$ alkoxy, and 3-12 membered heterocycloalkyl, and the $C_{1-6}$ alkyl, 3-8 membered cycloalkyl, $C_{1-6}$ alkoxy, and 3-12 membered heterocycloalkyl are optionally substituted with one or more $R_{31}$;

each $R_{31}$ is independently selected from the group consisting of halogen, hydroxy, amino, $C_{1-3}$ alkoxy, 3-7 membered cycloalkyl, and 3-7 membered heterocycloalkyl, and the $C_{1-3}$ alkoxy, 3-7 membered cycloalkyl, and 3-7 membered heterocycloalkyl are optionally substituted with one or more substituents selected from the group consisting of hydroxy and halogen;

Y is selected from the group consisting of hydrogen, halogen, cyano, $C_{1-6}$ alkyl, and $C_{1-6}$ haloalkyl;
X is selected from the group consisting of hydrogen, halogen, cyano, $C_{1-6}$ alkyl, and $C_{1-6}$ alkoxy, and the $C_{1-6}$ alkyl and $C_{1-6}$ alkoxy are optionally substituted with one or more $R^a$;
each $R^a$ is independently selected from the group consisting of halogen, hydroxy, amino, $C_{1-3}$ alkoxy, 3-7 membered cycloalkyl, and 3-7 membered heterocycloalkyl, and the $C_{1-3}$ alkoxy, 3-7 membered cycloalkyl, and 3-7 membered heterocycloalkyl are optionally substituted with one or more substituents selected from the group consisting of hydroxy and halogen;
$L_1$ is selected from the group consisting of -CO- and a bond;
$R_1$ is selected from the group consisting of $C_{1-6}$ alkyl, heterocycloalkyl, heteroaryl, - CO-NH-$R_{11}$, -S(O)$_2$-NH-$R_{12}$,

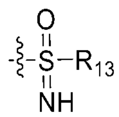 , and 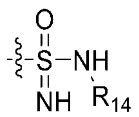 ,

and the $C_{1-6}$ alkyl, heterocycloalkyl, heteroaryl, -CO-NH-$R_{11}$, -S(O)$_2$-NH-$R_{12}$,

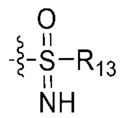 , and 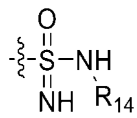

are optionally substituted with one or more $R^b$;
$R_{11}$, $R_{12}$, $R_{13}$, and $R_{14}$ are each independently selected from the group consisting of $C_{1-6}$ alkyl, 3-7 membered cycloalkyl, and 3-7 membered heterocycloalkyl;
each $R^b$ is independently selected from the group consisting of $C_{1-6}$ alkyl, $C_{1-6}$ alkoxy, $C_{1-6}$ haloalkyl, $C_{1-6}$ haloalkoxy, halogen, hydroxy, cyano, oxo, and -N($R^c$)$_2$;
each $R^c$ is independently selected from the group consisting of hydrogen, $C_{1-6}$ alkyl, and $C_{1-6}$ haloalkyl;
or the two $R^c$, together with the N atom to which they are attached, form 3-6 membered heterocycloalkyl, and the 3-6 membered heterocycloalkyl is optionally substituted with one or more halogens;
each $R_5$ is independently selected from the group consisting of hydroxy, cyano, halogen, $C_{1-6}$ alkyl, 3-8 membered cycloalkyl, $C_{1-6}$ alkoxy, and 3-12 membered heterocycloalkyl, and the $C_{1-6}$ alkyl, 3-8 membered cycloalkyl, $C_{1-6}$ alkoxy, and 3-12 membered heterocycloalkyl are optionally substituted with one or more $R_{51}$;
each $R_{51}$ is independently selected from the group consisting of halogen, hydroxy, amino, $C_{1-3}$ alkoxy, 3-7 membered cycloalkyl, and 3-7 membered heterocycloalkyl, and the $C_{1-3}$ alkoxy, 3-7 membered cycloalkyl, and 3-7 membered heterocycloalkyl are optionally substituted with one or more substituents selected from the group consisting of hydroxy and halogen;
n is selected from the group consisting of 0, 1, 2, 3, and 4.

**2.** The compound represented by formula I or the pharmaceutically acceptable salt thereof according to claim 1, wherein $L_1$ is a bond.

**3.** The compound represented by formula I or the pharmaceutically acceptable salt thereof according to claim 1, wherein $L_1$ is -CO-.

**4.** The compound represented by formula I or the pharmaceutically acceptable salt thereof according to any one of claims 1-3, wherein $R_1$ is selected from the group consisting of 5-7 membered heterocycloalkyl optionally substituted with one or more $R^b$; preferably, $R_1$ is selected from the group consisting of 6-membered heterocycloalkyl optionally substituted with one or more $R^b$; more preferably, $R_1$ is selected from the group consisting of

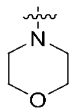 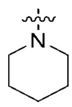 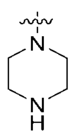 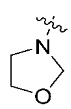 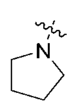

optionally substituted with one or more $R^b$;
wherein $R^b$ is as defined in claim 1.

**5.** The compound represented by formula I or the pharmaceutically acceptable salt thereof according to any one of claims 1-3, wherein $R_1$ is selected from the group consisting of $C_{1-6}$ alkyl, 5-6 membered heteroaryl, -CO-NH-$R_{11}$, -$S(O)_2$-NH-$R_{12}$,

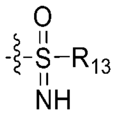 , and 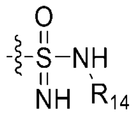 ,

and the $C_{1-6}$ alkyl, 5-6 membered heteroaryl, -CO-NH-$R_{11}$,-$S(O)_2$-NH-$R_{12}$,

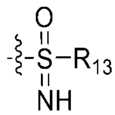 , and 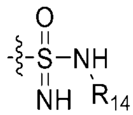

are optionally substituted with one or more $R^b$;
$R_{11}$, $R_{12}$, $R_{13}$, and $R_{14}$ are each independently selected from the group consisting of $C_{1-6}$ alkyl, 3-7 membered cycloalkyl, and 3-7 membered heterocycloalkyl; preferably, $R_{11}$, $R_{12}$, $R_{13}$, and $R_{14}$ are each independently selected from the group consisting of $C_{1-6}$ alkyl; wherein $R^b$ is as defined in claim 1.

**6.** The compound represented by formula I or the pharmaceutically acceptable salt thereof according to any one of claims 1-3, wherein each $R^b$ is independently selected from the group consisting of $C_{1-6}$ alkyl, $C_{1-6}$ alkoxy, $C_{1-6}$ haloalkyl, $C_{1-6}$ haloalkoxy, halogen, hydroxy, cyano, and oxo.

**7.** The compound represented by formula I or the pharmaceutically acceptable salt thereof according to claim 1, being a compound represented by formula II or a pharmaceutically acceptable salt thereof,

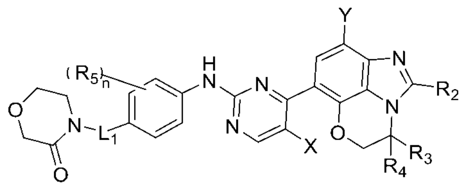

II

wherein $R_2$, $R_3$, $R_4$, $R_5$, X, Y, $L_1$, and n are as defined in claim 1.

**8.** The compound represented by formula I or the pharmaceutically acceptable salt thereof according to any one of claims 1-7, wherein $R_2$ is selected from the group consisting of hydrogen, $C_{1-6}$ alkyl, and 3-8 membered cycloalkyl, and the $C_{1-6}$ alkyl and 3-8 membered cycloalkyl are optionally substituted with one or more $R_{21}$;
each $R_{21}$ is independently selected from the group consisting of deuterium, halogen, hydroxy, $C_{1-3}$ alkoxy, 3-7 membered cycloalkyl, and 3-7 membered heterocycloalkyl, and the $C_{1-3}$ alkoxy, 3-7 membered cycloalkyl, and 3-7 membered heterocycloalkyl are optionally substituted with one or more substituents selected from the group consisting of hydroxy, halogen, and deuterium.

**9.** The compound represented by formula I or the pharmaceutically acceptable salt thereof according to any one of claims 1-8, wherein $R_3$ and $R_4$ are each independently selected from the group consisting of hydrogen, cyano, halogen, and $C_{1-6}$ alkyl, and the $C_{1-6}$ alkyl is optionally substituted with one or more $R_{31}$;
each $R_{31}$ is independently selected from the group consisting of halogen, hydroxy, amino, and $C_{1-3}$ alkoxy, and the $C_{1-3}$ alkoxy is optionally substituted with one or more substituents selected from the group consisting of hydroxy and halogen.

**10.** The compound represented by formula I or the pharmaceutically acceptable salt thereof according to any one of claims 1-9, wherein Y is selected from the group consisting of halogen; preferably, Y is selected from the group consisting of fluorine.

**11.** The compound represented by formula I or the pharmaceutically acceptable salt thereof according to any one of claims 1-10, wherein X is selected from the group consisting of hydrogen, halogen, and cyano; preferably, X is selected from the group consisting of fluorine and chlorine.

**12.** The compound represented by formula I or the pharmaceutically acceptable salt thereof according to any one of claims 1-11, wherein each $R_5$ is independently selected from the group consisting of hydroxy, cyano, halogen, $C_{1-6}$ alkyl, 3-8 membered cycloalkyl, $C_{1-6}$ alkoxy, and 3-6 membered heterocycloalkyl, and the $C_{1-6}$ alkyl, 3-8 membered cycloalkyl, $C_{1-6}$ alkoxy, and 3-6 membered heterocycloalkyl are optionally substituted with one or more $R_{51}$;
each $R_{51}$ is independently selected from the group consisting of halogen, hydroxy, amino, $C_{1-3}$ alkoxy, 3-7 membered cycloalkyl, and 3-7 membered heterocycloalkyl, and the $C_{1-3}$ alkoxy, 3-7 membered cycloalkyl, and 3-7 membered heterocycloalkyl are optionally substituted with one or more substituents selected from the group consisting of hydroxy and halogen.

**13.** A compound represented by the following formula or a pharmaceutically acceptable salt thereof:

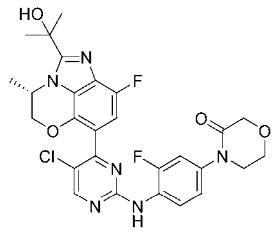 , 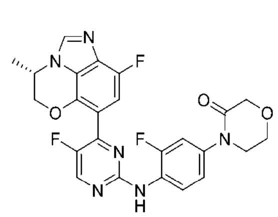 , 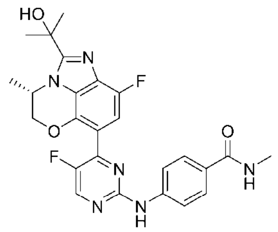 ,

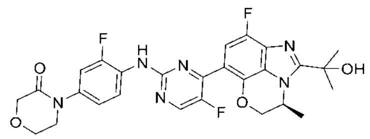 , 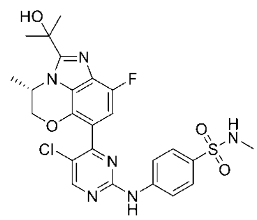 , 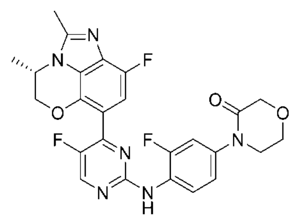 ,

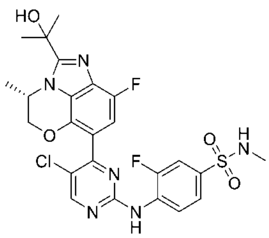 , 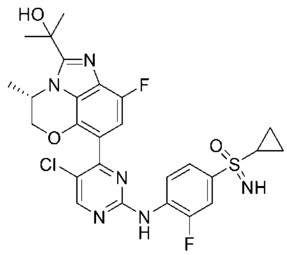 , 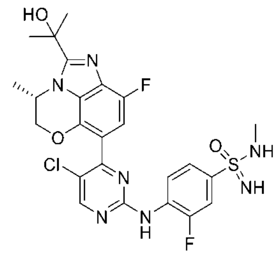 ,

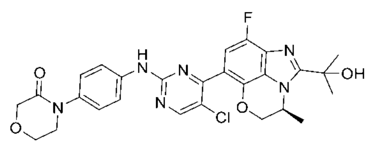 , 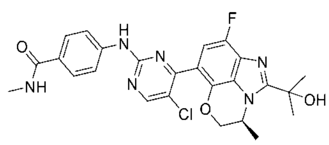 , 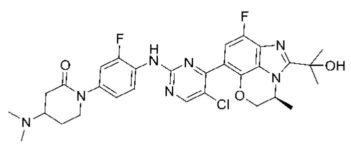 ,

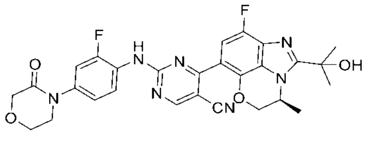 , 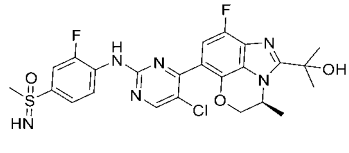 , 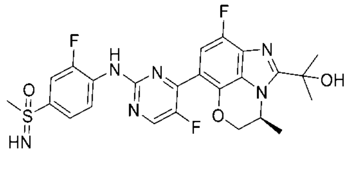 ,

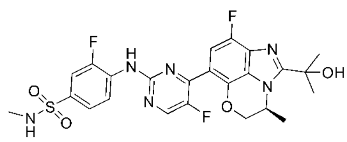, 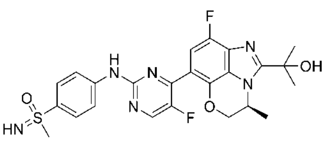, 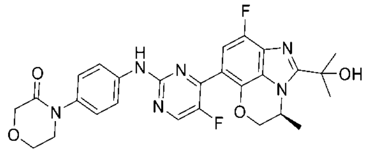,

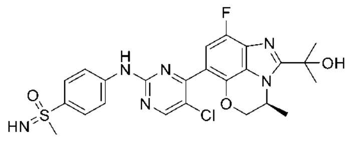, 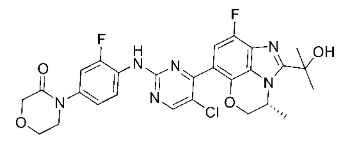, 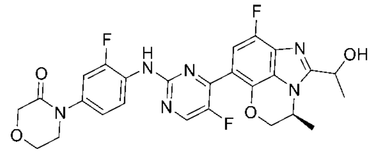,

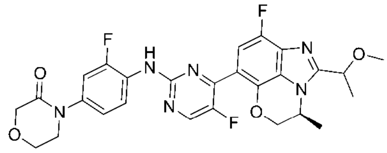, 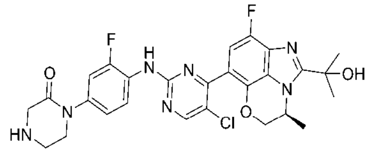, 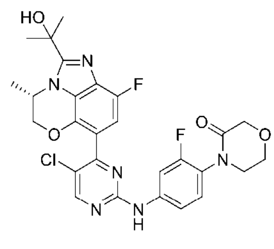,

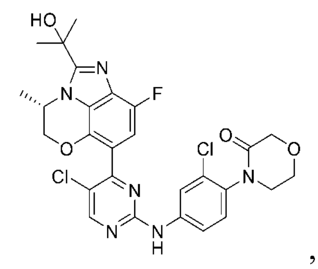, 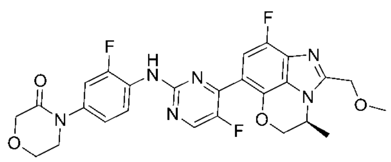, 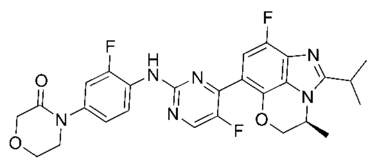,

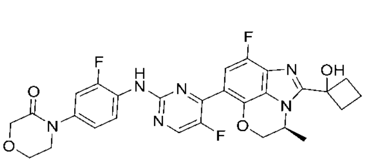, 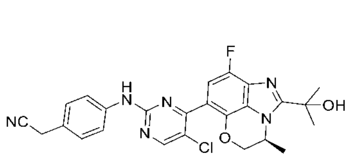, 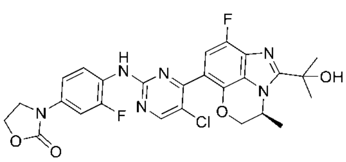,

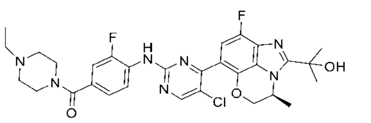, 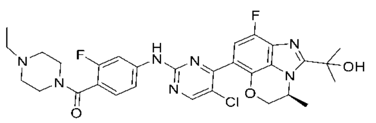,

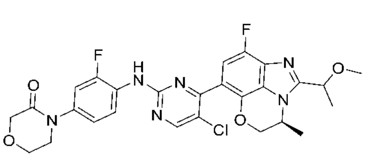, 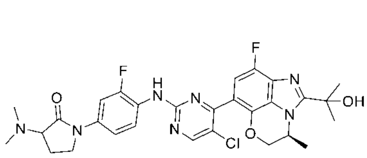, 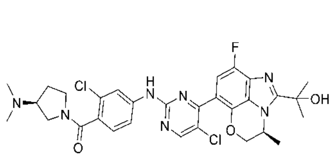,

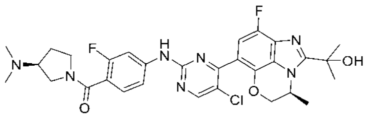, 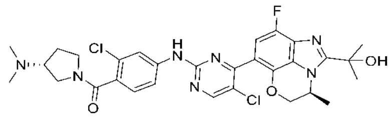,

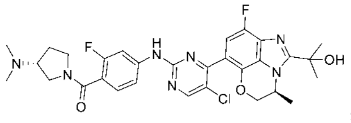,
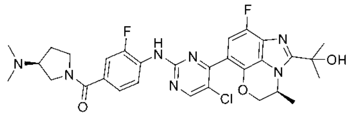,
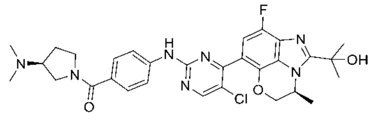,

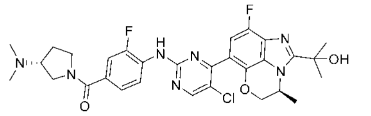,
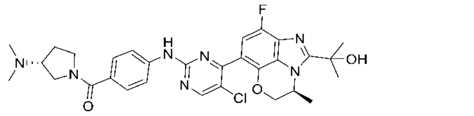,

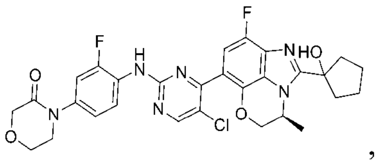,
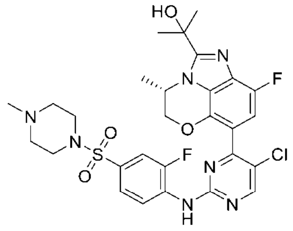,
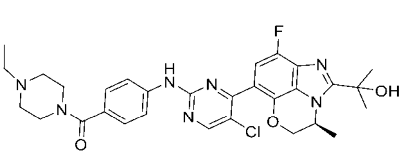,

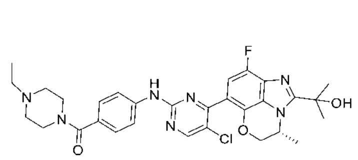,
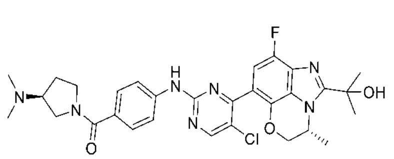,
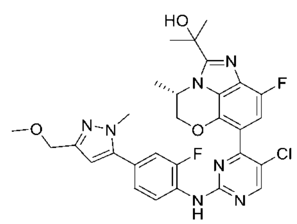,

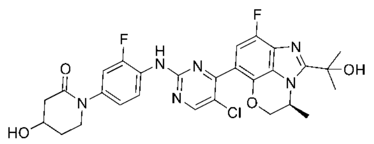,
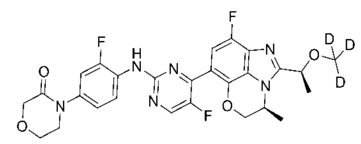,

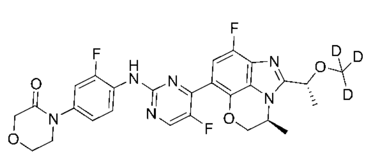,
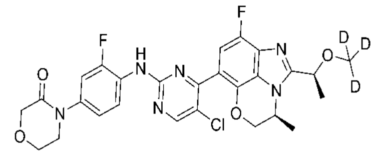,

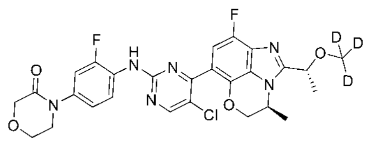,
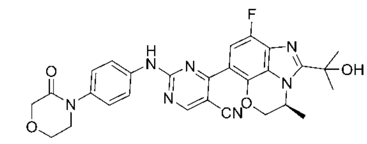,

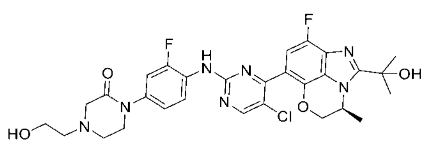,
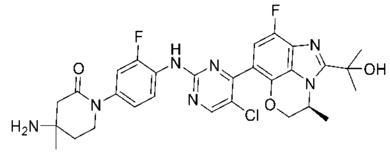,

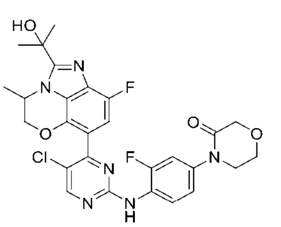,
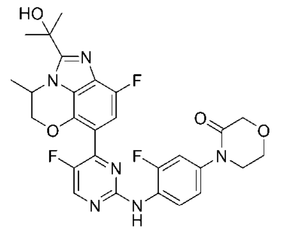,
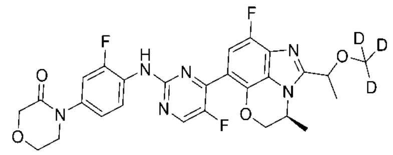,

or

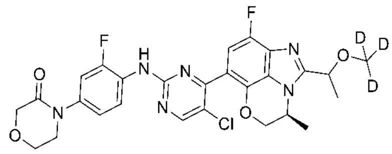.

**14.** An isotopically substituted form of the compound or the pharmaceutically acceptable salt thereof according to any one of claims 1-13, wherein preferably, the isotopically substituted form is deuterium-substituted.

**15.** A pharmaceutical composition, comprising at least one of the compound or the pharmaceutically acceptable salt thereof according to any one of claims 1-13 or the isotopically substituted form according to claim 14, and a pharmaceutically acceptable excipient.

**16.** Use of the compound or the pharmaceutically acceptable salt thereof according to any one of claims 1-13 or the isotopically substituted form according to claim 14 or the pharmaceutical composition according to claim 15 in the manufacture of a medicament for treating or preventing a disease or disorder associated with abnormal activity of a serine/threonine kinase.

**17.** Use of the compound or the pharmaceutically acceptable salt thereof according to any one of claims 1-13 or the isotopically substituted form according to claim 14 or the pharmaceutical composition according to claim 15 in the manufacture of a medicament for treating or preventing a disease or disorder associated with abnormal activity of CDK2.

**18.** Use of the compound or the pharmaceutically acceptable salt thereof according to any one of claims 1-13 or the isotopically substituted form according to claim 14 or the pharmaceutical composition according to claim 15 in the manufacture of a medicament for treating or preventing a disease or disorder associated with abnormal activity of CDK4.

**19.** Use of the compound or the pharmaceutically acceptable salt thereof according to any one of claims 1-13 or the isotopically substituted form according to claim 14 or the pharmaceutical composition according to claim 15 in the manufacture of a medicament for treating or preventing a disease or disorder, wherein the disease or disorder is selected from the group consisting of proliferative diseases, inflammatory diseases, auto inflammatory diseases, autoimmune diseases, and infectious diseases; preferably, the disease or disorder is a proliferative disease; most preferably, the proliferative disease is selected from the group consisting of breast cancer (e.g., triple-negative breast cancer or ER-negative, PR-negative, and Her2-positive breast cancer or CDK4/6 inhibitor-resistant breast cancer), intestinal cancer, lung cancer (e.g., non-small cell lung cancer), pancreatic cancer, prostate cancer, Ewing sarcoma, osteoma, neuroblastoma, cervical cancer, ovarian cancer, gastric cancer, and liver cancer.

**20.** A preparation method for the compound or the pharmaceutically acceptable salt thereof according to any one of claims 1 to 13 or the isotopically substituted form according to claim 14, comprising a step of reacting a compound represented by formula A with a compound represented by formula B,

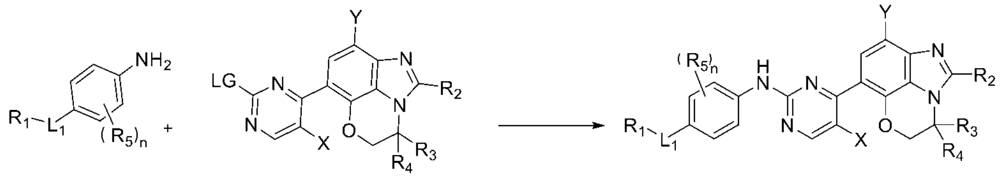

A B I

wherein $R_1$, $R_2$, $R_3$, $R_4$, $R_5$, X, Y, $L_1$, and n are as defined in claims 1-13;
wherein LG is selected from the group consisting of leaving groups; preferably, LG is selected from the group consisting of halogen, sulfonate, and borate.

**21.** A compound represented by formula B or a pharmaceutically acceptable salt thereof,

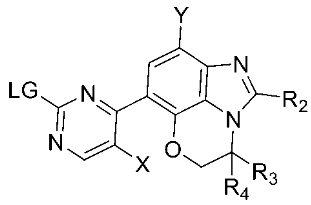

B

wherein $R_2$, $R_3$, $R_4$, X, and Y are as defined in claims 1-13;
wherein LG is selected from the group consisting of leaving groups; preferably, LG is selected from the group consisting of halogen, sulfonate, and borate.

**22.** A compound represented by formula A or a pharmaceutically acceptable salt thereof,

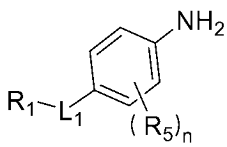

A

wherein $R_1$, $R_5$, $L_1$, and n are as defined in claims 1-13.

**TRANSLATION**

## INTERNATIONAL SEARCH REPORT

International application No.
**PCT/CN2024/107786**

**A. CLASSIFICATION OF SUBJECT MATTER**

C07D 498/06(2006.01)i； A61P 35/00(2006.01)i； A61K 31/5383(2006.01)i

According to International Patent Classification (IPC) or to both national classification and IPC

**B. FIELDS SEARCHED**

Minimum documentation searched (classification system followed by classification symbols)

IPC：C07D；A61P；A61K

Documentation searched other than minimum documentation to the extent that such documents are included in the fields searched

Electronic data base consulted during the international search (name of data base and, where practicable, search terms used)

CNTXT, ENTXTC, DWPI, CNKI, STN (CAPLUS, REGISTRY): 含氧稠三环, 嘧啶胺, CDK抑制剂, 癌, 肿瘤, Oxygen-containing fused tricyclic, aminopyrimidine, CDK inhibitor, cancer, tumor, 结构式检索, structural formula search

**C. DOCUMENTS CONSIDERED TO BE RELEVANT**

| Category* | Citation of document, with indication, where appropriate, of the relevant passages | Relevant to claim No. |
|---|---|---|
| X | CN 116217588 A (TUOJIE BIOTECH (SHANGHAI) CO., LTD.) 06 June 2023 (2023-06-06) claims 1-15, and description, paragraphs [0192-0255] | 1-6, 8-22 |
| A | CN 116217588 A (TUOJIE BIOTECH (SHANGHAI) CO., LTD.) 06 June 2023 (2023-06-06) claims 1-15, and description, paragraphs [0192-0255] | 7 |
| X | WO 2022166799 A1 (TUOJIE BIOTECH (SHANGHAI) CO., LTD.) 11 August 2022 (2022-08-11) claims 1-22, and description, embodiments 1-4 | 21 |
| A | WO 2022166799 A1 (TUOJIE BIOTECH (SHANGHAI) CO., LTD.) 11 August 2022 (2022-08-11) claims 1-22, and description, embodiments 1-4 | 1-20, 22 |
| X | CN 114364675 A (QILU REGOR THERAPEUTICS INC.) 15 April 2022 (2022-04-15) claims 1-33, and description, paragraphs [0428-0535] | 22 |
| A | CN 114364675 A (QILU REGOR THERAPEUTICS INC.) 15 April 2022 (2022-04-15) claims 1-33, and description, paragraphs [0428-0535] | 1-21 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **13 September 2024** | **08 October 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

TRANSLATION

INTERNATIONAL SEARCH REPORT

International application No.
PCT/CN2024/107786

**C. DOCUMENTS CONSIDERED TO BE RELEVANT**

| Category* | Citation of document, with indication, where appropriate, of the relevant passages | Relevant to claim No. |
|---|---|---|
| X | STN. "RN2290368-74-8 etc." *Registry*, 20 March 2019 (2019-03-20), text, pp. 1-30 | 22 |
| A | STN. "RN2290368-74-8 etc." *Registry*, 20 March 2019 (2019-03-20), text, pp. 1-30 | 1-21 |
| PX | WO 2024027631 A1 (JIANGSU HENGRUI MEDICINE CO., LTD.) 08 February 2024 (2024-02-08) description, page 29, drawings | 21 |
| PA | WO 2024027631 A1 (JIANGSU HENGRUI MEDICINE CO., LTD.) 08 February 2024 (2024-02-08) claims 1-23 | 1-20, 22 |
| PX | CN 117486898 A (JIANGSU HENGRUI MEDICINE CO., LTD.) 02 February 2024 (2024-02-02) description, paragraph [0225] | 21 |
| PA | CN 117486898 A (JIANGSU HENGRUI MEDICINE CO., LTD.) 02 February 2024 (2024-02-02) claims 1-21 | 1-20, 22 |

Form PCT/ISA/210 (second sheet) (July 2022)

TRANSLATION

INTERNATIONAL SEARCH REPORT

International application No.
PCT/CN2024/107786

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☑ Claims Nos.: **22**
because they relate to parts of the international application that do not comply with the prescribed requirements to such an extent that no meaningful international search can be carried out, specifically:

The scope of the structure defined in claim 22 is very broad, and the related compounds cover a large number of known compounds in the prior art, which make it difficult for the examiner to carry out an exhaustive search of the prior art with regard to the current scope of the claim. The present search report is based on the technical solution of the structure of $R_1$ in formula A involved in most of the compounds in the embodiments being

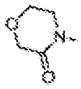

.

3. ☐ Claims Nos.:
because they are dependent claims and are not drafted in accordance with the second and third sentences of Rule 6.4(a).

TRANSLATION

**INTERNATIONAL SEARCH REPORT**
**Information on patent family members**

International application No.
**PCT/CN2024/107786**

| Patent document cited in search report | | | Publication date (day/month/year) | Patent family member(s) | | | Publication date (day/month/year) |
|---|---|---|---|---|---|---|---|
| CN | 116217588 | A | 06 June 2023 | None | | | |
| WO | 2022166799 | A1 | 11 August 2022 | US | 2024140963 | A1 | 02 May 2024 |
| | | | | EP | 4289848 | A1 | 13 December 2023 |
| | | | | CA | 3206898 | A1 | 11 August 2022 |
| | | | | AU | 2022217309 | A1 | 14 September 2023 |
| | | | | JP | 2024504452 | A | 31 January 2024 |
| | | | | KR | 20230142735 | A | 11 October 2023 |
| | | | | MX | 2023008896 | A | 09 August 2023 |
| CN | 114364675 | A | 15 April 2022 | US | 2024066031 | A1 | 29 February 2024 |
| | | | | AU | 2021268648 | A1 | 19 January 2023 |
| | | | | IL | 287767 | A | 01 January 2022 |
| | | | | TW | 202144351 | A | 01 December 2021 |
| | | | | JP | 2023525005 | A | 14 June 2023 |
| | | | | JP | 2022531687 | A | 08 July 2022 |
| | | | | BR | 112021022105 | A2 | 28 December 2021 |
| | | | | SG | 11202112229 | UA | 30 December 2021 |
| | | | | MA | 55909 | A | 16 March 2022 |
| | | | | MX | 2021013531 | A | 11 February 2022 |
| | | | | TW | 202108572 | A | 01 March 2021 |
| | | | | US | 2022296595 | A1 | 22 September 2022 |
| | | | | KR | 20220004755 | A | 11 January 2022 |
| | | | | CA | 3138973 | A1 | 12 November 2020 |
| | | | | EA | 202193015 | A1 | 17 March 2022 |
| | | | | WO | 2021226140 | A1 | 11 November 2021 |
| | | | | US | 2024033264 | A1 | 01 February 2024 |
| | | | | EP | 4146647 | A1 | 15 March 2023 |
| | | | | US | 2023174512 | A1 | 08 June 2023 |
| | | | | CO | 2021016504 | A2 | 17 January 2022 |
| | | | | AU | 2020269469 | A1 | 09 December 2021 |
| | | | | WO | 2020224568 | A1 | 12 November 2020 |
| | | | | EP | 3966213 | A1 | 16 March 2022 |
| | | | | EP | 3966213 | A4 | 19 April 2023 |
| | | | | CL | 2021002903 | A1 | 08 July 2022 |
| WO | 2024027631 | A1 | 08 February 2024 | TW | 202406912 | A | 16 February 2024 |
| CN | 117486898 | A | 02 February 2024 | None | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2022166799 A
- CN 114364675 A
- WO 2019207463 A
- WO 2020157652 A
- CN 116217588 A


### Non-patent literature cited in the description


- *Nature Reviews*, 2016, vol. 13, 417-430
- *J Clin Oncol*, 2017, vol. 35, 2875-2884
- **GUO CUIPING et al.** Regulation of Cyclin E and Malignant Tumors. *Journal of International Oncology*, 2012, vol. 39 (005), 337-340
- *Cell*, 08 June 2023, vol. 186 (12), 2628-2643